# EUROPEAN PATENT SPECIFICATION

(11) **EP 2 283 112 B1**
(45) Date of publication and mention of the grant of the patent: **12.10.2016**
(21) Application number: 09751313.9
(22) Date of filing: 18.05.2009
(51) Int. Cl.: A61K 39/02, C12N 15/74

(54) **COMPOSITIONS COMPRISING PRFA*MUTANT LISTERIA AND METHODS OF USE THEREOF**
ZUSAMMENSETZUNGEN MIT PRFA-MUTANTEN VON LISTERIEN UND ANWENDUNGSVERFAHREN DAFÜR
COMPOSITIONS COMPORTANT UN MUTANT PRFA DE LISTERIA, ET LEURS PROCÉDÉS D'UTILISATION

(30) Priority: 19.05.2008 US 54454 P
(43) Date of publication of application: 16.02.2011
(73) Proprietor: Aduro Biotech, Berkeley, California 94710-2225 (US)
(72) Inventor: LAUER, Peter, M., Albany CA 94706 (US); DUBENSKY, Thomas, W., Jr., Piedmont CA 94611 (US); SKOBLE, Justin, Berkeley CA 94703-1929 (US); BROCKSTEDT, Dirk, G., Oakland CA 94610 (US); LUCKETT, William, Stanford, Jr., Richmond CA 94805 (US); HANSON, William, Walnut Creek CA 94596-6721 (US)
(74) Representative: Brasnett, Adrian Hugh
(86) International application number: PCT/US2009/044408
(87) International publication number: WO 2009/143085

(56) References cited:
- WO-A2-2007/117371
- YAN, L ET AL.: 'Selected prfA* Mutations in recombinant attenuated Listeria monocytogenes strains augment expression of foreign immunogens and enhance vaccine-elicited humoral and cellular immune responses' INFECTION AND IMMUNITY vol. 76, no. 8, August 2008, pages 3439 - 3450, XP008145120
- BROCKSTEDT, D.G. ET AL: 'Killed but metabolically active microbes: a new vaccine paradigm for eliciting effector T-cell responses and protective immunity' NATURE MEDICINE vol. 11, no. 8, 2005, pages 853 - 860, XP002414545
- LAUER, P.E ET AL: 'Constitutive activation of the PrfA regulon enhances the potency of vaccines based on live-attenuated and killed but metabolically active Listeria monocytogenes strains' INFECTION AND IMMUNITY vol. 76, no. 8, August 2008, pages 3742 - 3753, XP008145170
- SHETRON-RAMA LYNNE M ET AL: "Intracellular induction of Listeria monocytogenes actA expression", INFECTION AND IMMUNITY, vol. 70, no. 3, March 2002 (2002-03), pages 1087-1096, ISSN: 0019-9567

## Description

### STATEMENT REGARDING FEDERALLY SPONSORED RESEARCH OR DEVELOPMENT

This invention was made, in part, with U.S. government support under grant 5U01AI070834-02 and SBIR grant 5R44CA101421-03, awarded by the National Institutes of Health. The government may have certain rights in the invention.

### FIELD OF THE INVENTION

The field of this invention relates generally to novel recombinant *Listeria* useful for expression of polypeptides, including heterologous polypeptides. In particular, this invention relates to recombinant bacteria comprising mutations in PrfA which are useful in vaccine compositions.

### BACKGROUND OF THE INVENTION

Recognition of the advantages of recombinant *Listeria monocytogenes* (Lm)-based vaccines as compared to other recombinant vaccine platforms has facilitated their ongoing development and current evaluation in early-phase clinical trials. These advantages include practical considerations such as straightforward fermentation methods for manufacturing, and other desirable features such as the ability to repeat administer even in the presence of protective Lm-specific immunity (Bouwer, H.G., et al. (1999) Infection and Immunity 67:253-258; Starks, H., et al. (2004) J Immunol 173:420-427, Stevens, R., et al. (2005) Vaccine 23:1479-1490). One compelling rationale for this vaccine platform is based on the well-known correlates of protection in the mouse listeriosis model: long-lived functional CD4+ and CD8+ memory T cells induced in response to a single immunization with *Listeria monocytogenes* (Harty, J.T., et al. (2000) Ann Rev Immunol 18:275-308; Pamer, E.G. (2004) Nat. Rev. Immunol. 4:812-823). There are now numerous publications that demonstrate striking efficacy of recombinant *Listeria monocytogenes* vaccines in several animal models due to robust innate and adaptive cellular immunity (Brockstedt, D. G., et al. (2004) Proc Natl Acad Sci U S A 101:13832-13837; Bruhn, K. W., et al. (2007) Microbes Infect 9:1226-1235; Paterson, Y., and Maciag, P.C. (2005) Curl Opin Mol Ther 7:454-460). The use of recombinant *Listeria monocytogenes* vaccines has been reported for the treatment of cancers and tumors (see, e.g., Brockstedt, et al. (2004) Proc. Natl. Acad. Sci. USA 101:13832-13837; Brockstedt, et al. (2005) Nature Med. 11:853-860); Starks, et al. (2004) J. Immunol. 173:420-427; Shen, et al. (1995) Proc. Natl. Acad. Sci. USA 92:3987-3991). *Listeria-*based vaccines are also reported, e.g., in U.S. Patent Publication Nos. 2005/0281783, 2005/0249748, 2004/0228877, and 2004/0197343, and in WO2007/117371. Recombinant Lm-based vaccines thus represent an emerging approach to address an acute global need for effective vaccines that elicit functional cellular immunity to prevent or treat infections such as HIV, HCV, tuberculosis, and malaria, as well as cancer. The results over the next several years will indicate whether the potent activity observed in pre-clinical studies will translate to efficacy in humans.

As *Listeria monocytogenes* a food-borne pathogen having increased virulence among immunocompromised individuals, attenuated vaccine platforms are a prerequisite for advancement to evaluation in humans (Lorber, B. (1997) Clin Infect Dis 24:1-9). Both live-attenuated and photochemically inactivated vaccine platforms derived from the wild-type strain 10403S have been described (Brockstedt, D. G., et al. (2005) Nat Med 11:853-860; Brockstedt, D. G., et al. (2004) Proc Natl Acad Sci U S A 101:13832-13837). The live-attenuated vaccine strain is deleted of both the *actA* and *inlB* virulence genes (*Listeria monocytogenes ΔactA*/*ΔinlB*), which in combination limit growth in the liver, a principal target organ of infection by the wild-type organism. This combination of deletions blocks direct hepatocyte infection via the InlB-hepatocyte growth factor receptor interaction (Dramsi, S., et al. (1995) Mol Microbiol 16:251-261) as well as indirect spread into hepatocytes via ActA-mediated cell-to-cell spread from infected liver-resident Kupffer cells. Liver toxicity in mice as measured by serum liver function tests (LFTs) alanine transaminase (ALT) and aspartate transaminase (AST) is dramatically lower in mice injected intravenously (IV) with *Listeria monocytogenes ΔactA*/*ΔinlB* as compared to wild-type Lm. Furthermore, liver toxicity was minimal and not dose limiting in two GLP toxicology studies performed in cynomolgus monkeys given escalating doses of *Listeria monocytogenes ΔactA*/*ΔinlB*-based strains (unpublished data). The *Listeria monocytogenes ΔactA*/*ΔinlB* vaccine strain forms the basis for two ongoing FDA approved Phase 1 clinical trials, being conducted in adult subjects with advanced cancers. The second vaccine platform, termed Killed But Metabolically Active (KBMA), is derived from *Listeria monocytogenes ΔactA*/*ΔinlB,* and also harbors deletions of both *uvrA* and *uvrB,* genes encoding the DNA repair enzymes of the nucleotide excision repair (NER) pathway. KBMA vaccines (*Listeria monocytogenes ΔactA*/*ΔinlB*/*ΔuvrAB*) are exquisitely sensitive to photochemical inactivation by the combined treatment with the synthetic psoralen, S-59, and long-wave UV light. While killed, KBMA *Listeria monocytogenes* vaccines can transiently express their gene products, allowing them to escape the phagolysosome and induce functional cellular immunity and protection against wild-type *Listeria monocytogenes* and vaccinia virus challenge (Brockstedt, D. G., et al. (2005) Nat Med 11:853-860).

PrfA is a transcription factor activated intracellularly that acts as a central virulence regulator, serving to enable what has been described as the "Dr. Jekyll and Mr. Hyde" dichotomy of *Listeria monocytogenes* growth lifestyles in mammals or as a saprophyte (Gray, M. J., et al. (2006) Infect Immun 74:2505-2512). PrfA knock-out strains are avirulent (Vazquez-Boland, J. A., et al. (2001) Clin Microbiol Rev 14:584-640). In wild-type Lm, PrfA is expressed upon infection of host cells, and in turn induces expression of the *prfA* regulon including the *hly* and *plcA* genes encoding lysteriolysin O (LLO) and phospholipase C, respectively. In combination, these gene products mediate escape of the bacterium from the harsh microenvironment of the phagolysosome. PfrA also regulates transcription of the internalin genes (e.g., *inlA* and *inlB*) which encode ligands that facilitate receptor-mediated infection of non-phagocytic cells (Scortti, M., et al. (2007) Microbes Inflect). PrfA-dependent promoters can be utilized to drive Ag expression in recombinant *Listeria monocytogenes* vaccines, by linking the heterologous gene to either the *hly* or *actA* promoters (Gunn, G. R., et al. (2001) J Immunology 167:6471-6479; Shen, H., et al. (1995) Proc Natl Acad Sci 92:3987-3991). Amino acid substitutions in PrfA that result in the constitutive activation of PrfA-dependent genes are known collectively as PrfA* mutants (Ripio, M. T., et al. (1997) J Bacteriol 179:1533-1540; Scortti, M., et al. (2007) Microbes Inflect). A number of wild-type *Listeria monocytogenes* strains with a hyper-hemolytic phenotype have a mutation in *prfA,* most commonly G145S, which can result in increased virulence as compared to laboratory adapted strains such as 10403S (Ripio, M. T., et al. (1997) J Bacteriol 179:1533-1540). Similarly, other *prfA* mutants with increased expression of PrfA-dependent genes that were selected by a chemical mutagenesis approach also had increased virulence in mice (Shetron-Rama, L. M., et al. (2003) Mol Microbiol 48:1537-1551). The induction of PrfA-dependent genes prior to immunization may enhance the efficiency of vaccines through diverse mechanisms, including increase of phagolysosomal escape and expression of PrfA-dependent encoded antigens in the cytosol of the host cell, leading to a more potent CD4+ and CD8+ T cell response. PrfA* mutations in recombinant attenuated *Listeria monocytogenes* strains are also described in Yan et al, Infection and Immunity, 76; 3493-3450 (2008).

Improvements in the methods for *Listeria*-mediated delivery of heterologous antigens to the cytosol of infected cells, especially antigen-presenting cells, are desired for the development of vaccines of increased efficacy. There is a continued need for refinements that either enhance potency or reduce toxicity of Lm-based vaccines in order to facilitate their ultimate clinical development.

### BRIEF SUMMARY OF THE INVENTION

The invention relates to *Listeria* comprising a constitutive mutant activator of virulence genes that are useful as heterologous antigen delivery vectors. In some embodiments, the delivery of heterologous polypeptides and/or polynucleotides from the *Listeria* to the cytosol of infected cells is enhanced by constitutive activation of the *prfA* regulon. Compositions such as pharmaceutical compositions and vaccines comprising the *Listeria* are described herein. Methods of using the *Listeria* to induce immune responses or treat or prevent disease in mammals are further described herein.

In one aspect, the invention provides a recombinant Listeria bacterium for use in a method of treatment, wherein (A) said bacterium comprises: (a) a polynucleotide encoding a PrfA* mutant polypeptide wherein the PrfA* mutant polypeptide comprises a G155S mutation; and (b) a recombinant polynucleotide comprising (i) an actA promoter; and (ii) a polynucleotide encoding a heterologous polypeptide, wherein the polynucleotide encoding the heterologous polypeptide is operably linked to the actA promoter, and wherein the heterologous polypeptide is non-bacterial or is an antigen of a heterologous infectious pathogen; and (B) said method is selected from: (i) a method of inducing an immune response in a host to the non-listerial antigen encoded by the heterologous polypeptide; (ii) a method of enhancing the immunogenicity of the non-listerial antigen encoded by the heterologous polypeptide in a host; (iii) a method of preventing or treating a non-listerial infectious or cancerous condition in a host; or (iv) a method for enhancing an immune response in a mammal to the non-listerial antigen encoded by the heterologous polypeptide in a host, wherein the mammal previously had been administered an effective amount of a prime dose of a vaccine that provided the non-listerial antigen, wherein (a) the vaccine does not contain live, metabolically active Listeria that encode the non-listerial antigen; and (b) when the vaccine contains naked DNA encoding the non-listerial antigen. The invention is further defined by the accompanying claims. In some aspects of the invention, the signal peptide is a signal peptide selected from the group consisting of an ActA signal peptide from *Listeria monocytogenes,* an LLO signal peptide from *Listeria monocytogenes,* a Usp45 signal peptide from *Lactococcus lactis,* a Protective Antigen signal peptide from *Bacillus anthracis,* a p60 signal peptide from *Listeria monocytogenes,* a PhoD signal peptide from *Bacillus subtilis*, a secA2 signal peptide and a Tat signal peptide. In some aspects, the signal peptide is an ActA signal peptide from *Listeria monocytogenes.* In some aspects, the fusion protein comprises the first 100 amino acids of ActA.

In some aspects of the invention, the recombinant *Listeria* bacterium comprises a heterologous polypeptide comprises an antigen selected from the group consisting of a tumor-associated antigen, a polypeptide derived from a tumor-associated antigen, an infectious disease antigen, and a polypeptide derived from an infectious disease antigen. In some aspects, the infectious disease antigen is from a virus or a heterologous infectious pathogen selected from the group consisting of a hepatitis virus, an influenza virus, a human immunodeficiency virus, papillomavirus, a herpes simplex virus 1, a herpes simplex virus 2, a cytomegalovirus, a *Mycobacterium tuberculosis,* a *Plasmodium falciparum* or a *Chlamydia trachomaitis.* Examples of hepatitis virus include, but are not limited to, hepatitis A virus, a hepatitis B virus, or a hepatitis C virus.

In some aspects of the invention, the *Listeria* comprising a constitutive mutant activator of virulence genes belongs to the species *Listeria monocytogenes.* In some aspects, the recombinant *Listeria* bacterium is attenuated; for example, for one or more of: cell-to-cell spread, entry into non-phagocytic cells, proliferation or DNA repair. In some cases the *Listeria* is attenuated by one or more of: an *actA* mutation, an *inlB* mutation, a *uvrA* mutation, a *uvrB* mutation, a *uvrC* mutation, a nucleic acid targeted compound, or a *uvrAB* mutation and a nucleic acid targeting compound. In some cases, the nucleic acid targeting compound is a psoralen. In some aspects, in the recombinant PrfA* *Listeria* bacterium defined above, the nucleic acid of the bacterium has been modified by reaction with a nucleic acid targeting compound that reacts directly with the nucleic acid so that the bacterium is attenuated for proliferation. In some cases, the bacterium comprises nucleic acid crosslinks that attenuate the modified bacterium for proliferation. In some cases, the bacterium comprises psoralen-nucleic acid adducts that attenuate the bacterium for proliferation. In some cases, the bacterium further comprises a genetic mutation that attenuates the ability of the bacterium to repair its modified nucleic acid. In some aspects of the invention, the bacterium comprises inactivating mutations in *actA, inlB, uvrA* and *uvrB*; and the bacterium has been attenuated for proliferation by psoralen-nucleic acid crosslinks. In some aspects of the invention the PrfA* *Listeria* is Killed But Metabolically Active (KBMA).

Described herein are pharmaceutical compositions comprising the recombinant PrfA* *Listeria* bacterium and one or more of a pharmaceutically acceptable excipient, an adjuvant and a costimulatory molecule. In some aspects, the composition further comprises a therapeutic agent.

Described herein are methods of inducing an immune response in a host to an non-listerial antigen comprising administering to the host an effective amount of a composition comprising a recombinant *Listeria* bacterium encoding a PrfA* mutant polypeptide; and a recombinant polynucleotide comprising *a prfA* responsive regulatory element and a polynucleotide encoding a heterologous polypeptide encoding the antigen operably linked to the *prfA* responsive regulatory element. In some aspects, methods of enhancing the immunogenicity of a non-listerial antigen in a host are described. In some aspects, methods of preventing or treating a non-listerial infectious or cancerous condition in a host are described. In some aspects, the antigen is selected from the group consisting of a tumor-associated antigen, a polypeptide derived from a tumor-associated antigen, an infectious disease antigen, and a polypeptide derived from an infectious disease antigen. In some aspects, the immune response is an innate immune response and in some aspects the immune response is an adaptive immune response. In some aspects of the invention, the host is human. In some aspects, the recombinant *Listeria* of the invention augment to functionality of T cells specific for encoded antigens that are elicited in response to administration with PrfA* recombinant *Listeria.*

Described herein are methods of inducing or enhancing an immune response in a host wherein the administration of the recombinant PrfA* *Listeria* bacterium is repeated. In some aspects, a recombinant PrfA* *Listeria* bacterium is administered first followed by one or more administrations with a non-listerial immunogenic composition. In some cases, a non-listerial immunogenic composition is administered first followed by one or more administrations of a recombinant PrfA* *Listeria* bacterium.

In some aspects, the immunogenicity to the antigen is enhanced relative to immunogenicity of the antigen induced by a recombinant *Listeria* bacterium wherein expression of the polynucleotide encoding the heterologous polypeptide is controlled by a wildtype PrfA polypeptide. In some cases, the enhanced immunogenicity comprises increased expression of one or any combination of MCP-1, IL-6, IFN-γ, TNFα or IL-12p70.

Described herein are methods of preparing a recombinant *Listeria* bacterium. For example, recombinant polynucleotide encoding a heterologous polypeptide under the control of a PrfA-responsive regulatory element is stably introduced into a PrfA* *Listeria* bacterium. In some cases the heterologous polypeptide is fused to a signal sequence. In some aspects, the recombinant polynucleotide encoding the heterologous polypeptide is integrated into the *Listeria* chromosome. In some cases, the recombinant polynucleotide encoding the heterologous polypeptide is integrated into a *tRNA^{arg}* gene or into the *actA* gene of the *Listeria* chromosome. In some aspects, a recombinant polynucleotide encoding a PrfA* mutant polypeptide, is stably introduced into a *Listeria* bacterium containing a nonfunctional *prfA* allele.

### DRAWINGS

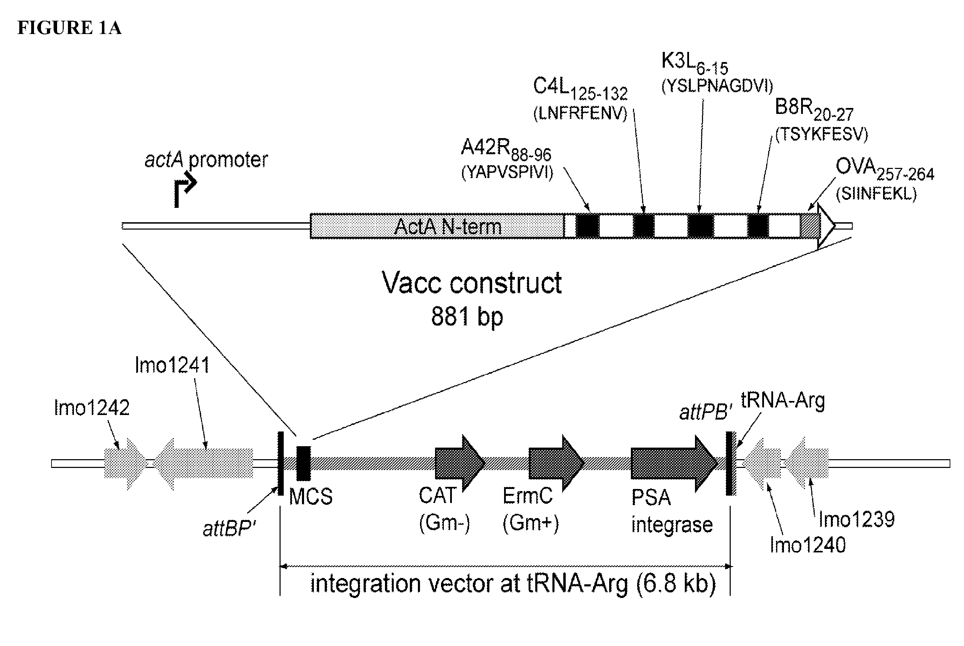

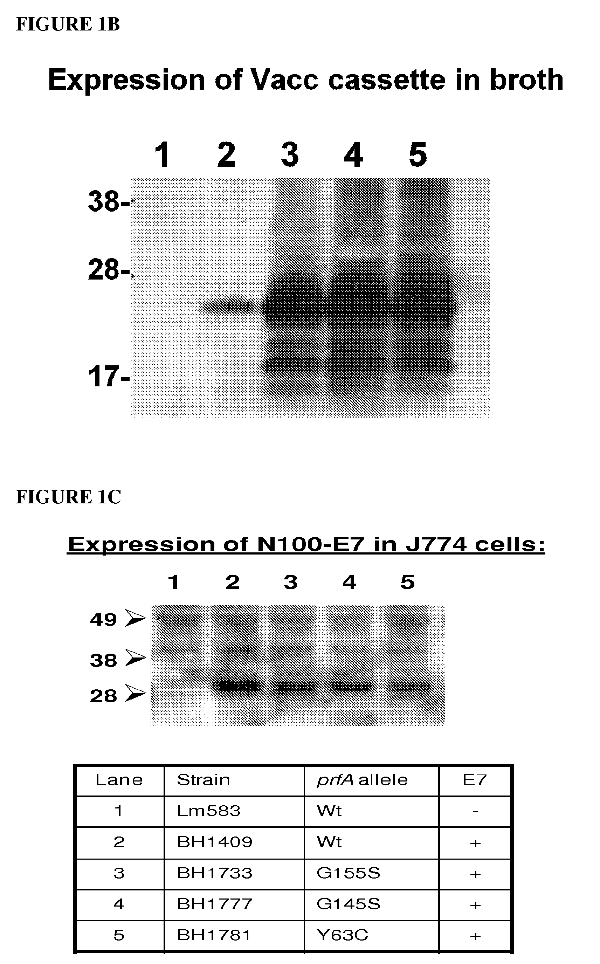

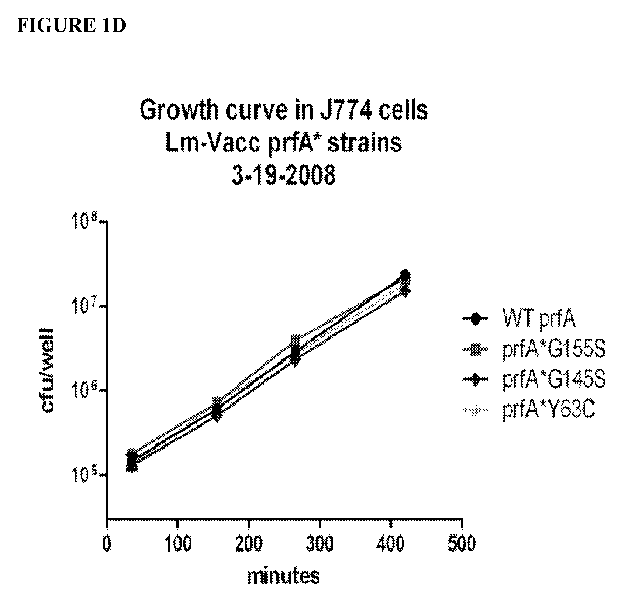

Figure 1 shows the characterization of *Listeria monocytogenes prfA** vaccine strains. (A) Construction of the *Listeria monocytogenes* Quadvac strain expressing four vaccinia virus T cell epitopes (A24R, C4L, K3L, and B8R) and the ovalbumin SL8 epitope spaced with linker sequences and fused to the first 100 amino acids of ActA (ActAN100) using the pPL2 site-specific integration vector. (B) Expression of the heterologous protein in yeast extract broth. (C) Expression of the heterologous Ag at 7 hr post infection in infected J774 macrophage cells. (D) Expression of the heterologous Ag at 2.5 hr post infection in infected DC2.4 dendritic cells. (E) Intracellular growth of isogenic *Listeria monocytogenes* vaccine strains in J774 macrophages.

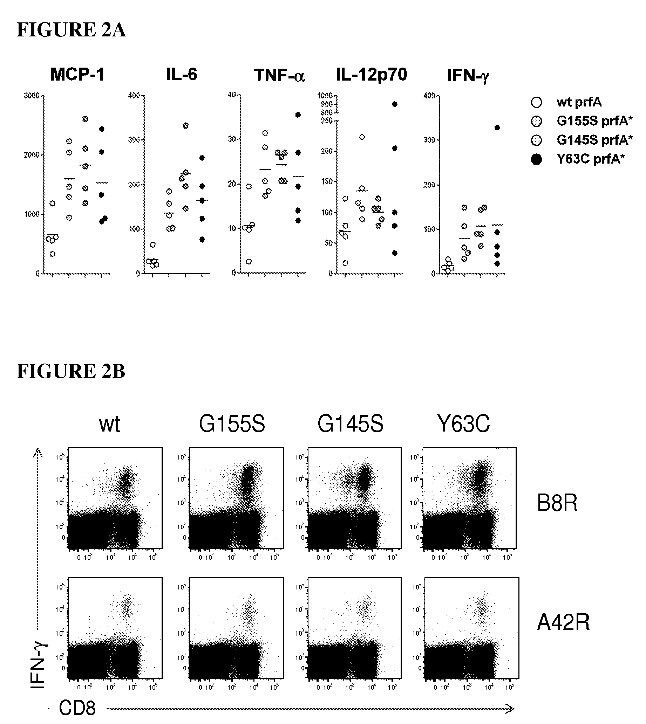

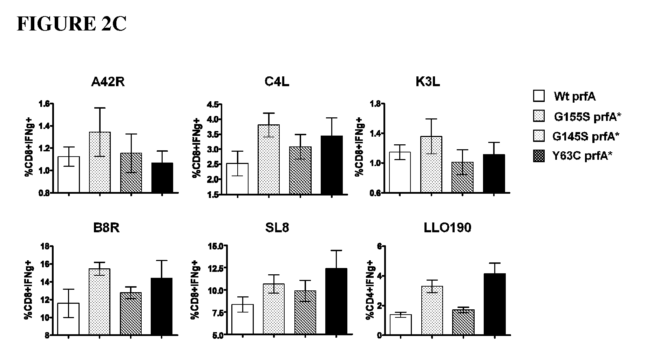

Figure 2 shows the improved innate and adaptive immunity induced by PrfA* vaccine strains. (A) Serum cytokine/chemokine levels determined 8 hours following a single intravenous administration of 5×10⁶ cfu of *Listeria monocytogenes ΔactA*/*ΔinlB*/WT *prfA,* PrfA* G155S, PrfA* G145S, and PrfA* Y63C strains. Cytokines/chemokines were determined by cytometric bead array (CBA). Each symbol represents a single animal. Data are from a single experiment, representative from at least two experiments. (B, C) Live-attenuated PrfA* vaccine strains induced antigen-specific immunity of higher magnitude. C57BL/6 mice were immunized intravenously with 5×10⁶ cfu *of Listeria monocytogenes* Δ*actA*/Δ*inlB*/WT *prfA,* PrfA *G155S, PrfA* G145S, and PrfA* Y63C strains. Antigen-specific T cell responses were determined by intracellular cytokine staining at the peak of the response 7 days following vaccination. (B) Dot blots from a representative animal from each group are shown. (C) The mean ± standard deviations are shown for each group of 5 animals.

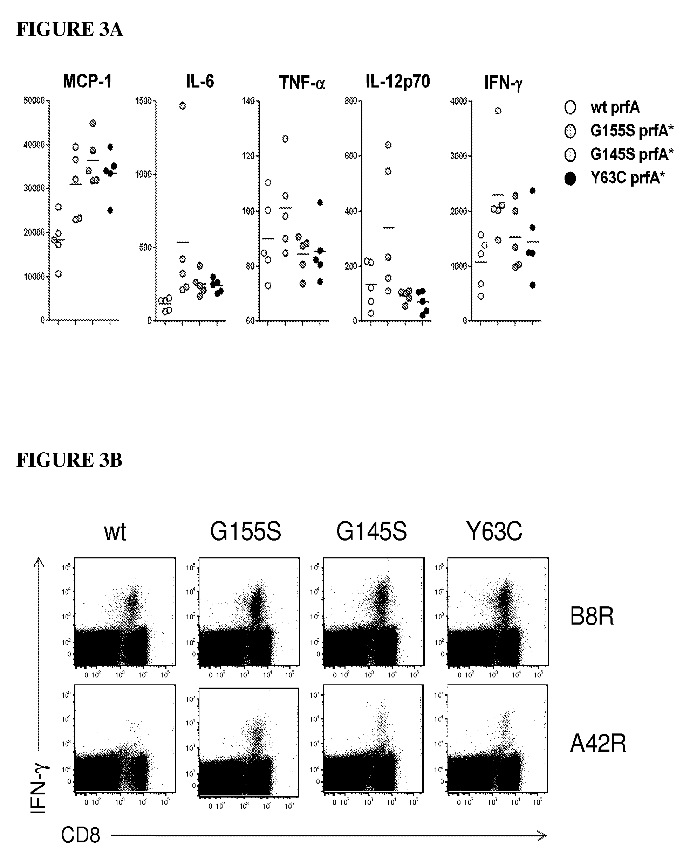

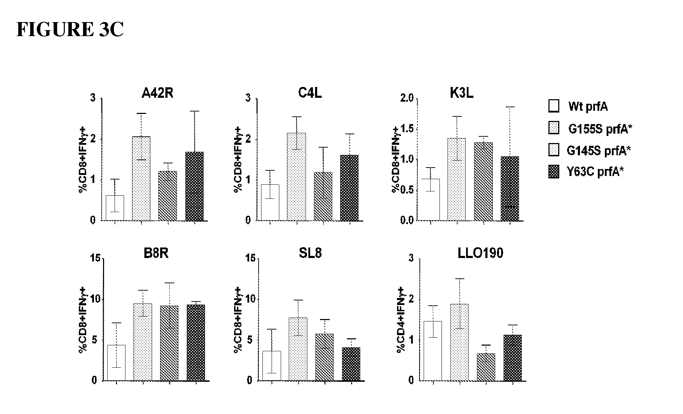

Figure 3 shows PrfA* enhances the immunogenicity of KBMA *Listeria monocytogenes* vaccines. (A) Serum cytokine/chemokine levels determined 8 hours following a single intravenous administration of 1×10⁸ particles of KBMA *Listeria monocytogenes ΔactA*/ *ΔinlB*/WT *prfA,* PrfA* G155S, PrfA* G145S, and PrfA* Y63C strains. Cytokines/chemokines were determined by cytometric bead analysis (CBA). Each symbol represents a single animal. (B, C) KBMA *Listeria monocytogenes* PrfA* strains induced antigen-specific immunity of higher magnitude. C57BL/6 mice were immunized intravenously with 1×10⁸ particles of KBMA *Listeria monocytogenes ΔactA*/*Δin*/*B*/WT *prfA,* PrfA* G155S, PrfA* G145S, and PrfA* Y63C strains. Antigen-specific T cell responses were determined by intracellular cytokine staining at the peak of the response 7 days following vaccination. (B) Dot blots from a representative animal from each group are shown. (C) The mean ± standard deviations are shown for each group of 5 animals.

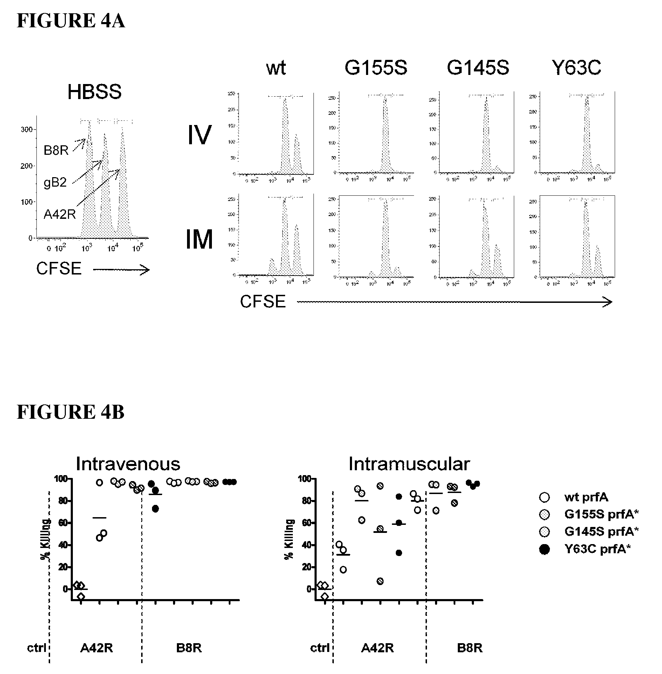

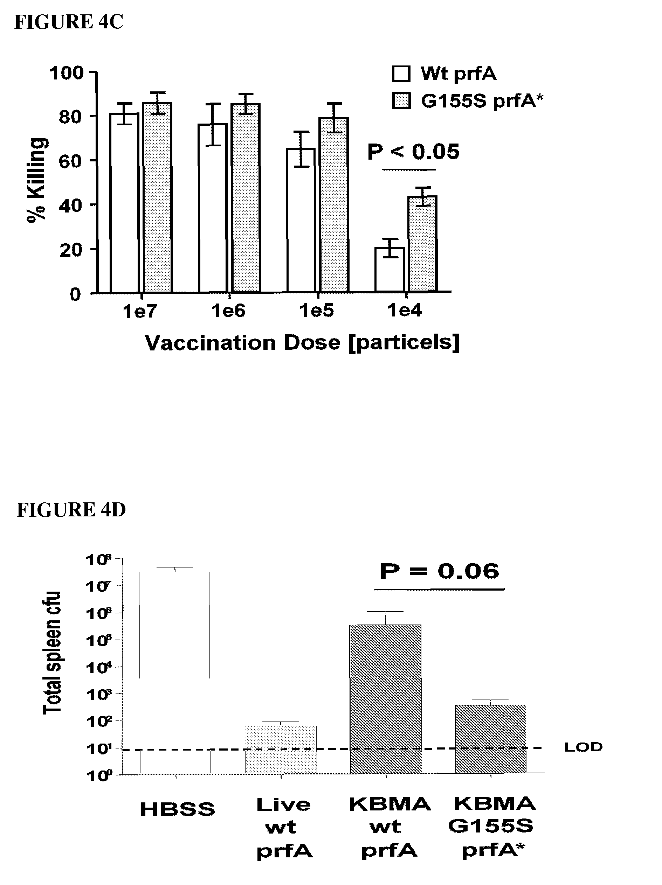

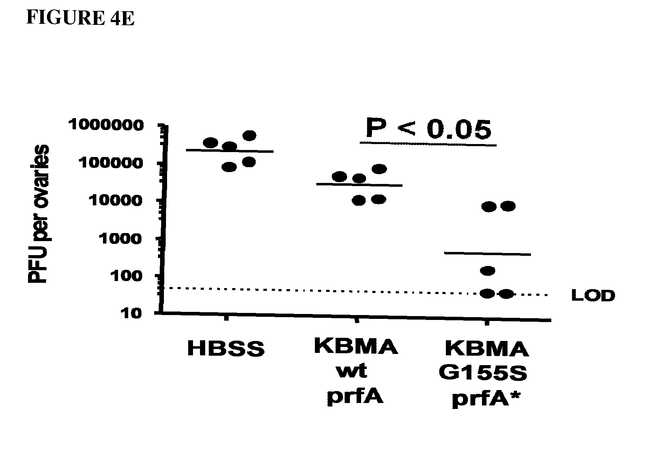

Figure 4 shows improved potency of the elicited T cell response by KBMA *Listeria monocytogenes* PrfA* strains. (A, B) C57BL/6 mice were immunized intravenously or intramuscularly (as indicated in the figure) twice two weeks apart with HBSS (left panel), or 1×10⁸ particles of KBMA *Listeria monocytogenes ΔactA*/*ΔinlB*/WT *prfA,* PrfA*G155S, PrfA*G145S, and PrfA*Y63C strains. In vivo cytolytic activity was determined 7 days later by challenging mice with gB2 (control; middle peak), A24R-loaded targets (right peak) or B8R-loaded targets (left peak). (A) A histogram for a representative animal is shown for each group. (B) In vivo cytolytic activity specific to A42R and B8R is shown for mice vaccinated intravenously or intramuscularly. Each symbol represents an individual animal. (C) In vivo cytolytic activity specific to B8R is shown following two vaccinations two weeks apart at various doses of KBMA *Listeria monocytogenes ΔactA*/ *ΔinlB*/WT *prfA* or PrfA*G155S. The mean ± standard deviation is shown for groups with each 5 animals. (D) Protective immunity to a 2×LD₅₀ challenge with wild-type *Listeria monocytogenes* is shown. Balb/c mice were immunized intravenously once with 1×10⁸ particles of KBMA *Listeria monocytogenes ΔactA*/*ΔinlB*/WT *prfA* or PrfA*G155S. HBSS served as control. Spleens were harvested three days after challenge and plated for CFU. The difference in log-protection between WT *prfA* and *prfA**G155S is statistically significant, as determined by Student T test. (E) Viral titers in ovaries following an intraperitoneal challenge with 1×¹⁰⁷ pfu of vaccinia virus. C57BL/6 mice were vaccinated twice intravenously with either 1×10⁸ particles of KBMA *Listeria monocytogenes ΔactA*/*ΔinlBl*WT *prfA* or *prfA**G155S*.* Viral titers were determined 5 days post vaccinia virus challenge. Each symbol represents an individual animal. The difference in log-protection between WT PrfA and PrfA*G155S is statistically significant, as determined by Student T test.

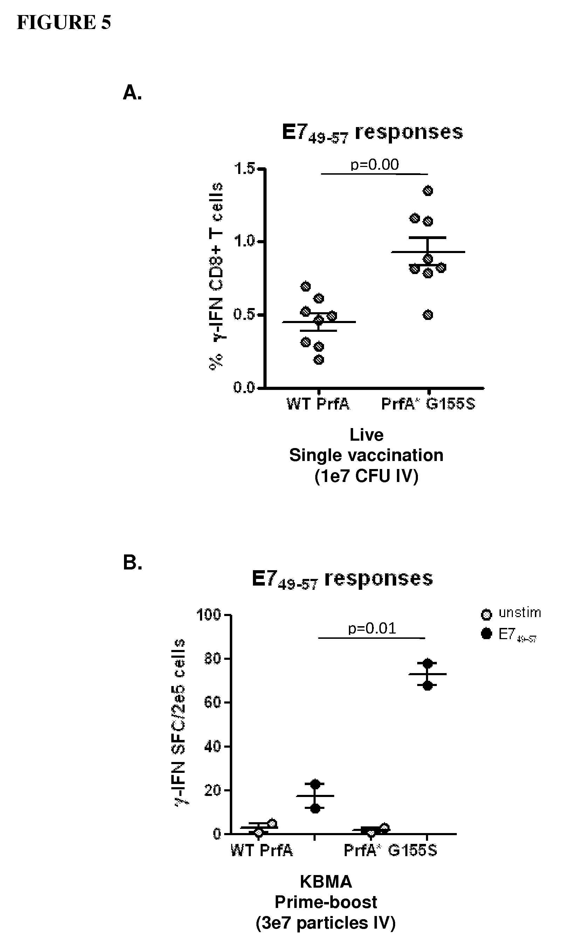

Figure 5 shows PrfA* enhances the immunogenicity of KBMA *Listeria monocytogenes* vaccines encoding HPV E7. HPV E7-specific T cell responses were determined by intracellular cytokine staining at the peak of the response 7 days following last vaccination. (A) Data from a single vaccination with a live *Listeria.* (B) Data form a prime-boost vaccination with KBMA.

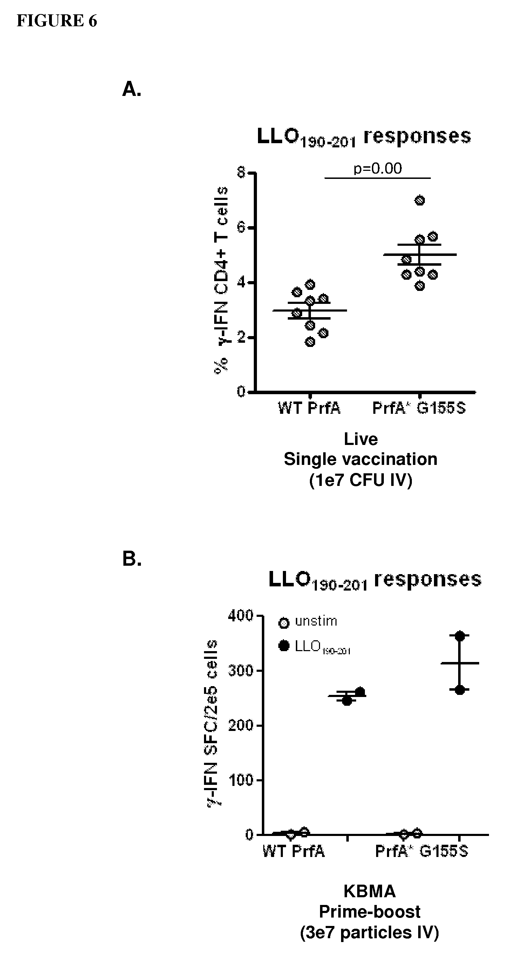

Figure 6 shows PrfA* enhances the immunogenicity of KBMA *Listeria monocytogenes* vaccines encoding HPV E7. LLO-specific T cell responses were determined by intracellular cytokine staining at the peak of the response 7 days following last vaccination. (A) Data from a single vaccination with a live *Listeria.* (B) Data form a prime-boost vaccination with KBMA.

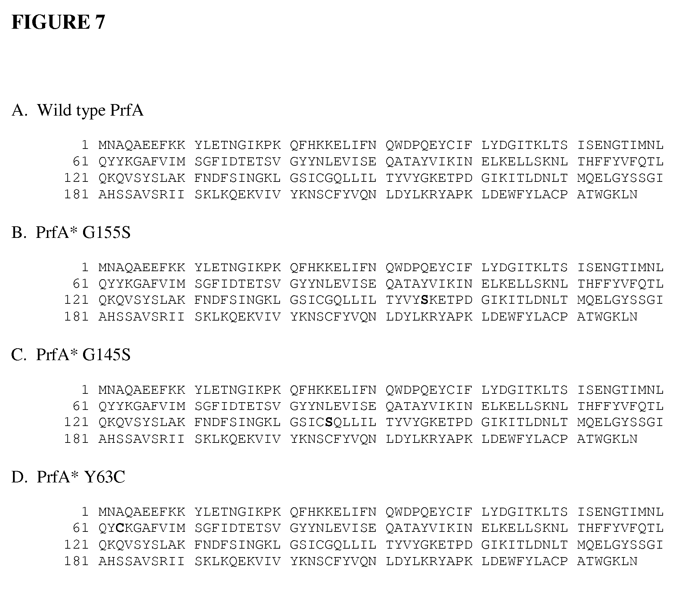

Figure 7 shows the amino acid sequences of PrfA, PrfA* G155S, PrfA* G145S and PrfA* Y63C.

### DETAILED DESCRIPTION OF THE INVENTION

### I. Introduction

The invention is based, in part, on the discovery that an immune response to an antigen may be enhanced when the antigen is expressed under the control of a constitutive mutant activator of virulence genes in *Listeria.* The dichotomous lifestyle of *Listeria monocytogenes* has been compared to "Dr. Jekyll and Mr. Hyde." As a saprophyte, *Listeria* lives a benign life in the environment. Upon infection of a mammalian host, however, *Listeria* turns pernicious by way of expression of a number of virulence genes which allow the *Listeria* to grow and prosper under mammalian physiological conditions. A key player in the switch from the benign saprophytic lifestyle to the pernicious virulent lifestyle is the PrfA protein. Activation of PrfA in response to numerous stimuli including temperature, pH, iron concentration, carbohydrate concentration and reactive oxygen species. Mutants of PrfA have been identified, in part, by constitutive expression of PrfA-dependent genes. Such constitutive PrfA mutants have been designated PrfA* mutants. In some cases, constitutive PrfA* mutant polypeptides have been shown to exhibit a hypervirulent phenotype in *Listeria*; for example, the G155S PrfA* mutant.

The present invention provides a recombinant *Listeria* bacterium for use in a method of treatment in accordance with the claims.

In some aspects of the invention, the heterologous polypeptide is a tumor antigen and in some aspects of the invention, the polypeptide is an antigen associated with infectious disease. In some aspects of the invention, the heterologous polypeptide is a non-listerial polypeptide and in some aspects of the invention, the heterologous polypeptide is non-bacterial.

In some aspects of the invention, the *Listeria* is *Listeria monocytogenes.* In some cases, the *Listeria* is attenuated for cell-to-cell spread and/or entry into nonphagocytic cells. In some cases, the *Listeria* comprises an inactivating mutation in *actA* and/or *inlB.* In some cases, the *Listeria* is an *actA inlB* double deletion mutant. In some cases, the *Listeria* comprises an inactivating mutation in at least one nucleic acid repair gene, such as *uvrA*, *uvrB, uvrC,* or a recombinational repair gene. For instance, the *Listeria* may be an *uvrAB* deletion mutant. In some cases, the bacterium further comprises a nucleic acid cross-linking agent (e.g., a psoralen). In some aspects of the invention, the *Listeria* is killed, but metabolically active (KBMA).

Pharmaceutical compositions, immunogenic compositions, and/or vaccines comprising the *Listeria* of the aforementioned aspects are further provided. In some aspects, the pharmaceutical compositions further comprise a pharmaceutically acceptable carrier. In some aspects of the invention, the *Listeria* further comprises an adjuvant.

In some aspects of the invention, the *Listeria* of the invention is used in combination with a therapeutic agent. In some cases, the *Listeria* is administered with the therapeutic agent, in some cases the *Listeria* is administered before the therapeutic agent. In some cases, the *Listeria* is administered after the therapeutic agent.

Described herein are methods of using the recombinant *Listeria* of the invention. In some cases, methods of inducing an immune response to an antigen are described. In some cases, methods of enhancing the immunogenicity of an antigen are described. In some cases, the immunogenicity of the antigen is enhanced relative to the immunogenicity of the antigen is expressed in *Listeria* under the control of the wild type PrfA polypeptide. The enhanced immunogenicity can be measured by methods known in the art. In some aspects, the enhanced immunogenicity can be measured by measuring increased expression of cytokines, chemokines and polypeptides known to be induced by an immune response. For example, MCP-1, IL-6, IFN-γ, TNFα and/or IL-12 p70. In some aspects of the invention, the increased expression of cytokines, chemokines and polypeptides known to be induced by an immune response can be increased relative to the their expression induced by the antigen when expressed in *Listeria* under the control of the wild type PrfA polypeptide. The present disclosure provides methods of preventing or treating an infectious or cancerous condition.

The use of recombinant *Listeria monocytogenes*vaccines has been reported for the treatment of cancers and tumors (see, e.g., Brockstedt, et al. (2004) Proc. Natl. Acad. Sci. USA 101:13832-13837; Brockstedt, et al (2005) Nature Med. 11:853-860); Starks, et al. (2004) J. Immunol. 173:420-427; Shen, et al. (1995) Proc. Natl. Acad. Sci. USA 92:3987-3991). *Listeria*-based vaccines are also reported, e.g., in U.S. Patent Publication Nos. 2007/0207170; 2007/0207171; 2007/0190063; 2005/0281783, 2005/0249748, 2004/0228877, and 2004/0197343.

### II. PrfA and the prfA regulon

PrfA (positive regulatory factor A) plays a key role in the induction of a set of genes that are important in listerial virulence (Scortti, M. et al. (2007) Microbes and Infection 9(10):1196-207). PrfA activates transcription by binding to a palindromic promoter element with the canonical sequence tTAACanntGTtAa (capitals represent invariant nucleotides). The core PrfA regulon is outlined in Table 1. A number of other genes are weakly or inconsistently regulated by PrfA (see Scortti, M *et al.* (2007)). In some aspects of the invention, a heterologous polypeptide is expressed under the control of a promoter of the PrfA regulon.

**Table 1 The core PrfA regulon**

| **Gene** | **Function** |
|---|---|
| *hly* | Listeriolysin O |
| *plcA* | Phosphatidylinositol-specific phospholipase C |
| *prfA* | Positive regulatory factor A |
| *mpl* | Zinc metalloproteinase precursor |
| *actA* | Actin-polymerizing protein |
| *plcB* | Broad substrate range phospholipase C |
| *hpt* | Hexose phosphate transporter |
| *inlC* | Internalin C |
| *inlA* | Internalin A |
| *inlB* | Internalin B |

PrfA is a 27 kDal protein that is structurally related to Crp (cAMP receptor protein) of enterobacteria (Figure 7). The protein is comprised of an N-terminal domain with a β-roll, an α-helix, a hinge/interdomain region, a DNA binding helix-turn-helix (HTH) domain and a C-terminal αG domain that helps stabilize the HTH motif. PrfA exists in two functional states, a weakly active state in the native form and a highly active state following a conformational change. The conformational change from the native low active state to the highly activated state appears to be activated by environmental stimuli including temperature, pH, low iron concentration, low carbohydrate sources and the presence of reactive oxygen species. PrfA activation appears to take place primarily in the cytosol of the infected cell. The regulation of PrfA-dependent transcription relies on three main elements, i) changes in PrfA protein activity, ii) changes in PrfA concentration and iii) differential expression based on promoter configuration. Accordingly, in some aspects of the invention, expression of a heterologous polypeptide is responsive to these changes in PrfA activity.

A number of *Listeria monocytogenes* strains containing PrfA mutant polypeptides have been identified in which PrfA-dependent genes are constitutively overexpressed (Scortti, M. et al. (2007) Microbes and Inflection 9(10):1196-207). Such PrfA polypeptide mutants are referred to as PrfA* mutations or PrfA* mutant polypeptides and include, but are not limited to I45S, Y63C, E77K, L140F, G145S and G155S mutations (Figure 7). It has been postulated that PrfA* mutant polypeptides mimic the conformational change responsible for the switch from the low active state to the high active state of PrfA. In *Listeria* strains harboring PrfA* mutants, a hyperactive PrfA protein causes the constitutive overexpression of PrfA-dependent genes under conditions in which they are normally downregulated. In some cases, the mutation in the PrfA* polypeptide may enhance binding of the PrfA polypeptide to DNA, for example, PrfA* G145S and PrfA* L140F; and in some cases, the PrfA* mutation may enhance cofactor binding to the PrfA polypeptide, for example, PrfA* Y63C (Miner, M.D., et al. (2008) submitted to J. Biol. Chem.). *Listeria* strains harboring PrfA* mutants are virulent and, at least in the case of PrfA* G155S, the mutant *Listeria* is hypervirulent.

In addition to the PrfA* mutant polypeptides outlined above, one of skill in the art may generate other PrfA* mutant polypeptides. For example, *Listeria* may be contacted with an immunogen such as ethylmethane sulfonate, followed by growth under conditions in which PrfA-dependent genes are normally downregulated (Shetron-Rama, L.M. et al. (2003) Mol. Microbiol. 48:1537-1551). Examples of conditions which repress expression of PrfA-dependent genes include the presence of readily metabolizable sugars and low pH. *Listeria* harboring PrfA* mutants may be identified by a number of different screening methods including but not limited to direct measurement of expression of PrfA-dependent genes (see Table 1) or expression of a reporter gene under the control of a PrfA-regulon promoter. Other methods of mutagenizing the *prfA* gene are known in the art, including but not limited to site-directed mutagenesis, chemical synthesis of genes with specifically placed mutations and DNA shuffling technologies.

The impact of a three PrfA* mutants, including G145S, G155S and Y63C, on the potency of isogenic live-attenuated and KBMA vaccine strains has been assessed (see Example 1). To enable one to distinguish immunologic potency differences between isogenic *Listeria monocytogenes* vaccine strains, an Ag expression cassette was generated that encoded five well-defined MHC class I epitopes that have been shown previously to elicit a wide range of CD8+ T cell responses in mice (Moutaftsi, M., et al. (2006) Nat Biotechnol 24:817-819). Although the growth curves of the isogenic strains in broth culture were indistinguishable, significantly increased levels of Ag expression and secretion were observed in the PrfA* vaccine strains compared to the *Listeria monocytogenes* strain with native *prfA.* Interestingly, Ag expression levels measured in the cytosol from infected macrophage or dendritic cell lines as well as infection and intracellular growth between all isogenic vaccine strains was equivalent. Strikingly however, immunogenicity in mice was *prfA* dependent, and was clearly optimal with PrfA* G155S, as compared to the other *prfA* alleles tested. The magnitude and functionality of vaccine-induced CD8+ T cells as measured by protection against bacterial or viral challenge was significantly improved among live-attenuated and KBMA recombinant *Listeria monocytogenes* vaccine strains with PrfA* G155S, as compared to vaccines with all other *prfA* alleles tested. Thus, while relative PrfA dependent expression in broth culture did not necessarily correlate with vaccine potency, the *prfA* G155S activated mutation did enhance vaccine potency. Taken together, these findings indicate that activation of the *prfA* regulon and induction of Ag expression prior to immunization enhances the potency of Lm-based vaccines.

In the present invention, a heterologous polypeptide is expressed under the control of a PrfA regulon in a recombinant *Listeria* bacterium comprising a PrfA* G155S mutant polypeptide.

### III. Signal peptides

The terms "signal peptide" and "signal sequence," are used interchangeably herein. In some embodiments, the signal peptide helps facilitate transportation of a polypeptide fused to the signal peptide across the cell membrane of a cell (e.g., a bacterial cell) so that the polypeptide is secreted from the cell. Accordingly, in some embodiments, the signal peptide is a "secretory signal peptide" or "secretory sequence." A signal peptide is typically positioned at the N-terminal end of the polypeptide to be secreted.

In some embodiments, the signal peptides that are a part of the fusion proteins and/or protein chimeras encoded by the recombinant nucleic acid molecules, expression cassettes and/or expression vectors, are heterologous to at least one other polypeptide sequence in the fusion protein and/or protein chimera. In some embodiments, the signal peptide encoded by the recombinant nucleic acid molecule, expression cassette and/or expression vector is heterologous (i.e., foreign) to the bacterium into which the recombinant nucleic acid molecule, expression cassette and/or expression vector is to be incorporated or has been incorporated. In some embodiments, the signal peptide is native to the bacterium in which the recombinant nucleic acid molecule, expression cassette and/or expression vector is to be incorporated.

In some embodiments, the polynucleotide encoding the signal peptide is codon-optimized for expression in a bacterium (e.g., *Listeria* such as *Listeria monocytogenes*). In some embodiments, the polynucleotide that is codon-optimized for a particular bacterium is foreign to the bacterium. In other embodiments, the polynucleotide that is codon-optimized for a particular bacterium is native to that bacterium.

A large variety of signal peptides are known in the art. In addition, a variety of algorithms and software programs, such as the "SignalP" algorithms, which can be used to predict signal peptide sequences are available in the art. For instance, see: Antelmann et al., Genome Res., 11:1484-502 (2001); Menne et al., Bioinformatics, 16:741-2 (2000); Nielsen et al., Protein Eng., 10:1-6 (1997); Zhang et al., Protein Sci., 13:2819-24 (2004); Bendtsen et al., J. Mol. Biol., 340:783-95 (2004) (regarding SignalP 3.0); Hiller et al., Nucleic Acids Res., 32:W375-9 (2004); Schneider et al., Proteomics 4:1571-80 (2004); Chou, Curr. Protein Pept. Sci., 3:615-22 (2002); Shah et al., Bioinformatics, 19:1985-96 (2003); and Yuan et al., Biochem Biophys Res. Commun. 312:1278-83 (2003).

In some embodiments the signal peptide is prokaryotic. In some alternative embodiments, the signal peptide is eukaryotic. The use of eukaryotic signal peptides for expression of proteins in *Escherichia coli* for example, is described in Humphreys et al., Protein Expression and Purification, 20:252-264 (2000).

In some embodiments, the signal peptide is a bacterial signal peptide. In some embodiments, the signal peptide is a non-Listerial signal peptide. In some embodiments, the signal peptide is a non-Listerial signal peptide. In some embodiments the signal peptide is derived from a gram-positive bacterium. In some embodiments, the signal peptide is derived from an intracellular bacterium.

In some embodiments, the signal peptide used in a recombinant nucleic acid molecule is derived from *Listeria.* In additional embodiments, this signal peptide is derived from *Listeria monocytogenes.* In some embodiments, the signal peptide is a signal peptide from *Listeria monocytogenes.* In alternative embodiments, the bacterial signal peptide is derived from *Bacillus.* In some embodiments, the bacterial signal peptide is derived from *Staplylococcus.* In some embodiments, the bacterial signal peptide is derived from *Lactococcus.* In some embodiments, the bacterial signal peptide is derived from *Bacillus, Staphylococcus*, or *Lactococcus.* In some embodiments, the bacterial signal peptide is a signal peptide from a *Bacillus, Staphylococcus*, or *Lactococcus* bacterium. In some embodiments, the bacterial signal peptide is a signal peptide from *Bacillus anthracis, Bacillus subtilis, Staphylococcus aureus,* or *Lactococcus lactis.*

In some embodiments of the polynucleotides described herein, the signal peptide that is derived from an organism, such as a bacterium, is identical to a naturally occurring signal peptide sequence obtained from the organism. In other embodiments, the signal peptide sequence encoded by the recombinant nucleic acid molecule is a fragment and/or variant of a naturally occurring signal peptide sequence, wherein the variant still functions as a signal peptide. A variant includes polypeptides that differ from the original sequence by one or more substitutions, deletions, additions, and/or insertions. For instance, in some embodiments the signal peptide that is encoded by the polynucleotides contains one or more conservative mutations. Possible conservative amino acid changes are well known to those of ordinary skill in the art.

A signal peptide derived from another signal peptide (i.e., a fragment and/or variant of the other signal peptide) is preferably substantially equivalent to the original signal peptide. For instance, the ability of a signal peptide derived from another signal peptide to function as a signal peptide should be substantially unaffected by the variations (deletions, mutations, etc.) made to the original signal peptide sequence. In some embodiments, the derived signal peptide is at least about 70%, at least about 80%, at least about 90%, or at least about 95% able to function as a signal peptide as the native signal peptide sequence. In some embodiments, the signal peptide has at least about 70%, at least about 80%, at least about 90%, or at least about 95% identity in amino acid sequence to the original signal peptide. In some embodiments, the only alterations made in the sequence of the signal peptide are conservative amino acid substitutions. Fragments of signal peptides are preferably at least about 80 or 90% of the length of the original signal peptides.

In some embodiments, the signal peptide encoded by a polynucleotide encoding a heterologous polypeptide is a secA1 signal peptide, a secA2 signal peptide, or a Twin-arginine translocation (Tat) signal peptide. In some embodiments, the signal peptide is a secA1 signal peptide signal peptide. In some embodiments, the signal peptide is a non-secA1 signal peptide. In some embodiments, the signal peptide is a secA2 signal peptide. In some embodiments, the signal peptide is a Twin-arginine translocation (Tat) signal peptide. In some embodiments, these secA1 secA2, or Tat signal peptides are derived from *Listeria.* In some embodiments, these secA1 secA2, or Tat signal peptides are non-Listerial. For instance, in some embodiments, the secA1 secA2, and Tat signal peptides are derived from bacteria belonging to one of the following genera: *Bacillus, Staphylococcus*, or *Lactococcus.*

In some embodiments the fusion protein comprises signal peptide is ActA signal peptide from *Listeria monocytogenes* and a heterologous polypeptide. In some cases the present invention, in certain aspects, provides a polynucleotide comprising a first nucleic acid encoding a modified ActA, operably linked and in frame with a second nucleic acid encoding a heterologous antigen. The invention also provides a *Listeria* containing the polynucleotide, where expression of the polynucleotide generates a fusion protein comprising the modified ActA and the heterologous antigen. The modified ActA can include the natural secretory sequence of ActA, a secretory sequence derived from another *Listeria* protein, a secretory sequence derived from a non *Listeria* bacterial protein, or the modified ActA can be devoid of any secretory sequence.

The ActA derived fusion protein partner finds use in increasing expression, increasing stability, increasing secretion, enhancing immune presentation, stimulating immune response, improving survival to a tumor, improving survival to a cancer, increasing survival to an infectious agent, and the like.

In one aspect, a polynucleotide comprising a PrfA-dependent promoter is operably linked to a nucleic acid sequence encoding a fusion protein, wherein the fusion protein comprises (a) modified ActA and (b) a heterologous antigen. In some embodiments, the promoter is ActA promoter. In some embodiments, the modified ActA comprises at least the first 59 amino acids of ActA. In some embodiments, the modified ActA comprises more than the first 59 amino acids of ActA. In some embodiments, the modified ActA is a fragment of ActA comprising the signal sequence of ActA (or is derived from a fragment of ActA comprising the signal sequence of ActA). In some embodiments, the modified ActA comprises at least the first 59 amino acids of ActA, but less than about the first 265 amino acids of ActA. In some embodiments, the modified ActA comprises more than the first 59 amino acids of ActA, but less than about the first 265 amino acids of ActA. In other words, in some embodiments, the modified ActA sequence corresponds to an N-terminal fragment of ActA (including the ActA signal sequence) that is truncated somewhere between amino acid 59 and about amino acid 265 of the Act A sequence. In some embodiments, the modified ActA comprises the first 59 to 200 amino acids of ActA, the first 59 to 150 amino acids of ActA, the first 59 to 125 amino acids of ActA, or the first 59 to 110 amino acids of ActA. In some embodiments, the modified ActA consists of the first 59 to 200 amino acids of ActA, the first 59 to 150 amino acids of ActA, the first 59 to 125 amino acids of ActA, or the first 59 to 110 amino acids of ActA. In some embodiments, the modified ActA comprises about the first 65 to 200 amino acids of ActA, about the first 65 to 150 amino acids of ActA, about the first 65 to 125 amino acids of ActA, or about the first 65 to 110 amino acids of ActA. In some embodiments, the modified ActA consists of about the first 65 to 200 amino acids of ActA, about the first 65 to 150 amino acids of ActA, about the first 65 to 125 amino acids of ActA, or about the first 65 to 110 amino acids of ActA. In some embodiments, the modified ActA comprises the first 70 to 200 amino acids of ActA, the first 80 to 150 amino acids of ActA, the first 85 to 125 amino acids of ActA, the first 90 to 110 amino acids of ActA, the first 95 to 105 amino acids of ActA, or about the first 100 amino acids of ActA. In some embodiments, the modified ActA consists of the first 70 to 200 amino acids of ActA, the first 80 to 150 amino acids of ActA, the first 85 to 125 amino acids of ActA, the first 90 to 110 amino acids of ActA, the first 95 to 105 amino acids of ActA, or about the first 100 amino acids of ActA. In some embodiments, the modified ActA comprises amino acids 1-100 of ActA. In some embodiments, the modified ActA consists of amino acids 1-100 of ActA.

In some aspects, the recombinant *Listeria* utilizing ActA-N-100 heterologous antigen fusion partner configurations is functionally linked to the said antigen fusion construct with the native *actA* promoter and 5' untranslated region (UTR) RNA. PrfA-dependent transcription from the *actA* promoter results in synthesis of a 150 nucleotide 5' UTR RNA prior to the ActA protein GUG translation initiation site. *L*. *monocytogenes* mutants deleted of the *actA* promoter 5' UTR express low levels of ActA, resulting in a phenotype characterized by absence of intracellular actin recruitment, inability to spread from cell-to-cell, and attenuated, as compared to the wild-type parent bacterium (Wong et. al. Cellular Microbiology 6:155-166).

In another aspect, a polynucleotide comprising a first nucleic acid encoding a modified ActA is operably linked and in frame with, a second nucleic acid encoding a heterologous antigen. In some embodiments, the modified ActA comprises at least the first 59 amino acids of ActA, but less than about the first 265 amino acids of ActA. In some embodiments, the modified ActA comprises the first 59 to 200 amino acids of ActA, the first 59 to 150 amino acids of ActA, the first 59 to 125 amino acids of ActA, or the first 59 to 110 amino acids of ActA. In some embodiments, the modified ActA comprises the first 70 to 200 amino acids of ActA, the first 80 to 150 amino acids of ActA, the first 85 to 125 amino acids of ActA, the first 90 to 110 amino acids of ActA, the first 95 to 105 amino acids of ActA, or about the first 100 amino acids of ActA. In some embodiments, the first nucleic acid encodes amino acids 1-100 of ActA. In some embodiments, the polynucleotide is genomic. In some alternative embodiments, the polynucleotide is plasmid based. In some embodiments, the polynucleotide is operably linked with a promoter.

In some embodiments, the *L. monocytogenes* native sequence encoding the first 100 amino acids of ActA is functionally linked in frame with a desired heterologous antigen sequence. In the some embodiments, the heterologous antigen sequence is synthesized according to the optimal codon usage of *L. monocytogenes,* a low GC percentage organism. In some embodiments, compositions utilizing the *actA* promoter together with the 5' untranslated sequences are desired.

Table 2 discloses nucleic acids and polypeptides used for making constructs that contain ActA N100 as a fusion protein partner. Sequences codon optimized for expression in *L. monocytogenes,* and non codon optimized sequences, are identified.

**Table 2. ActA sequences**

| | |
|---|---|
| Nucleic acid encoding ActA-N100 native sequence (not codon optimized), including Shine-Dalgarno sequence. (SEQ ID NO:) | GTGGGATTAAATAGATTTATGCGTGCGATGATGGTAGT TTTCATTACTGCCAACTGCATTACGATTAACCCCGACA TAATATTTGCAGCGACAGATAGCGAAGATTCCAGTCTA AACACAGATGAATGGGAAGAAGAAAAAACAGAAGAGCA GCCAAGCGAGGTAAATACGGGACCAAGATACGAAACTG CACGTGAAGTAAGTTCACGTGATATTGAGGAACTAGAA AAATCGAATAAAGTGAAAAATACGAACAAAGCAGACCT AATAGCAATGTTGAAAGCAAAAGCAGAGAAAGGT |
| ActA promoter *L. monocytogenes* 10403S. (SEQ ID NO:) | AAGCTTGGGAAGCAGTTGGGGTTAACTGATTAACAAATGTTAGAGAA AAATTAATTCTCCAAGTGATATTCTTAAAATAATTCATGAATATTTT TTCTTATATTAGCTAATTAAGAAGATAATTAACTGCTAATCCAATTT TTAACGGAATAAATTAGTGAAAATGAAGGCCGAATTTTCCTTGTTCT AAAAAGGTTGTATTAGCGTATCACGAGGAGGGAGTATAA |
| Nucleic acid encoding full-length ActA *L. monocytogenes* 10403S. (SEQ ID NO:) | GTGGGATTAAATAGATTTATGCGTGCGATGATGGTAGTTTT CATTACTGCCAACTGCATTACGATTAACCCCGACATAATAT TTGCAGCGACAGATAGCGAAGATTCCAGTCTAAACACAGA TGAATGGGAAGAAGAAAAAACAGAAGAGCAGCCAAGCGA GGTAAATACGGGACCAAGATACGAAACTGCACGTGAAGTA AGTTCACGTGATATTGAGGAACTAGAAAAATCGAATAAAG TGAAAAATACGAACAAAGCAGACCTAATAGCAATGTTGAA AGCAAAAGCAGAGAAAGGTCCGAATAACAATAATAACAAC GGTGAGCAAACAGGAAATGTGGCTATAAATGAAGAGGCTTC AGGAGTCGACCGACCAACTCTGCAAGTGGAGCGTCGTCATC CAGGTCTGTCATCGGATAGCGCAGCGGAAATTAAAAAAAGA AGAAAAGCCATAGCGTCGTCGGATAGTGAGCTTGAAAGCCT TACTTATCCAGATAAACCAACAAAAGCAAATAAGAGAAAAG TGGCGAAAGAGTCAGTTGTGGATGCTTCTGAAAGTGACTTAG |
| | ATTCTAGCATGCAGTCAGCAGACGAGTCTACACCACAACCTT TAAAAGCAAATCAAAAACCATTTTTCCCTAAAGTATTTAAAA AAATAAAAGATGCGGGAAATGGGTACGTGATAAAATCGAC GAAAATCCTGAAGTAAAGAAAGCGATTGTTGATAAAAGTGC AGGGTTAATTGACCAATTATTAACCAAAAAGAAAAGTGAAG AGGTAAATGCTTCGGACTTCCCGCCACCACCTACGGATGAAG AGTTAAGACTTGCTTTGCCAGAGACACCGATGCTTCTCGGTTT TAATGCTCCTACTCCATCGGAACCGAGCTCATTCGAATTTCCG CCGCCACCTACGGATGAAGAGTTAAGACTTGCTTTGCCAGAG ACGCCAATGCTTCTTGGTTTTAATGCTCCTGCTACATCGGAAC CGAGCTCATTCGAATTTCCACCGCCTCCAACAGAAGATGAAC TAGAAATTATGCGGGAAACAGCACCTTCGCTAGATTCTAGTT TTACAAGCGGGGATTTAGCTAGTTTGAGAAGTGCTATTAATC GCCATAGCGAAAATTTCTCTGATTTCCCACTAATCCCAACAG AAGAAGAGTTGAACGGGAGAGGCGGTAGACCAACATCTGAA GAATTTAGTTCGCTGAATAGTGGTGATTTTACAGATGACGAA AACAGCGAGACAACAGAAGAAGAAATTGATCGCCTAGCTGA TTTAAGAGATAGAGGAACAGGAAAACACTCAAGAAATGCGG GTTTTTTACCATTAAATCCATTTATTAGTAGCCCTGTTCCTTCA TTAACTCCAAAGGTACCGAAAATAAGCGCGCCGGCTCTGATA AGTGACATAACTAAAAAAGCGCCATTTAAGAATCCATCACAG CCATTAAATGTGTTTAATAAAAAAACTACAACGAAAACAGTG ACTAAAAAACCAACCCCTGTAAAGACCGCACCAAAGCTAGCA GAACTTCCTGCCACAAAACCACAAGAAACCGTACTTAGGGAA AATAAAACACCCTTTATAGAAAAACAAGCAGAAACAAACAAG CAGTCAATCAATATGCCGAGCCTACCAGTAATCCAAAAAGAA GCTACAGAGAGCGATAAAGAGGAAATGAAACCACAAACCGA GGAAAAAATGGTAGAGGAAAGCGAATCAGCTAATAACGCAA ACGGAAAAAATCGTTCTGCTGGCATTGAAGAAGGAAAACTAA TTGCTAAAAGTGCAGAAGACGAAAAAGCGAAGGAAGAACCA GGGAACCATACGACGTTAATTCTTGCAATGTTAGCTA TTGGCGTGTTCTCTTTAGGGGCGTTTATCAAAATTATT CAATTAAGAAAAAATAATTAA |
| ActA polypeptide from *L. monocytogenes* 10403S. | SLGAFIKIIQLRKNN |
| (SEQ ID NO:) | Ggtaccgggaagcagttggggttaactgattaacaaatgttagagaaa Aattaattctccaagtgatattcttaaaataattcatgaatatttttt Cttatattagctaattaagaagataattaactgctaatccaattttta Acggaataaattagtgaaaatgaaggccgaatttttccttgttctaaaa AggttgtattagcgtatcacgaggagggagtataaGTGGATTAAATA GATTTATGCGTGCGATGATGGTAGTTTTCATTACTGCCAACTGCATTA CGATTAACCCCGACATAATATTTGCAGCGACAGATAGCGAAGATTCCA GTCTAAACACAGATGAATGGGAAGAAGAAAAAACAGAAGAGCAGCCAA GCGAGGTAAATACGGACCAAGATACGAAACTGCACGTGAAGTAAGTT CACGTGATATTGAGGAACTAGAAAAATCGAATAAAGTGAAAAATACGA ACAAAGCAGACCTAATAGCAATGTTGAAAGCAAAAGCAGAGAAAGGT ggatcc |
| Amino acid sequence of ActA-N100. The nucleic acid encoding ActA-N100 contains a valine codon at the N-terminus, but the *Listeria* actually biosynthesizes a polypeptide starting with methionine, not valine. (SEQ ID NO:) | VGLNRFMRAMMVVFITANCITINPDIIFAATDSEDSS LNTDEWEEEKTEEQPSEVNTGPRYETAREVSSRDIEE LEKSNKVKNTNKADLIAMLKAKAEKG |
| ActA promoter and ActA-N100: N100 coding sequence is native. Tumor antigens are inserted at the BamHI site (GGATCC). (SEQ ID NO:) | AAGCTTGGGAAGCAGTTGGGGTTAACTGATTAACAAATGTTAGAGAAAAA TTAATTCTCCAAGTGATATTCTTAAAATAATTCATGAATATTTTTTCTTA TATTAGCTAATTAAGAAGATAATTAACTGCTAATCCAATTTTTAACGGAA TAAATTAGTGAAAATGAAGGCCGAATTTTCCTTGTTCTAAAAAGGTTGTA TTAGCGTATCACGAGGAGGGAGTATAAGTGGGATTAAATAGATTTATGCG TGCGATGATGGTAGTTTTCATTACTGCCAACTGCATTACGATTAACCCCG ACATAATATTTGCAGCGACAGATAGCGAAGATTCCAGTCTAAACACAGAT GAATGGAAGAAGAAAAAACAGAAGAGCAGCCAAGCGAGGTAAATACGGG ACCAAGATACGAAACTGCACGTGAAGTAAGTTCACGTGATATTGAGGAAC TAGAAAAATCGAATAAAGTGAAAAATACGAACAAAGCAGACCTAATAGCA ATGTTGAAAGCAAAAGCAGAGAAAGGTGGATCC |
| Amino acid sequence of ActAN100: the BamHI site adds two amino acids (GS). (SEQ ID NO:) | VGLNRFMRAMMVVFITANCITINPDIIFAATDSEDSSLNTDEWEEEK TEEQPSEVNTGPRYETAREVSSRDIEELEKSNKVKNTNKADLIAMLK AKAEKGGS |

Other examples of signal peptides include but are not limited to an LLO signal peptide from *Listeria monocytogenes,* a Usp45 signal peptide from *Lactococcus lactis,* a Protective Antigen signal peptide from *Bacillus anthracis,* a p60 signal peptide from *Listeria monocytogenes,* a PhoD signal peptide from *Bacillus subtilis*.

Table 3 discloses nucleic acids and polypeptides used for making constructs that contain LLO and BaPa as fusion protein partners. Sequences codon optimized for expression in *L. monocytogenes,* and non codon optimized sequences, are identified.

**Table 3 LLO and BaPa sequences**

| | |
|---|---|
| Nucleic acid of LLO open reading frame (ORF) from wild type *Listeria* 10403S. (SEQ ID NO:) | atgaaaaaataatgctagtttttattacacttatattagttagtcta ccaattgcgcaacaaactgaagcaaaggatgcatctgcattcaataaa gaaaattcaatttcatccatggcaccaccagcatctccgcctgcaagt cctaagacgccaatcgaaaagaaacacgcggatgaaatcgataagtat atacaaggattggattacaataaaaacaatgtattagtataccacgg agatgcagtgacaaatgtgccgccaagaaaaggttacaaagatggaa atgaatatattgttggagaaaaagaagaaatccatcaatcaaaat aatgcagacattcaagttgtgaatgcaatttcgagcctaacctatcc aggtgctctcgtaaaagcgaattcggaattagtagaaaatcaaccag atgttctccctgtaaaacgtgattcattaacactcagcattgatttg ccaggtatgactaatcaagacaataaaatcgttgtaaaaaatgccac taaatcaaacgttaacaacgcagtaaatacattagtggaaagatgga atgaaaaatatgctcaagcttatccaaatgtaagtgcaaaaattgat tatgatgacgaaatggcttacagtgaatcacaattaattgcgaaatt tggtacagcatttaaagctgtaaataatagcttgaatgtaaacttcg gcgcaatcagtgaagggaaaatgcaagaagaagtcattagttttaaa caaatttactataacgtgaatgttaatgaacctacaagaccttccag atttttcggcaaagctgttactaaagagcagttgcaagcgcttggag tgaatgcagaaaatcctcctgcatatatctcaagtgtggcgtatggc cgtcaagtttatttgaaattatcaactaattcccatagtactaaagt aaaagctgcttttgatgctgccgtaagcggaaaatctgtctcaggtg atgtagaactaacaaatatcatcaaaaattcttccttcaaagccgta atttacggaggttccgcaaaagatgaagttcaaatcatcgacggcaa cctcggagacttacgcgatattttgaaaaaaggcgctacttttaatc gagaaacaccaggagttcccattgcttatacaacaaacttcctaaaa gacaatgaattagctgttattaaaaacaactcagaatatattgaaac aacttcaaaagcttatacagatggaaaaattaacatcgatcactctg gaggatacgttgctcaattcaacatttcttgggatgaagtaaattat gatcctgaaggtaacgaaattgttcaacataaaaactggagcgaaaa caataaaagcaagctagctcatttcacatcgtccatctatttgcctg gtaacgcgagaaatattaatgtttacgctaaagaatgcactggttta gcttgggaatggtggagaacggtaattgatgaccggaacttaccact tgtgaaaaatagaaatatctccatctgggcaccacgctttatccga aatatagtaataaagtagataatccaatcgaataa |
| Codon optimized LLO (GGATCC is a BamHI site added at the 3' end for in-frame fusions). (SEQ ID NO:) | atgaaaaaataatgctagtctttattacattaattttagtaagtctaccaa ttgca caacaaaccgaagctaaagatgcatcagcgttcaacaaagaaattcaatta gttca atggccccaccagcttctccaccagcatctccaaaaacaccaattgaaaaa aacat gcagacgaaattgataaatatattcaaggtttagattacaataagaataacg tttta gtataccacggcgatgcagtaacaaatgtacctccaagaaaaggctataaag acgga aatgaatatattgttgttgaaaaaaaaagaaatctattaatcaaaacaatg ccgac atccaagtagttaacgcgattagctcattgacgtatccaggcgcccttgtaa aagct aactctgaattagtggaaaatcaaccagacgtacttccagtcaaacgtgata gtcta accttaagtattgatttaccaggaatgacaaatcaagataacaaaattgttg ttaaa aatgcaactaaatccaatgtaaataatgcagttaacacattagtagaacgat |
| | ggaac gaaaaatacgcacaggcatacccaaatgtatcagctaaaattgattacgacg acgaa atggcctactcagaaagtcaattaattgctaaatttggtacagcattcaaag cagtc aataatagtttaaatgtaaattttggagcgatctctgaaggaaagatgcagg aagaa gtaatttcattcaaacaaatttattataatgttaacgtaaatgaaccaaccc gtcct tcccgtttctttggcaaagcagttactaaagaacaattacaagcactaggtg tgaat gcagaaaacccaccggcatatatttcaagcgtcgcttacggacgacaagttt actta aaattatctacaaacagtcatagtacaaaagtaaaagcagcattcgatgcag ctgtg tcaggaaaatcagttagtggagatgtagaattaaccaatattattaaaaatt cgagt tttaaagctgttatttatggaggttctgcaaaagatgaagtacaaattattg acgga aacttaggcgatttacgtgacattttaaaaaaaggcgcaacatttaatagag aaaca ccaggggttccaattgcttatacaactaattttcttaaagataatgaacttg cagta attaaaaacaattcagaatacattgaaacaacttcgaaagcatatacagacg gaaaa attaatattgatcactcaggagggtacgttgcacaatttaatattagttggg atgaa gtaaactatgatccagaaggcaatgaaattgtacaacataaaaattggtctg aaaat aacaaatctaaactagcacactttaccagttctatctatttaccaggaaatg ctcgc aatattaatgtttacgcaaaagaatgtaccggattagcatgggaatggtggc gcaca gttattgacgaccgcaatcttcctctagtaaaaaacagaaacatcagcattt gggga acaacgctttatccgaaatacagtaataaagttgataatccaattgaa GGATCC |
| One mutant variation on codon optimized LLO (as a translational fusion - GGATCC is a BamHI site added at the 3' end for in-frame fusions; mutant variation is in CAPS, | atgaaaaaaataatgctagtctttattacattaattttagtaagtctaccaa ttgc acaacaaaccgaagctaaagatgcatcagcgttcaacaaagaaaattcaatt agtt caatggccccaccagcttctccaccagcatctccaaaaacaccaattgaaaa aaaa catgcagacgaaattgataaatatattcaaggtttagattacaataagaata acgt tttagtataccacggcgatgcagtaacaaatgtacctccaagaaaaggctat aaag acggaaatgaatatattgttgttgaaaaaaaaagaaatctattaatcaaaa caat gccgacatccaagtagttaacgcgattagctcattgacgtatccaggcgccc ttgt aaaagctaactctgaattagtggaaaatcaaccagacgtacttccagtcaaa cgtg atagtctaaccttaagtattgatttaccaggaatgacaaatcaagataacaa |
| changes TGGTGG to TTTTTT amino acid changes WW to FF). (SEQ ID NO:) | aatt gttgttaaaaatgcaactaaatccaatgtaaataatgcagttaacacattag taga acgatggaacgaaaaatacgcacaggcatacccaaatgtatcagctaaaatt gatt acgacgacgaaatggcctactcagaaagtcaattaattgctaaatttggtac agca ttcaaagcagtcaataatagtttaaatgtaaattttggagcgatctctgaag gaaa gatgcaggaagaagtaatttcattcaaacaaatttattataatgttaacgta aatg aaccaacccgtccttcccgtttctttggcaaagcagttactaaagaacaatt acaa gcactaggtgtgaatgcagaaaacccaccggcatatatttcaagcgtcgctt acgg acgacaagtttacttaaaattatctacaaacagtcatagtacaaaagtaaaa gcag cattcgatgcagctgtgtcaggaaaatcagttagtggagatgtagaattaac caat attattaaaaattcgagttttaaagctgttatttatggaggttctgcaaaag atga agtacaaattattgacggaaacttaggcgatttacgtgacattttaaaaaaa ggcg caacatttaatagagaaacaccaggggttccaattgcttatacaactaattt tctt aaagataatgaacttgcagtaattaaaaacaattcagaatacattgaaacaa cttc gaaagcatatacagacggaaaaattaatattgatcactcaggaggtacgtt gcac aatttaatattagttgggatgaagtaaactatgatccagaaggcaatgaaat tgta caacataaaaattggtctgaaaataacaaatctaaactagcacactttacca gttc tatctatttaccaggaaatgctcgcaatattaatgtttacgcaaaagaatgt accg gattagcatgggaatttttcgcacagttattgacgaccgcaatcttcctct agta aaaaacagaaacatcagcatttggggaacaacgctttatccgaaatacagta ataa agttgataatccaattgaa GGATCC |
| Nucleic acid of LL059 (not codon optimized). (SEQ ID NO:) | ATGAAAAAAATAATGCTAGTTTTTATTACACTTATATT AGTTAGTCTACCAATTGCGCAACAAACTGAAGCAAAGG ATGCATCTGCATTCAATAAAGAAAATTCAATTTCATCC ATGGCACCACCAGCATCTCCGCCTGCAAGTCCTAAGAC GCCAATCGAAAAGAAACACGCGGAT |
| Nucleic acid of LLO59, codon optimized for expression in *Listeria.* (SEQ ID NO:) | ATGAAAAAAATTATGTTAGTTTTTATTACATTAATTTT AGTTAGTTTACCAATTGCACAACAAACAGAAGCAAAAG ATGCAAGTGCATTTAATAAAGAAAATAGTATTAGTAGT ATGGCACCACCAGCAAGTCCACCAGCAAGTCCAAAAAC ACCAATTGAAAAAAAACATGCAGAT |
| Amino acids of LLO59. (SEQ ID NO:) | MKKIMLVFITLILVSLPIAQQTEAKDASAFNKENSISS MAPPASPPASPKTPIEKKHAD |
| *hly* promoter. (SEQ ID NO:) | GGTACCTCCTTTGATTAGTATATTCCTATCTTAAAGTTACT TTTATGTGGAGGCATTAACATTTGTTAATGACGTCAAAAGG ATAGCAAGACTAGAATAAAGCTATAAAGCAAGCATATAATA TTGCGTTTCATCTTTAGAAGCGAATTTCGCCAATATTATAA TTATCAAAAGAGAGGGGTGGCAAACGGTATTTGGCATTATT AGGTTAAAAAATGTAGAAGGAGAGTGAAACCC |
| Nucleic acid for codon-optimized BaPA signal peptide. (SEQ ID NO:) | ATGAAAAAACGTAAAGTTTTAATTCCATTAATGGCATTAAGTACAA TTTTAGTTAGTAGTACAGGTAATTTAGAAGTTATTCAAGCAGAAGT TGGATCC |
| Amino acids of BaPA signal peptide. (SEQ ID NO:) | MKKRKVLIPLMALSTILVSSTGNLEVIQAEVGS |
| The *hly* promoter and BaPA signal peptide are fused seamlessly together. The *hly* promoter and BaPA signal peptide are fused seamlessly together (no restriction sites) and the promoter-signal peptide assembly is inserted into plasmids as a *KpnI* (GGTACC)-*BamHI* | GGTACCTCCTTTGATTAGTATATTCCTATCTTAAAGTTACTTTTATGT GG AGGCATTAACATTTGTTAATGACGTCAAAAGGATAGCAAGACTAGAAT AA AGCTATAAAGCAAGCATATAATATTGCGTTTCATCTTTAGAAGCGAAT TT CGCCAATATTATAATTATCAAAAGAGAGGGGTGGCAAACGGTATTTGG CA TTATTAGGTTAAAAAATGTAGAAGGAGAGTGAAACCCATGAAAAAACG TA AAGTTTTAATTCCATTAATGGCATTAAGTACAATTTTAGTTAGTAGTA CA GGTAATTTAGAAGTTATTCAAGCAGAAGTTGGATCC |
| (GGATCC) fragment. The tumor antigen is inserted at the *BamHI* site. (SEQ ID NO:) | |

Bacteria utilize diverse pathways for protein secretion, including secA1 secA2, and Twin-Arg Translocation (Tat). Which pathway is utilized is largely determined by the type of signal sequence located at the N-terminal end of the pre-protein. The majority of secreted proteins utilize the Sec pathway, in which the protein translocates through the bacterial membrane-embedded proteinaceous Sec pore in an unfolded conformation. In contrast, the proteins utilizing the Tat pathway are secreted in a folded conformation. Nucleotide sequence encoding signal peptides corresponding to any of these protein secretion pathways can be fused genetically in-frame to a desired heterologous protein coding sequence. The signal peptides optimally contain a signal peptidase cleavage site at their carboxyl terminus for release of the authentic desired protein into the extra-cellular environment (Sharkov and Cai. 2002 J. Biol. Chem. 277:5796-5803; Nielsen et. al. 1997 Protein Engineering 10:1-6; and, www.cbs.dtu.dk/services/SignalP/).

The signal peptides used in the polynucleotides can be derived not only from diverse secretion pathways, but also from diverse bacterial genera. Signal peptides generally have a common structural organization, having a charged N-terminus (N-domain), a hydrophobic core region (H-domain) and a more polar C-terminal region (C-domain), however, they do not show sequence conservation. In some embodiments, the C-domain of the signal peptide carries a type I signal peptidase (SPase I) cleavage site, having the consensus sequence A-X-A, at positions -1 and -3 relative to the cleavage site. Proteins secreted via the sec pathway have signal peptides that average 28 residues. The secA2 protein secretion pathway was first discovered in *Listeria monocytogenes*; mutants in the secA2 paralogue are characterized by a rough colony phenotype on agar media, and an attenuated virulence phenotype in mice (Lenz and Portnoy, 2002 Mol. Microbiol. 45:1043-1056; and, Lenz et. al 2003 PNAS 100:12432-12437). Signal peptides related to proteins secreted by the Tat pathway have a tripartite organization similar to Sec signal peptides, but are characterized by having an RR-motif (R-R-X-#-#, where # is a hydrophobic residue), located at the N-domain / H-domain boundary. Bacterial Tat signal peptides average 14 amino acids longer than sec signal peptides. The *Bacillus subtilis* secretome may contain as many as 69 putative proteins that utilize the Tat secretion pathway, 14 of which contain a SPase I cleavage site (Jongbloed et. al. 2002 J. Biol. Chem. 277:44068-44078; Thalsma et. al., 2000 Microbiol. Mol. Biol. Rev. 64:515-547).

### IV. Heterologous polypeptides and polynucleotides encoding the heterologous polypeptides

"Heterologous polypeptides" that are encoded by polynucleotides within the *Listeria* and/or expressed by the *Listeria* are heterologous with respect to the *Listeria.* In certain embodiments, the heterologous polypeptides are non-listerial. In certain embodiments, the heterologous polypeptides are not found in *Listeria* in nature in either the genomic DNA or in any bacteriophage that has infected the *Listeria.* In some embodiments, the polynucleotides encoding the heterologous polypeptide(s) are recombinant. In certain embodiments, the heterologous polypeptides are non-bacterial.

In some embodiments, where the polynucleotide encoding the heterologous polypeptide is to be expressed within the *Listeria,* operably linked promoters capable of directing expression in *Listeria* are preferred. In some embodiments, the promoters are prokaryotic (e.g., *Listeria* promoters such as the *hly* or *actA* promoters). In some embodiments, the polynucleotides encoding the heterologous antigen are codon-optimized for expression in *Listeria* (see, e.g., U.S. Patent Publication No. 2005/0249748).

In some embodiments, the heterologous polypeptides which are delivered or which are encoded by the nucleic acids that are delivered by the *Listeria* of the invention into cells (e.g., mammalian cells) comprise an antigen. In some embodiments, the antigen is a tumor antigen (e.g., a human tumor antigen), or an antigenic fragment or variant thereof. In some alternative embodiments, the antigen is an antigen from an infectious agent, or an antigenic fragment or variant thereof.

The recombinant nucleic acid molecules described herein, as well as the expression cassettes or expression vectors described herein, can be used to encode any desired polypeptide. In particular, the recombinant nucleic acid molecules, expression cassettes, and expression vectors are useful for expressing heterologous polypeptides in a bacterium.

In some embodiments (depending on the recombinant nucleic acid molecule, expression cassette or expression vector used), the polypeptide encoded by a polynucleotide is encoded as part of a fusion protein with a signal peptide. In other embodiments, the encoded polypeptide is encoded as a discrete polypeptide by the recombinant nucleic acid molecule. In still other embodiments, the polypeptide encoded by a polynucleotide of the recombinant nucleic acid molecule is encoded as part of a fusion protein that does not include a signal peptide, but does include the recombinant nucleic acid molecule. In still other embodiments, the polypeptide encoded by a polynucleotide of the recombinant nucleic acid molecule is encoded as part of a fusion protein (also referred to herein as a protein chimera) in which the polypeptide is embedded within another polypeptide sequence.

Thus, it is understood that each of the polypeptides listed herein (below and elsewhere) which are encoded by polynucleotides of the recombinant nucleic acid molecules of the disclosure may be expressed as either fusion proteins (fused to signal peptides and/or to or in other polypeptides) or as discrete polypeptides by the recombinant nucleic acid molecule, depending on the particular recombinant nucleic acid molecule.

In some embodiments, the polypeptide is part of a fusion protein encoded by the recombinant nucleic acid molecule and is heterologous to the signal peptide of the fusion protein. In some embodiments, the polypeptide is positioned in another polypeptide sequence to which it is heterologous.

In some embodiments, the polypeptide is bacterial (either Listerial or non-Listerial). In some embodiments, the polypeptide is not bacterial. In some embodiments, the polypeptide encoded by the polynucleotide is a mammalian polypeptide. For instance, the polypeptide may correspond to a polypeptide sequence found in humans (i.e., a human polypeptide). In some embodiments, the polypeptide is Listerial. In some embodiments, the polypeptide is non-Listerial. In some embodiments, the polypeptide is not native (i.e., is foreign) to the bacterium in which the recombinant nucleic acid molecule is to be incorporated or is incorporated.

In some embodiments, the polynucleotide encoding the polypeptide is codon-optimized for expression in a bacterium. In some embodiments, the polynucleotide encoding the polypeptide is fully codon-optimized for expression in a bacterium. In some embodiments, the polypeptide which is encoded by the codon-optimized polynucleotide is foreign to the bacterium (i.e., is heterologous to the bacterium).

The term "polypeptide" is used interchangeably herein with "peptide" and "protein" and no limitation with respect to the length or size of the amino acid sequence contained therein is intended. Typically, however, the polypeptide will comprise at least about 6 amino acids. In some embodiments, the polypeptide will comprise, at least about 9, at least about 12, at least about 20, at least about 30, or at least about 50 amino acids. In some embodiments, the polypeptide comprises at least about 100 amino acids. In some embodiments, the polypeptide is one particular domain of a protein (e.g., an extracellular domain, an intracellular domain, a catalytic domain, or a binding domain). In some embodiments, the polypeptide comprises an entire (i.e., full-length) protein.

In some embodiments, the polypeptide that is encoded by a polynucleotide of a recombinant nucleic acid molecule is an antigen or a protein that provides a palliative treatment for a disease. In some embodiments, the polypeptide that is encoded is a therapeutic protein.

In some embodiments, the polypeptide that is encoded by a polynucleotide of a recombinant nucleic acid molecule is an antigen. In some embodiments, the antigen is a bacterial antigen. In some embodiments, the antigen is a non-Listerial bacterial antigen. In some embodiments, however, the antigen is a non-Listerial antigen. In other embodiments, the antigen is a non-bacterial antigen. In some embodiments, the antigen is a mammalian antigen. In some embodiments, the antigen is a human antigen. In some embodiments, the polypeptide is an antigen comprising one or more immunogenic epitopes. In some embodiments, the antigen comprises one or more MHC class I epitopes. In other embodiments, the antigen comprises one or more MHC class II epitope. In some embodiments, the epitope is a CD4+ T-cell epitope. In other embodiments, the epitope is a CD8+ T-cell epitope.

The polynucleotide encoding an antigen is not limited to any exact nucleic acid sequence (i.e., that encoding a naturally occurring, full-length antigen) but can be of any sequence that encodes a polypeptide that is sufficient to elicit the desired immune response when administered to an individual within the bacteria or compositions of the invention. The term "antigen," as used herein, is also understood to include fragments of larger antigen proteins so long as the fragments are antigenic (i.e., immunogenic). In addition, in some embodiments, the antigen encoded by a polynucleotide of the recombinant nucleic acid may be a variant of a naturally occurring antigen sequence. (Similarly for polynucleotides encoding other, non-antigen proteins, the sequences of the polynucleotides encoding a given protein may vary so long as the desired protein that is expressed provides the desired effect (e.g. a palliative effect) when administered to an individual.)

An antigen that is derived from another antigen includes an antigen that is an antigenic fragment of the other antigen, an antigenic variant of the other antigen, or an antigenic variant of a fragment of the other antigen. A variant of an antigen includes antigens that differ from the original antigen in one or more substitutions, deletions, additions, and/or insertions.

The antigenic fragment may be of any length, but is most typically at least about 6 amino acids, at least about 9 amino acids, at least about 12 amino acids, at least about 20 amino acids, at least about 30 amino acids, at least about 50 amino acids, or at least about 100 amino acids. An antigenic fragment of an antigen comprises at least one epitope from the antigen. In some embodiments, the epitope is a MHC class I epitope. In other embodiments, the epitope is a MHC class II epitope. In some embodiments, the epitope is a CD4+ T-cell epitope. In other embodiments, the epitope is a CD8+ T-cell epitope.

A variety of algorithms and software packages useful for predicting antigenic regions (including epitopes) within proteins are available to those skilled in the art. For instance, algorthims that can be used to select epitopes that bind to MHC class I and class II molecules are publicly available. For instance, the publicly available "SYFPEITHI" algorithm can be used to predict MHC-binding peptides (Rammensee et al. (1999) Immunogenetics 50:213-9). For other examples of publicly available algorithms, see the following references: Parker et al. (1994) J. Immunol 152:163-75; Singh and Raghava (2001) Bioinformatics 17:1236-1237; Singh and Raghava (2003) Bioinformatics 19:1009-1014; Mallios (2001) Bioinformatics 17:942-8; Nielsen et al. (2004) Bioinformatics 20:1388-97; Donnes et al. (2002) BMC Bioinformatics 3:25; Bhasin, et al. (2004) Vaccine 22:3195-204; Guan et al. (2003) Nucleic Acids Res 31:3621-4; Reche et al. (2002) Hum. Immunol. 63:701-9; Schirle et al. (2001) J. Immunol. Methods 257:1-16; Nussbaum et al. (2001) Immunogenetics (2001) 53:87-94; Lu et al. (2000) Cancer Res. 60:5223-7. See also, e.g., Vector NTI® Suite (Informax, Inc, Bethesda, MD), GCG Wisconsin Package (Accelrys, Inc., San Diego, CA), Welling, et al. (1985) FEBS Lett. 188:215-218, Parker, et al. (1986) Biochemistry 25:5425-5432, Van Regenmortel and Pellequer (1994) Pept. Res. 7:224-228, Hopp and Woods (1981) Proc. Natl. Acad. Sci. USA 78:3824-3828, and Hopp (1993) Pept. Res. 6:183-190. Some of the algorthims or software packages discussed in the references listed above in this paragraph are directed to the prediction of MHC class I and/or class II binding peptides or epitopes, others to identification of proteasomal cleavage sites, and still others to prediction of antigenicity based on hydrophilicity.

Once a candidate antigenic fragment believed to contain at least one epitope of the desired nature has been identified, the polynucleotide sequence encoding that sequence can be incorporated into an expression cassette and introduced into a *Listeria* vaccine vector or other bacterial vaccine vector. The immunogenicity of the antigenic fragment can then be confirmed by assessing the immune response generated by the *Listeria* or other bacteria expressing the fragments. Standard immunological assays such as ELISPOT assays, Intracellular Cytokine Staining (ICS) assay, cytotoxic T-cell activity assays, or the like, can be used to verify that the fragment of the antigen chosen maintains the desired immunogenicity. In addition, the anti-tumor efficacy of the *Listeria* and/or bacterial vaccines can also be assessed using the known methods; for example, implantation of CT26 murine colon cells expressing the antigen fragment in mice, followed by vaccination of the mice with the candidate vaccine and observation of effect on tumor size, metastasis, survival, etc. relative to controls and/or the full-length antigen.

In addition, large databases containing epitope and/or MHC ligand information using for identifying antigenic fragments are publicly available. See, e.g., Brusic et al. (1998) Nucleic Acids Res. 26:368-371; Schonbach et al. (2002) Nucleic Acids Research 30:226-9; and Bhasin et al. (2003) Bioinformatics 19:665-666; and Rammensee et al. (1999) Immunogenetics 50:213-9.

The amino acid sequence of an antigenic variant has at least about 60%, at least about 70%, at least about 80%, at least about 90%, at least about 95%, or at least about 98% identity to the original antigen.

In some embodiments, the antigenic variant is a conservative variant that has at least about 80% identity to the original antigen and the substitutions between the sequence of the antigenic variant and the original antigen are conservative amino acid substitutions. The following substitutions are considered conservative amino acid substitutions: valine, isoleucine, or leucine are substituted for alanine; lysine, glutamine, or asparagine are substituted for arginine; glutamine, histidine, lysine, or arginine are substituted for asparagine; glutamic acid is substituted for aspartic acid; serine is substituted for cysteine; asparagine is substituted for glutamine; aspartic acid is substituted for glutamic acid; proline or alanine is substituted for glycine; asparagine, glutamine, lysine or arginine is substituted for histidine; leucine, valine, methionine, alanine, phenylalanine, or norleucine is substituted for isoleucine; norleucine, isoleucine, valine, methionine, alanine, or phenylalanine is substituted for leucine; arginine, glutamine, or asparagine is substituted for lysine; leucine, phenylalanine, or isoleucine is substituted for methionine; leucine, valine, isoleucine, alanine, or tyrosine is substituted for phenylalanine; alanine is substituted for proline; threonine is substituted for serine; serine is substituted for threonine; tyrosine or phenylalanine is substituted for tryptophan; tryptophan, phenylalanine, threonine, or serine is substituted for tyrosine; tryptophan, phenylalanine, threonine, or serine is substituted for tyrosine; isoleucine, leucine, methionine, phenylalanine, alanine, or norleucine is substituted for valine. In some embodiments, the antigenic variant is a conservative variant that has at least about 90% identity to the original antigen.

In some embodiments, an antigen encoded by a recombinant nucleic acid molecule that is derived from another antigen is substantially equivalent to the other antigen. An antigen derived from another antigen is substantially equivalent to the original antigen from which it is derived if the antigen if the derived antigen has at least about 70% identity in amino acid sequence to the original antigen and maintains at least about 70% of the immunogenicity of the original antigen. In some embodiments, the substantially equivalent antigen has at least about 80%, at least about 90%, at least about 95%, or at least about 98% identity in amino acid sequence to the original antigen. In some embodiments, the substantially equivalent antigen comprises only conservative substitutions relative to the original antigen. In some embodiments, the substantially equivalent antigen maintains at least about 80%, at least about 90%, or at least about 95% of the immunogenicity of the original antigen. To determine the immunogenicity of a particular derived antigen and compare to that of the original antigen to determine whether the derived antigen is substantially equivalent to the original antigen, one can test both the derived and original antigen in any of a number of immunogenicity assays known to those skilled in the art. For instance, *Listeria* expressing either the original antigen or the derived antigen can be prepared as described herein. The ability of those *Listeria* expressing the different antigens to produce an immune response can be measured by vaccinating mice with the *Listeria* and then assessing the immunogenic response using the standard techniques of ELISPOT assays, Intracellular Cytokine Staining (ICS) assay, cytotoxic T-cell activity assays, or the like.

In some embodiments, the antigen encoded by the recombinant nucleic acid molecule is a tumor-associated antigen or is an antigen that is derived from a tumor-associated antigen. In some embodiments, the antigen is a tumor-associated antigen.

In some embodiments, the recombinant nucleic acid molecule encodes an antigen that is not identical to a tumor-associated antigen, but rather is derived from a tumor-associated antigen. For instance, in some embodiments, the antigen encoded by a polynucleotide of a recombinant nucleic acid molecule may comprise a fragment of a tumor-associated antigen, a variant of a tumor-associated antigen, or a variant of a fragment of a tumor-associated antigen. In some cases, an antigen, such as a tumor antigen, is capable of inducing a more significant immune response in a vaccine when the amino acid sequence differs slightly from that endogenous to a host. In other cases, the derived antigen induces a less significant immune response than the original antigen, but is, for instance, more convenient for heterologous expression in a *Listeria* vaccine vector due to a smaller size. In some embodiments, the amino acid sequence of a variant of a tumor-associated antigen, or a variant of a fragment of a tumor-associated antigen, differs from that of the tumor-associated antigen, or its corresponding fragment, by one or more amino acids. The antigen derived from a tumor-associated antigen will comprise at least one epitope sequence capable of inducing the desired immune response upon expression of the polynucleotide encoding the antigen within a host.

Accordingly, in some embodiments, a polynucleotide in the recombinant nucleic acid molecule encodes an antigen that is derived from a tumor-associated antigen, wherein the antigen comprises at least one antigenic fragment of a tumor-associated antigen. The antigenic fragment comprises at least one epitope of the tumor-associated antigen. In some embodiments, the antigen that is derived from another antigen is an antigenic (i.e., immunogenic) fragment or an antigenic variant of the other antigen. In some embodiments, the antigen is an antigenic fragment of the other antigen. In some embodiments, the antigen is an antigenic variant of the other antigen.

A large number of tumor-associated antigens that are recognized by T cells have been identified (Renkvist et al., Cancer Immunol Innumother 50:3-15 (2001)). These tumor-associated antigens may be differentiation antigens (e.g., PSMA, Tyrosinase, gp100), tissue-specific antigens (e.g. PAP, PSA), developmental antigens, tumor-associated viral antigens (e.g. HPV 16 E7), cancer-testis antigens (e.g. MAGE, BAGE, NY-ESO-1), embryonic antigens (e.g. CEA, alpha-fetoprotein), oncoprotein antigens (e.g. Ras, p53), over-expressed protein antigens (e.g. ErbB2 (Her2/Neu), MUC1), or mutated protein antigens. The tumor-associated antigens that may be encoded by the heterologous nucleic acid sequence include, but are not limited to, 707-AP, Annexin II, AFP, ART-4, BAGE, β-catenin/m, BCL-2, bcr-abl, bcr-abl p190, bcr-abl p210, BRCA-1, BRCA-2, CAMEL, CAP-1, CASP-8, CDC27/m, CDK-4/m, CEA (Huang et al., ExperRev. Vaccines (2002)1:49-63), CT9, CT10, Cyp-B, Dek-cain, DAM-6 (MAGE-B2), DAM-10 (MAGE-B1), EphA2 (Zantek et al., Cell Growth Differ. (1999) 10:629-38; Carles-Kinch et al., Cancer Res. (2002) 62:2840-7), ELF2M, EphA2 (Zantek et al., Cell Growth Differ. (1999) 10:629-38; Carles-Kinch et al., Cancer Res. (2002) 62:2840-7), ETV6-AML1, G250, GAGE-1, GAGE-2, GAGE-3, GAGE-4, GAGE-5, GAGE-6, GAGE-7B, GAGE-8, GnT-V, gp100, HAGE, HER2/neu, HLA-A*0201-R170I, HPV-E7, H-Ras, HSP70-2M, HST-2, hTERT, hTRT, iCE, inhibitors of apoptosis (e.g. survivin), KIAA0205, K-Ras, 12-K-Ras (K-Ras with codon 12 mutation), LAGE, LAGE-1, LDLR/FUT, MAGE-1, MAGE-2, MAGE-3, MAGE-6, MAGE-A1, MAGE-A2, MAGE-A3, MAGE-A4, MAGE-A6, MAGE-A10, MAGE-A12, MAGE-B5, MAGE-B6, MAGE-C2, MAGE-C3, MAGE-D, MART-1, MART-1/Melan-A, MC1R, MDM-2, mesothelin, Myosin/m, MUC1, MUC2, MUM-1, MUM-2, MUM-3, neo-polyA polymerase, NA88-A, N-Ras, NY-ESO-1, NY-ESO-1a (CAG-3), PAGE-4, PAP, Proteinase 3 (PR3) (Molldrem et al., Blood (1996) 88:2450-7; Molldrem et al., Blood (1997) 90:2529-34), P15, p190, Pm1/RARα, PRAME, PSA, PSM, PSMA, RAGE, RAS, RCAS1, RU1, RU2, SAGE, SART-1, SART-2, SART-3, SP17, SPAS-1, TEL/AML1, TPI/m, Tyrosinase, TARP, TRP-1 (gp75), TRP-2, TRP-2/INT2, WT-1, and alternatively translated NY-ESO-ORF2 and CAMEL proteins, derived from the NY-ESO-1 and LAGE-1 genes.

The antigen encoded by the polynucleotide in the recombinant nucleic acid molecule may encompass any tumor-associated antigen that can elicit a tumor-specific immune response, including antigens yet to be identified. The recombinant nucleic acid can also encode more than one tumor-associated antigen.

In some embodiments, the antigen is mesothelin (Argani et al., Clin Cancer Res. 7(12):3862-8 (2001)), Sp17 (Lim et al., Blood 97(5):1508-10 (2001)), gp100 (Kawakami et al., Proc. Natl. Acad. Sci. USA 91:6458 (1994)), PAGE-4 (Brinkmann et al., Cancer Res. 59(7):1445-8 (1999)), TARP (Wolfgang et al., Proc. Natl. Acad. Sci. USA 97(17):9437-42 (2000)), EphA2 (Tatsumi et al., Cancer Res. 63(15):4481-9 (2003)), PR3 (Muller-Berat et al., Clin. Immunol. Immunopath. 70(1):51-9 (1994)), prostate stem cell antigen (PSCA) (Reiter et al., Proc. Natl. Acad. Sci., 95:1735-40 (1998); Kiessling et al., Int. J. Cancer, 102:390-7 (2002)), or SPAS-1 (U.S. Patent Application Publication No. 2002/0150588).

In some embodiments of the invention, the antigen encoded by the recombinant nucleic acid molecule or expression cassette is CEA. In other embodiments, the antigen is an antigenic fragment and/or antigenic variant of CEA. CEA is a 180-kDA membrane intercellular adhesion glycoprotein that is over-expressed in a significant proportion of human tumors, including 90% of colorectal, gastric, and pancreatic, 70% of non-small cell lung cancer, and 50% of breast cancer (Hammarstrom, Semin. Cancer Biol., 9:67-81). A variety of immunotherapeutics such as anti-idiotype monoclonal antibody mimicking CEA (Foon et al., Clin. Cancer Res., 87:982-90 (1995), or vaccination using a recombinant vaccinia virus expressing CEA (Tsang et al., J. Natl. Cancer Inst., 87:982-90 (1995)) have been investigated, unfortunately, however, with limited success. Nonetheless, investigators have identified a HLA*0201-restricted epitope, CAP-1(CEA605-613), that is recognized by human T cell lines that were generated from vaccinated patients. Vaccination of patients with DC pulsed with this epitope failed to induce clinical responses (Morse et al., Clin. Cancer Res., 5:1331-8 (1999)). Recently, a CEA605-613 peptide agonist was identified with a heteroclitic aspartate to asparagine substitution at position 610 (CAP1-6D). Although this amino acid substitution did not alter MHC binding affinity of this peptide, the use of the altered peptide ligand (APL) resulted in improved generation of CEA-specific cytotoxic T lymphocytes (CTL) in vitro. CAP1-6D-specific CTL maintained their ability to recognize and lyse tumor cells expressing native CEA (Zaremba et al., Cancer Res., 57: 4570-7 (1997); Salazar et al., Int. J. Cancer, 85:829-38 (2000)). Fong et al. demonstrated induction of CEA-specific immunity in patients with colon cancer vaccinated with Flt3-ligand expanded DC incubated with this APL. Encouragingly, 2 of 12 patients after vaccination experienced dramatic tumor regressions that correlated with the induction of peptide-MHC tetramer+ T cells (Fong et al., Proc. Natl. Acad. Sci. U.S.A., 98:8809-14 (2001)).

In another embodiment, the antigen encoded by the recombinant nucleic acid molecule is an antigen that is proteinase-3 or is derived from proteinase-3. For instance, in one embodiment, the antigen comprises the HLA-A2.1-restricted peptide PR1 (aa 169-177; VLQELNVTV). Information on proteinase-3 and/or the PR1 epitope is available in the following references: US Patent No. 5,180,819, Molldrem, et al., Blood, 90:2529-2534 (1997); Molldrem et al., Cancer Research, 59:2675-2681 (1999); Molldrem, et al., Nature Medicine, 6:1018-1023 (2000); and Molldrem et al., Oncogene, 21: 8668-8673 (2002).

In some embodiments, the antigen encoded by the recombinant nucleic acid molecule or expression cassette is an antigen selected from the group consisting of K-Ras, H-Ras, N-Ras, 12-K-Ras, mesothelin, PSCA, NY-ESO-1, WT-1, survivin, gp100, PAP, proteinase 3, SPAS-1, SP-17, PAGE-4, TARP, B-raf, tyrosinase, mdm-2, MAGE, RAGE, MART-1, bcr/abl, Her-2/neu, alphafetoprotein, mammoglobin, hTERT(telomerase), PSA, or CEA. In some embodiments, the antigen is K-Ras. In some embodiments, the antigen is H-Ras. In some embodiments, the antigen is N-Ras. In some embodiments, the antigen is K-Ras. In some embodiments, the antigen is mesothelin. In some embodiments, the antigen is PSCA. In some embodiments, the antigen is NY-ESO-1. In some embodiments, the antigen is WT-1. In some embodiments, the antigen is survivin. In some embodiments, the antigen is gp100. In some embodiments, the antigen is PAP. In some embodiments, the antigen is proteinase 3. In some embodiments, the antigen is SPAS-1. In some embodiments, the antigen is SP-17. In some embodiments, the antigen is PAGE-4. In some embodiments, the antigen is TARP. In some embodiments, the antigen is CEA.

In some embodiments, the antigen is human mesothelin.

In some embodiments, the antigen is mesothelin, SPAS-1, proteinase-3, EphA2, SP-17, gp100, PAGE-4, TARP, B-raf, tyrosinase, mdm-2, MAGE, RAGE, MART-1, bcr/abl, Her-2/neu, alphafetoprotein, mammoglobin, hTERT(telomerase), PSA or CEA, or an antigen derived from one of those proteins. In some embodiments the antigen is mesothelin or is derived from mesothelin. In other embodiments, the antigen is EphA2 or is an antigen derived from EphA2. In some embodiments, the antigen encoded by a recombinant nucleic acid molecule described herein is not Epha2 (or an antigen derived from Epha2). In some embodiments, the antigen is a tumor-associated antigen other than Epha2. In some embodiments, the antigen is derived from a tumor-associated antigen other than Epha2.

In some embodiments, a polynucleotide in the recombinant nucleic acid molecule encodes an antigen derived from K-Ras, H-Ras, N-Ras, 12-K-Ras, mesothelin, PSCA, NY-ESO-1, WT-1, survivin, gp100, PAP, proteinase 3, SPAS-1, SP-17, PAGE-4, TARP, B-raf, tyrosinase, mdm-2, MAGE, RAGE, MART-1, bcr/abl, Her-2/neu, alphafetoprotein, mammoglobin, hTERT(telomerase), PSA or CEA. In some embodiments, the antigen is derived from K-Ras. In some embodiments, the antigen is derived from H-Ras. In some embodiments, the polypeptide is N-Ras. In some embodiments, the antigen is derived from 12-K-Ras. In some embodiments, the antigen is an antigen derived from mesothelin. In some embodiments, the antigen is an antigen derived from PSCA. In some embodiments, the antigen is an antigen derived from NY-ESO-1. In some embodiments, the antigen is an antigen derived from WT-1. In some embodiments, the antigen is an antigen derived from survivin. In some embodiments, the antigen is an antigen that is derived from gp100. In some embodiments, the antigen is an antigen that is derived from PAP. In some embodiments, the antigen is an antigen that is derived from proteinase 3. In some embodiments, the antigen is an antigen derived from SPAS-1. In some embodiments, the antigen is an antigen derived from SP-17. In some embodiments, the antigen is an antigen derived from PAGE-4. In some embodiments, the antigen is an antigen derived from TARP. In some embodiments, the antigen is an antigen derived from CEA.

In some embodiments, the antigen is mesothelin, or an antigenic fragment or antigenic variant thereof. In some embodiments, the antigen is mesothelin in which the mesothelin signal peptide and/or GPI anchor has been deleted. In some embodiments, the antigen is human mesothelin in which the mesothelin signal peptide and/or GPI anchor has been deleted. In some embodiments, the antigen is human mesothelin in which the mesothelin signal peptide and GPI anchor has been deleted.

In some embodiments, the antigen is NY-ESO-1, or an antigenic fragment or antigenic variant thereof.

In some embodiments, a polypeptide encoded by polynucleotide in a recombinant nucleic acid molecule comprises at least one antigenic fragment of a tumor-associated antigen, e.g., human prostate stem cell antigen (PSCA; GenBank Acc. No.AF043498), human testes antigen (NY-ESO-1; GenBank Acc. No. NM_001327), human carcinoembryonic antigen (CEA; GenBank Acc. No. M29540), human Mesothelin (GenBank Acc. No. U40434), human survivin (GenBank Acc. No. U75285), human Proteinase 3 (GenBank No. X55668), human K-Ras (GenBank Acc. Nos. M54969 & P01116), human H-Ras (GenBank Acc. No. P01112), human N-Ras (GenBank Acc. No. P01111), and human 12-K-Ras (K-Ras comprising a Gly12Asp mutation) (see, e.g., GenBank Acc. No. K00654). In some embodiments, a polypeptide encoded by polynucleotide in a recombinant nucleic acid molecule comprises an antigenic fragment of a tumor-associated antigen with at least one conservatively substituted amino acid. In some embodiments, a polypeptide encoded by polynucleotide in a recombinant nucleic acid molecule comprises an antigenic fragment with at least one deleted amino acid residue. In some embodiments, a polypeptide encoded by polynucleotide in a recombinant nucleic acid molecule comprises combinations of antigenic sequences derived from more than one type of tumor-associated antigen, e.g., a combination of antigenic fragments derived from both mesothelin and Ras.

Exemplary regions of tumor antigens predicted to be antigenic include the following: amino acids 25-35; 70-80; and 90-118 of the PSCA amino acid sequence in GenBank Acc. No. AF043498; amino acids 40-55, 75-85, 100-115, and 128-146 of the NY-ESO-1 of GenBank Acc. No. NM_001327; amino acids 70-75, 150-155, 205-225, 330-340, and 510-520 of the CEA amino acid sequence of GenBank Acc. No. M29540; amino acids 90-110, 140-150, 205-225, 280-310, 390-410, 420-425, and 550-575; of the mesothelin polypeptide sequence of GenBank Acc. No. U40434; amino acids 12-20, 30-40, 45-55, 65-82, 90-95, 102-115, and 115-130 of the surviving polypeptide sequence of GenBank Acc. No. U75285; amino acids 10-20, 30-35, 65-75, 110-120, and 160-170, of the amino acid sequence of proteinase-3 found in GenBank Acc. No. X55668; amino acids 10-20, 30-50, 55-75, 85-110, 115-135, 145-155, and 160-185 of GenBank Acc. Nos. P01117 or M54968 (human K-Ras); amino acids 10-20, 25-30, 35-45, 50-70, 90-110, 115-135, and 145-175 of GenBank Acc. No. P01112 (human H-Ras); amino acids 10-20, 25-45, 50-75, 85-110, 115-135, 140-155, and 160-180 of GenBank Acc. No. P01111 (human N-Ras); and the first 25-amino acids of 12-K-Ras (sequence disclosed in GenBank Acc. No. K00654). These antigenic regions were predicted by Hopp-Woods and Welling antigenicity plots.

In some embodiments, the polypeptides encoded by the polynucleotides of the invention either as discrete polypeptides, as fusion proteins with the chosen signal peptide, or as a protein chimera in which the polypeptide has been inserted in another polypeptide, are polypeptides comprising one or more of the following peptides of human mesothelin: SLLFLLFSL (amino acids 20-28); VLPLTVAEV (amino acids 530-538); ELAVALAQK (amino acids 83-92); ALQGGGPPY (amino acids 225-234); FYPGYLCSL (amino acids 435-444); and LYPKARLAF (amino acids 475-484). For instance, in some embodiments, the antigen encoded by a polynucleotide of the invention is an (antigenic) fragment of human mesothelin comprising one or more of these peptides. Additional information regarding these mesothelin peptide sequences and their correlation with medically relevant immune responses can be found in the PCT Publication WO 2004/006837.

Alternatively, polynucleotides in the recombinant nucleic acid molecule can encode an autoimmune disease-specific antigen. In a T cell mediated autoimmune disease, a T cell response to self antigens results in the autoimmune disease. The type of antigen for use in treating an autoimmune disease with the vaccines of the present invention might target the specific T cells responsible for the autoimmune response. For example, the antigen may be part of a T cell receptor, the idiotype, specific to those T cells causing an autoimmune response, wherein the antigen incorporated into a vaccine of the invention would elicit an immune response specific to those T cells causing the autoimmune response. Eliminating those T cells would be the therapeutic mechanism to alleviating the autoimmune disease. Another possibility would be to incorporate into the recombinant nucleic acid molecule a polynucleotide encoding an antigen that will result in an immune response targeting the antibodies that are generated to self antigens in an autoimmune disease or targeting the specific B cell clones that secrete the antibodies. For example, a polynucleotide encoding an idiotype antigen may be incorporated into the recombinant nucleic acid molecule that will result in an anti-idiotype immune response to such B cells and/or the antibodies reacting with self antigens in an autoimmune disease. Autoimmune diseases treatable with vaccines comprising bacteria comprising the expression cassettes and recombinant nucleic acid molecules of the present invention include, but are not limited to, rheumatoid arthritis, multiple sclerosis, Crohn's disease, lupus, myasthenia gravis, vitiligo, scleroderma, psoriasis, pemphigus vulgaris, fibromyalgia, colitis and diabetes. A similar approach may be taken for treating allergic responses, where the antigens incorporated into the vaccine microbe target either T cells, B cells or antibodies that are effective in modulating the allergic reaction. In some autoimmune diseases, such as psoriasis, the disease results in hyperproliferative cell growth with expression of antigens that may be targeted as well. Such an antigen that will result in an immune response to the hyperproliferative cells is considered.

Optionally, the recombinant nucleic acid molecule encodes an antigen that targets unique disease associated protein structures. One example of this is the targeting of antibodies, B cells or T cells using idiotype antigens as discussed above. Another possibility is to target unique protein structures resulting from a particular disease. An example of this would be to incorporate an antigen that will generate an immune response to proteins that cause the amyloid plaques observed in diseases such as Alzheimer's disease, Creutzfeldt-Jakob disease (CJD) and Bovine Spongiform Encephalopathy (BSE). While this approach may only provide for a reduction in plaque formation, it may be possible to provide a curative vaccine in the case of diseases like CJD. This disease is caused by an infectious form of a prion protein. In some embodiments, the polynucleotides encode an antigen to the infectious form of the prion protein such that the immune response generated by the vaccine may eliminate, reduce, or control the infectious proteins that cause CJD.

In some embodiments, the polypeptide encoded by the recombinant nucleic acid molecule is an infectious disease antigen or is derived from an infectious disease antigen. In some embodiments, the polypeptide encoded by the recombinant nucleic acid molecule is an infectious disease antigen. In some embodiments, the polypeptide encoded by the recombinant nucleic acid molecule is derived from an infectious disease antigen.

In other embodiments of the invention, the antigen is derived from a human or animal pathogen. The pathogen is optionally a virus, bacterium, fungus, or a protozoan. For instance, the antigen may be a viral or fungal or bacterial antigen. In one embodiment, the antigen encoded by the recombinant nucleic acid molecule that is derived from the pathogen is a protein produced by the pathogen, or is derived from a protein produced by the pathogen. For instance, in some embodiments, the polypeptide encoded by the recombinant nucleic acid molecules, expression cassette and/or expression vector is a fragment and/or variant of a protein produced by the pathogen.

For instance, in some embodiments, the antigen is derived from Human Immunodeficiency virus (such as gp 120, gp 160, gp41, gag antigens such as p24gag and p55gag, as well as proteins derived from the pol, env, tat, vif, rev, nef, vpr, vpu and LTR regions of HIV), Feline Immunodeficiency virus, or human or animal herpes viruses. For example, in some embodiments, the antigen is gp 120. In one embodiment, the antigen is derived from herpes simplex virus (HSV) types 1 and 2 (such as gD, gB, gH, Immediate Early protein such as ICP27), from cytomegalovirus (such as gB and gH), from metapneumovirus, from Epstein-Barr virus or from Varicella Zoster Virus (such as gpI, II or III). (See, e. g., Chee et al. (1990) Cytomegaloviruses (J. K. McDougall, ed., Springer Verlag, pp. 125-169; McGeoch et al. (1988) J. Gen. Virol. 69: 1531-1574; U.S. Pat. No. 5,171,568; Baer et al. (1984) Nature 310: 207-211; and Davison et al. (1986) J. Gen. Virol. 67: 1759-1816.)

In another embodiment, the antigen is derived from a hepatitis virus such as hepatitis B virus (for example, Hepatitis B Surface antigen), hepatitis A virus, hepatitis C virus, delta hepatitis virus, hepatitis E virus, or hepatitis G virus. See, e.g., WO 89/04669; WO 90/11089; and WO 90/14436. The hepatitis antigen can be a surface, core, or other associated antigen. The HCV genome encodes several viral proteins, including E1 and E2. See, e. g., Houghton et al., Hepatology 14: 381-388 (1991).

An antigen that is a viral antigen is optionally derived from a virus from any one of the families Picornaviridae (e. g., polioviruses, rhinoviruses, etc.); Caliciviridae; Togaviridae (e. g., rubella virus, dengue virus, etc.); Flaviviridae; Coronaviridae; Reoviridae (e. g., rotavirus, etc.); Birnaviridae; Rhabodoviridae (e. g., rabies virus, etc.); Orthomyxoviridae (e. g., influenza virus types A, B and C, etc.); Filoviridae; Paramyxoviridae (e. g., mumps virus, measles virus, respiratory syncytial virus, parainfluenza virus, etc.); Bunyaviridae; Arenaviridae; Retroviradae (e. g., HTLV-I; HTLV-11; HIV-1 (also known as HTLV-111, LAV, ARV, hTLR, etc.)), including but not limited to antigens from the isolates HIVI11b, HIVSF2, HTVLAV, HIVLAI, HIVMN); HIV-1CM235, HIV-1; HIV-2, among others; simian immunodeficiency virus (SIV)); Papillomavirus, the tick-borne encephalitis viruses; and the like. See, e. g. Virology, 3rd Edition (W. K. Joklik ed. 1988); Fundamental Virology, 3rd Edition (B. N. Fields, D. M. Knipe, and P.M. Howley, Eds. 1996), for a description of these and other viruses. In one embodiment, the antigen is Flu-HA (Morgan et al., J. Immunol. 160:643 (1998)).

In some alternative embodiments, the antigen is derived from bacterial pathogens such as Mycobacterium, Bacillus, Yersinia, Salmonella, Neisseria, Borrelia (for example, OspA or OspB or derivatives thereof), Chlamydia, or Bordetella (for example, P.69, PT and FHA), or derived from parasites such as plasmodium or Toxoplasma. In one embodiment, the antigen is derived from Mycobacterium tuberculosis (e.g. ESAT-6, 85A, 85B, 85C, 72F), Bacillus anthracis (e.g.PA), or Yersinia pestis (e.g. F1, V). In addition, antigens suitable for use in the present invention can be obtained or derived from known causative agents responsible for diseases including, but not limited to, Diptheria, Pertussis, Tetanus, Tuberculosis, Bacterial or Fungal Pneumonia, Otitis Media, Gonorrhea, Cholera, Typhoid, Meningitis, Mononucleosis, Plague, Shigellosis or Salmonellosis, Legionaire's Disease, Lyme Disease, Leprosy, Malaria, Hookworm, Onchocerciasis, Schistosomiasis, Trypanosomiasis, Leishmaniasis, Giardia, Amoebiasis, Filariasis, Borelia, and Trichinosis. Still further antigens can be obtained or derived from unconventional pathogens such as the causative agents of kuru, Creutzfeldt-Jakob disease (CJD), scrapie, transmissible mink encephalopathy, and chronic wasting diseases, or from proteinaceous infectious particles such as prions that are associated with mad cow disease.

In still other embodiments, the antigen is obtained or derived from a biological agent involved in the onset or progression of neurodegenerative diseases (such as Alzheimer's disease), metabolic diseases (such as Type I diabetes), and drug addictions (such as nicotine addiction). Alternatively, the antigen encoded by the recombinant nucleic acid molecule is used for pain management and the antigen is a pain receptor or other agent involved in the transmission of pain signals.

In some embodiments, the antigen is a human protein or is derived from a human protein. In other embodiments, the antigen is a non-human protein or is derived from a non-human protein (a fragment and/or variant thereof). In some embodiments, the antigen portion of the fusion protein encoded by the expression cassette is a protein from a non-human animal or is a protein derived from a non-human animal. For instance, even if the antigen is to be expressed in a Listeria-based vaccine that is to be used in humans, in some embodiments, the antigen can be murine mesothelin or derived from murine mesothelin.

### V. Listeria

In some embodiments, the *Listeria* belong to the species *Listeria monocytogenes.* In some alternative embodiments the bacteria are members of the *Listeria ivanovii*, *Listeria seeligeri, Listeria innocua, L. welshimeri,* or *L. gravi* species.

In some embodiments, the *Listeria* are non-naturally occurring. In some embodiments, the *Listeria* are attenuated. In some embodiments, the *Listeria* are viable. In some embodiments, the *Listeria* are mutant *Listeria,* recombinant *Listeria,* or otherwise modified. In some embodiments, the *Listeria* are attenuated. In some embodiments, the *Listeria* are metabolically active. In certain embodiments, the *Listeria* are not infected with bacteriophage. The invention further provides *Listeria* that are recombinant. In addition, the *Listeria* may be isolated and/or substantially purified.

In some embodiments, the attenuated *Listeria* is attenuated in one or more of growth, cell to cell spread, binding to or entry into a host cell, replication, or DNA repair. In some embodiments, the *Listeria* is attenuated by one or more of an *actA* mutation, an *inlB* mutation, a *uvrA* mutation, a *uvrB* mutation, a *uvrC* mutation, a nucleic acid targeting compound, or a *uvrAB* mutation and a nucleic acid targeting compound. In some embodiments, the attenuated *Listeria* is attenuated in cell to cell spread and/or entry into nonphagocytic cells. In some embodiments, the *Listeria* is attenuated by one or more of an *actA* mutation or an *actA* mutation and an *inlB* mutation. In some embodiments, the *Listeria* is Δ*actA* or Δ*actA*Δ*inlB.*

In some embodiments, the attenuated *Listeria* is attenuated for cell-to-cell spread. In some embodiments, the *Listeria* attenuated for cell-to-cell spread are defective with respect to ActA (e.g., relative to the non-modified or wild-type *Listeria*). In some embodiments, the *Listeria* comprises an attenuating mutation in the *actA* gene. In some embodiments, the *Listeria* comprises a full or partial deletion in the *actA* gene.

In some embodiments, the capacity of the attenuated *Listeria* bacterium for cell-to-cell spread is reduced by at least about 10%, at least about 25%, at least about 50%, at least about 75%, or at least about 90%, relative to *Listeria* without the attenuating mutation (e.g., wild type *Listeria*). In some embodiments, the capacity of the attenuated *Listeria* bacterium for cell-to-cell spread is reduced by at least about 25% relative to *Listeria* without the attenuating mutation. In some embodiments, the capacity of the attenuated *Listeria* bacterium attenuated for cell-to-cell spread is reduced by at least about 50% relative to the *Listeria* without the attenuating mutation.

*In vitro* assays for determining whether a *Listeria* bacterium is attenuated for cell-to-cell spread are known to those of ordinary skill in the art. For example, the diameter of plaques formed over a time course after infection of selected cultured cell monolayers can be measured. Plaque assays within L2 cell monolayers can be performed as described previously in Sun, A., A. Camilli, and D.A. Portnoy. 1990, Isolation of Listeria monocytogenes small-plaque mutants defective for intracellular growth and cell-to-cell spread. Inflect. Immun. 58:3770-3778, with modifications to the methods of measurement, as described by in Skoble, J., D.A. Portnoy, and M.D. Welch. 2000, Three regions within ActA promote Arp2/3 complex-mediated actin nucleation and Listeria monocytogenes motility. J. Cell Biol. 150:527-538. In brief, L2 cells are grown to confluency in six-well tissue culture dishes and then infected with bacteria for 1 h. Following infection, the cells are overlayed with media warmed to 40°C that is comprised of DME containing 0.8% agarose, Fetal Bovine Serum (e.g., 2%), and a desired concentration of Gentamicin. The concentration of Gentamicin in the media dramatically affects plaque size, and is a measure of the ability of a selected *Listeria* strain to effect cell-to-cell spread (Glomski, I J., M. M. Gedde, A. W. Tsang, J. A. Swanson, and D. A. Portnoy. 2002. J. Cell Biol. 156:1029-1038). For example, in some embodiments at 3 days following infection of the monolayer the plaque size of *Listeria* strains having a phenotype of defective cell-to-cell spread is reduced by at least 50% as compared to wild-type *Listeria,* when overlayed with media containing Gentamicin at a concentration of 50 µg/ml. On the other hand, the plaque size between *Listeria* strains having a phenotype of defective cell-to-cell spread and wild-type *Listeria* is similar when infected monolayers are overlayed with media + agarose containing only 5 µg/ml gentamicin. Thus, the relative ability of a selected strain to effect cell-to-cell spread in an infected cell monolayer relative to wild-type *Listeria* can be determined by varying the concentration of gentamicin in the media containing agarose. Optionally, visualization and measurement of plaque diameter can be facilitated by the addition of media containing Neutral Red (GIBCO BRL; 1:250 dilution in DME + agarose media) to the overlay at 48 h. post infection. Additionally, the plaque assay can be performed in monolayers derived from other primary cells or continuous cells. For example HepG2 cells, a hepatocyte-derived cell line, or primary human hepatocytes can be used to evaluate the ability of selected *Listeria* mutants to effect cell-to-cell spread, as compared to wild-type *Listeria.* In some embodiments, *Listeria* comprising mutations or other modifications that attenuate the *Listeria* for cell-to-cell spread produce "pinpoint" plaques at high concentrations of gentamicin (about 50 µg/ml).

In some embodiments, the *Listeria* is attenuated for entry into non-phagocytic cells (relative or the non-mutant or wildtype *Listeria* ). In some embodiments, the *Listeria* is defective with respect to one or more internalins (or equivalents). In some embodiments, the *Listeria* is defective with respect to internalin A. In some embodiments, the *Listeria* is defective with respect to internalin B. In some embodiments, the *Listeria* comprise a mutation in *inlA.* In some embodiments, the *Listeria* comprise a mutation in *inlB.* In some embodiments, the *Listeria* comprise a mutation in both *actA* and *inlB.* In some embodiments, the *Listeria* is deleted in functional ActA and internalinB. In some embodiments, the attenuated *Listeria* bacterium is an Δ*actA*Δ*inlB* double deletion mutant. In some embodiments, the *Listeria* bacterium is defective with respect to both ActA and internalin B.

In some embodiments, the capacity of the attenuated *Listeria* bacterium for entry into non-phagocytic cells is reduced by at least about 10%, at least about 25%, at least about 50%, at least about 75%, or at least about 90%, relative to *Listeria* without the attenuating mutation (e.g., the wild type bacterium). In some embodiments, the capacity of the attenuated *Listeria* bacterium for entry into non-phagocytic cells is reduced by at least about 25% relative to *Listeria* without the attenuating mutation. In some embodiments, the capacity of the attenuated bacterium for entry into non-phagocytic cells is reduced by at least about 50% relative to *Listeria* without the attenuating mutation. In some embodiments, the capacity of the attenuated *Listeria* bacterium for entry into non-phagocytic cells is reduced by at least about 75% relative to *Listeria* without the attenuating mutation.

In some embodiments, the attenuated *Listeria* is not attenuated for entry into more than one type of non-phagocytic cell. For instance, the attenuated strain may be attenuated for entry into hepatocytes, but not attenuated for entry into epithelial cells. As another example, the attenuated strain may be attenuated for entry into epithelial cells, but not hepatocytes. It is also understood that attenuation for entry into a non-phagocytic cell of a particular modified *Listeria* is a result of mutating a designated gene, for example a deletion mutation, encoding an invasin protein which interacts with a particular cellular receptor, and as a result facilitates infection of a non-phagocytic cell. For example, *Listeria* Δ*inlB* mutant strains are attenuated for entry into non-phagocytic cells expressing the hepatocyte growth factor receptor (c-met), including hepatocyte cell lines (e.g., HepG2), and primary human hepatocytes.

In some embodiments, even though the *Listeria* is attenuated for entry into non-phagocytic cells, the *Listeria* is still capable of uptake by phagocytic cells, such as at least dendritic cells and/or macrophages. In one embodiment the ability of the attenuated *Listeria* to enter phagocytic cells is not diminished by the modification made to the strain, such as the mutation of an invasin (i.e. approximately 95% or more of the measured ability of the strain to be taken up by phagocytic cells is maintained post-modification). In other embodiments, the ability of the attenuated *Listeria* to enter phagocytic cells is diminished by no more than about 10%, no more than about 25%, no more than about 50%, or no more than about 75%.

In some embodiments of the invention, the amount of attenuation in the ability of the *Listeria* to enter non-phagocytic cells ranges from a two-fold reduction to much greater levels of attenuation. In some embodiments, the attenuation in the ability of the *Listeria* to enter non-phagocytic cells is at least about 0.3 log, about 1 log, about 2 log, about 3 log, about 4 log, about 5 log, or at least about 6 log. In some embodiments, the attenuation is in the range of about 0.3 to > 8 log, about 2 to >8 log, about 4 to >8 log, about 6 to >8 log, about 0.3-8 log, also about 0.3-7 log, also about 0.3-6 log, also about 0.3-5 log, also about 0.3-4 log, also about 0.3-3 log, also about 0.3-2 log, also about 0.3-1 log. In some embodiments, the attenuation is in the range of about 1 to >8 log, 1-7 log, 1-6 log, also about 2-6 log, also about 2-5 log, also about 3-5 log.

*In vitro* assays for determining whether or not a *Listeria* bacterium is attenuated for entry into non-phagocytic cells are known to those of ordinary skill in the art. For instance, both Dramsi et al., Molecular Microbiology 16:251-261 (1995) and Gaillard et al., Cell 65:1127-1141 (1991) describe assays for screening the ability of mutant *L. monocytogenes* strains to enter certain cell lines. For instance, to determine whether a *Listeria* bacterium with a particular modification is attenuated for entry into a particular type of non-phagocytic cells, the ability of the attenuated *Listeria* bacterium to enter a particular type of non-phagocytic cell is determined and compared to the ability of the identical *Listeria* bacterium without the modification to enter non-phagocytic cells. Likewise, to determine whether a *Listeria* strain with a particular mutation is attenuated for entry into a particular type of non-phagocytic cells, the ability of the mutant *Listeria* strain to enter a particular type of non-phagocytic cell is determined and compared to the ability of the *Listeria* strain without the mutation to enter non-phagocytic cells. For instance, the ability of a modified *Listeria* bacterium to infect non-phagocytic cells, such as hepatocytes, can be compared to the ability of non-modified *Listeria* or wild type *Listeria* to infect phagocytic cells. In such an assay, the modified and non-modified *Listeria* is typically added to the non-phagocytic cells *in vitro* for a limited period of time (for instance, an hour), the cells are then washed with a gentamicin-containing solution to kill any extracellular bacteria, the cells are lysed and then plated to assess titer. Examples of such an assay are found in U.S. Patent Publication No. 2004/0228877. In addition, confirmation that the strain is defective with respect to internalin B may also be obtained through comparison of the phenotype of the strain with the previously reported phenotypes for internalin B mutants.

A *Listeria monocytogenes* Δ*actA*Δ*inlB* strain was deposited with the American Type Culture Collection (ATCC), 10801 University Blvd., Manassas, Virginia 20110-2209, United States of America (P.O. Box 1549, Manassas, Virginia, 20108, United States of America), on October 3, 2003, under the provisions of the Budapest Treaty on the International Recognition of the Deposit of Microorganisms for the Purposes of Patent Procedure, and designated with accession number PTA-5562. Another *Listeria monocytogenes* strain, an Δ*actA*Δ*uvrAB* strain, was also deposited with the ATCC on October 3, 2003, under the provisions of the Budapest Treaty on the International Recognition of the Deposit of Microorganisms for the Purposes of Patent Procedure, and designated with accession number PTA-5563.

In some embodiments, *Listeria* is attenuated for nucleic acid repair (e.g., relative to wildtype). For instance, in some embodiments, the *Listeria* is defective with respect to at least one DNA repair enzyme (e.g., *Listeria monocytogenes uvrAB* mutants). In some embodiments, the *Listeria* is defective with respect to PhrB, UvrA, UvrB, UvrC, UvrD, and/or RecA. In some embodiments, the bacteria are defective with respect to UvrA, UvrB, and/or UvrC. In some embodiments, the bacteria comprise attenuating mutations in *phrB*, *uvrA, uvrB, uvrC, uvrD,* and/or *recA* genes. In some embodiments, the bacteria comprise one or more mutations in the *uvrA, uvrB,* and/or *uvrC* genes. In some embodiments, the bacteria are functionally deleted in UvrA, UvrB, and/or UvrC. In some embodiments, the bacteria are deleted in functional UvrA and UvrB. In some embodiments, the bacteria are *uvrAB* deletion mutants. In some embodiments, the bacteria are Δ*uvrAB*Δ*actA* mutants. In some embodiments, the nucleic acid of the bacteria which are attenuated for nucleic acid repair and/or are defective with respect to at least one DNA repair enzyme are modified by reaction with a nucleic acid targeting compound. Nucleic acid repair mutants, such as Δ*uvrAB Listeria monocytogenes* mutants, and methods of making the mutants, are described in detail in U.S. Patent Publication No. 2004/0197343 (see, e.g., Example 7 of U.S. 2004/0197343).

In some embodiments, the capacity of the attenuated *Listeria* bacterium for nucleic acid repair is reduced by at least about 10%, at least about 25%, at least about 50%, at least about 75%, or at least about 90%, relative to a *Listeria* bacterium without the attenuating mutation (e.g., the wild type bacterium). In some embodiments, the capacity of the attenuated *Listeria* bacterium for nucleic acid repair is reduced by at least about 25% relative to a *Listeria* bacterium without the attenuating mutation. In some embodiments, the capacity of the attenuated *Listeria* bacterium attenuated for nucleic acid repair is reduced by at least about 50% relative a *Listeria* bacterium without the attenuating mutation.

Confirmation that a particular mutation is present in a bacterial strain can be obtained through a variety of methods known to those of ordinary skill in the art. For instance, the relevant portion of the strain's genome can be cloned and sequenced. Alternatively, specific mutations can be identified via PCR using paired primers that code for regions adjacent to a deletion or other mutation. Southern blots can also be used to detect changes in the bacterial genome. Also, one can analyze whether a particular protein is expressed by the strain using techniques standard to the art such as Western blotting. Confirmation that the strain contains a mutation in the desired gene may also be obtained through comparison of the phenotype of the strain with a previously reported phenotype. For example, the presence of a nucleotide excision repair mutation such as deletion of *uvrAB* can be assessed using an assay which tests the ability of the bacteria to repair its nucleic acid using the nucleotide excision repair (NER) machinery and comparing that ability against wild-type bacteria. Such functional assays are known in the art. For instance, cyclobutane dimer excision or the excision of UV-induced (6-4) products can be measured to determine a deficiency in an NER enzyme in the mutant (see, e.g., Franklin et al., Proc. Natl. Acad. Sci. USA, 81: 3821-3824 (1984)). Alternatively, survival measurements can be made to assess a deficiency in nucleic acid repair. For instance, the *Listeria* can be subjected to psoralen/UVA treatment and then assessed for their ability to proliferate and/or survive in comparison to wild-type. The invention provides a *Listeria* bacterium, or a *Listeria* strain, that is killed but metabolically active (KBMA) (see, e.g., Brockstedt, et al. (2005) Nat. Med. [July 24 epub ahead of point]). A KBMA *Listeria* bacterium is metabolically active, but cannot form a colony, e.g., on agar. An inactivating mutation in at least one DNA repair gene, e.g., Δ*urvrAB*, enables killing of *Listeria* using concentrations of a nucleic acid cross-linking agent (e.g., psoralen) at low concentrations, where these concentrations are sufficient to prevent colony formation but not sufficient to substantially impair metabolism. The result of limited treatment with psoralen/UVA light, and/or of treatment with a nucleic acid cross-linking agent that is highly specific for making interstrand genomic cross links, is that the bacterial cells are killed but remain metabolically active.

The disclosure supplies a number of *Listeria* strains for making or engineering an attenuated *Listeria* for use in the present invention (Table 4). The *Listeria* of the present invention are not to be limited by the strains disclosed in this table.

**Table 4. Exemplary strains of Listeria for use as parental strains in the present invention.**

| | |
|---|---|
| *L. monocytogenes* 10403S wild type. | Bishop and Hinrichs (1987) J. Immunol. 139:2005-2009; Lauer, et al. (2002) J. Bact. 184:4177-4186. |
| *L. monocytogenes* DP-L4056 (phage cured). The prophage-cured 10403S strain is designated DP-L4056. | Lauer, et al. (2002) J. Bact. 184:4177-4186. |
| *L. monocytogenes* DP-L4027, which is DP-L2161, phage cured, deleted in hly gene. | Lauer, et al. (2002) J. Bact. 184:4177-4186; Jones and Portnoy (1994) Infect. Immunity 65:5608-5613. |
| *L. monocytogenes* DP-L4029, which is DP-L3078, phage cured, deleted in *actA.* | Lauer, et al. (2002) J. Bact. 184:4177-4186; Skoble, et al. (2000) J. Cell Biol. 150:527-538. |
| *L. monocytogenes* DP-L4042 (delta PEST) | Brockstedt, et al. (2004) Proc. Natl. Acad. Sci. USA 101:13832-13837; supporting information. |
| *L. monocytogenes* DP-L4097 (LLO-S44A). | Brockstedt, et al. (2004) Proc. Natl. Acad. Sci. USA 101:13832-13837; supporting information. |
| *L. monocytogenes* DP-L4364 (delta *lplA*; lipoate protein ligase). | Brockstedt, et al. (2004) Proc. Natl. Acad. Sci. USA 101:13832-13837; supporting information. |
| L. monocytogenes DP-L4405 (delta in1A). | Brockstedt, et al. (2004) Proc. Natl. Acad. Sci. USA 101:13832-13837; supporting information. |
| L. monocytogenes DP-L4406 (delta inlB). | Brockstedt, et al. (2004) Proc. Natl. Acad. Sci. USA 101:13832-13837; supporting information. |
| *L. monocytogenes* CS-L0001 (delta *actA-*delta *inlB).* | Brockstedt, et al. (2004) Proc. Natl. Acad. Sci. USA 101:13832-13837; supporting information. |
| *L. monocytogenes* CS-L0002 (delta *actA-*delta *lplA*). | Brockstedt, et al. (2004) Proc. Natl. Acad. Sci. USA 101:13832-13837; supporting information. |
| *L. monocytogenes* CS-L0003 (L461T-delta *lplA*). | Brockstedt, et al. (2004) Proc. Natl. Acad. Sci. USA 101:13832-13837; supporting information. |
| *L. monocytogenes* DP-L4038 (delta *actA-*LLO L461T). | Brockstedt, et al. (2004) Proc. Natl. Acad. Sci. USA 101:13832-13837; supporting information. |
| *L. monocytogenes* DP-L4384 (S44A-LLO L461T). | Brockstedt, et al. (2004) Proc. Natl. Acad. Sci. USA 101:13832-13837; supporting information. |
| *L. monocytogenes.* Mutation in lipoate protein ligase (Lp1A1). | O'Riordan, et al. (2003) Science 302:462-464. |
| *L. monocytogenes* DP-L4017 (10403S with LLO L461T point mutation in hemolysin gene). | U.S. Provisional Pat. Appl. Ser. No. 60/490,089 filed July 24, 2003. |
| *L. monocytogenes* EGD. | GenBank Acc. No. AL591824. |
| *L. monocytogenes* EGD-e. | GenBank Acc. No. NC_003210. ATCC Acc. No. BAA-679. Glaser P, et al. (2001) Science 294:849-852. |
| *L. monocytogenes* strain EGD, complete genome, segment 3/12 | GenBank Acc. No. AL591975 |
| *L. monocytogenes.* | ATCC Nos. 13932; 15313; 19111-19120; 43248-43251; 51772-51782. |
| *L. monocytogenes* DP-L4029 deleted in *uvrAB.* | U.S. Provisional Pat. Appl. Ser. No. 60/541,515 filed February 2, 2004; U.S. Provisional Pat. Appl. Ser. No. 60/490,080 filed July 24, 2003. |
| *L. monocytogenes* DP-L4029 deleted in *uvrAB* treated with a psoralen. | U.S. Provisional Pat. Appl. Ser. No. 60/541,515 filed February 2, 2004. |
| *L. monocytogenes actA⁻*/*inlB⁻* double mutant. | Deposited with ATCC on October 3, 2003. Acc. No. PTA-5562. |
| *L. monocytogenes lplA* mutant or hly mutant. | U.S. Pat. Applic. No. 20040013690 of Portnoy, et al. |
| *L. monocytogenes* DAL/DAT double mutant. | U.S. Pat. Applic. No. 20050048081 of Frankel and Portnoy. |
| *L. monocytogenes* str. 4b F2365. | GenBank Acc. No. NC_002973. |
| *Listeria ivanovii* | ATCC No. 49954 |
| *Listeria innocua* Clip 11262. | GenBank Acc. No. NC_003212; AL592022. |
| *Listeria innocua*, a naturally occurring hemolytic strain containing the PrfA-regulated virulence gene cluster. | Johnson, et al. (2004) Appl. Environ. Microbiol. 70:4256-4266. |
| *Listeria seeligeri.* | Howard, et al. (1992) Appl. Eviron. Microbiol. 58:709-712. |
| *Listeria innocua* with *L. monocytogenes* pathogenicity island genes. | Johnson , et al. (2004) Appl. Environ. Microbiol. 70:4256-4266. |
| *Listeria innocua* with *L. monocytogenes* internalin A gene, e.g., as a plasmid or as a genomic nucleic acid. | See, e.g., Lingnau, et al. (1995) Infection Immunity 63:3896-3903; Gaillard, et al. (1991) Cell 65:1127-1141). |
| *L. monocytogenes* L028 | Perez-Diaz J.C. et al. (1982) Plasmid 8:112-118. |
| *L. monocytogenes* F2365 | Nelson, K.E. et al. (2004) Nuc. Acids Res. 32:2386-2395. |
| *L. monocytogenes* H7858 | Nelson, K.E. et al. (2004) Nuc. Acids Res. 32:2386-2395 |
| *L. monocytogenes* F6854 | Nelson, K.E. et al. (2004) Nuc. Acids Res. 32:2386-2395 |
| The present invention encompasses reagents and methods that comprise the above listerial strains, as well as these strains that are modified, e.g., by a plasmid and/or by genomic integration, to contain a nucleic acid encoding one of, or any combination of, the following genes: *hly* (LLO; listeriolysin); *iap* (p60); *inlA*; *inlB*; *inlC*; *dal* (alanine racemase); *daaA* (*dat*; D-amino acid aminotransferase); *plcA*; *plcB*; *actA*; or any nucleic acid that mediates growth, spread, breakdown of a single walled vesicle, breakdown of a double walled vesicle, binding to a host cell, uptake by a host cell. The present invention is not to be limited by the particular strains disclosed above. | |

In some embodiments, the attenuation of *Listeria* can be measured in terms of biological effects of the *Listeria* on a host. The pathogenicity of a strain can be assessed by measurement of the LD₅₀ in mice or other vertebrates. The LD₅₀ is the amount, or dosage, of *Listeria* injected into vertebrates necessary to cause death in 50% of the vertebrates. The LD₅₀ values can be compared for bacteria having a particular modification (e.g., mutation) versus the bacteria without the particular modification as a measure of the level of attenuation. For example, if the bacterial strain without a particular mutation has an LD₅₀ of 10³ bacteria and the bacterial strain having the particular mutation has an LD₅₀ of 10⁵ bacteria, the strain has been attenuated so that is LD₅₀ is increased 100-fold or by 2 log.

In some embodiments, the attenuated *Listeria* has an LD₅₀ that is at least about 5 times higher, at least about 10 times higher, at least about 100 times higher, at least about 1000 times higher, or at least about 1 x 10⁴ higher than the LD₅₀ of parental or wildtype *Listeria.*

As a further example, the degree of attenuation may also be measured qualitatively by other biological effects, such as the extent of tissue pathology or serum liver enzyme levels. Alanine aminotransferase (ALT), aspartate aminotransferase (AST), albumin and bilirubin levels in the serum are determined at a clinical laboratory for mice injected with *Listeria* (or other bacteria). Comparisons of these effects in mice or other vertebrates can be made for *Listeria* with and without particular modifications/mutations as a way to assess the attenuation of the *Listeria.* Attenuation of the *Listeria* may also be measured by tissue pathology. The amount of *Listeria* that can be recovered from various tissues of an infected vertebrate, such as the liver, spleen and nervous system, can also be used as a measure of the level of attenuation by comparing these values in vertebrates injected with mutant versus non-mutant *Listeria.* For instance, the amount of *Listeria* that can be recovered from infected tissues such as liver or spleen as a function of time can be used as a measure of attenuation by comparing these values in mice injected with mutant vs. non-mutant *Listeria.*

Accordingly, the attenuation of the *Listeria* can be measured in terms of bacterial load in particular selected organs in mice known to be targets by wild-type *Listeria.* For example, the attenuation of the *Listeria* can be measured by enumerating the colonies (Colony Forming Units; CFU or cfu) amusing from plating dilutions of liver or spleen homogenates (homogenized in H₂O + 0.2% NP40) on BHI agar media. The liver or spleen cfu can be measured, for example, over a time course following administration of the modified *Listeria* via any number of routes, including intravenous, intraperitoneal, intramuscular, and subcutaneous. Additionally, the *Listeria* can be measured and compared to a drug-resistant, wild type *Listeria* (or any other selected *Listeria* strain) in the liver and spleen (or any other selected organ) over a time course following administration by the competitive index assay, as described.

Methods of producing mutant *Listeria* are well known in the art. Bacterial mutations can be achieved through traditional mutagenic methods, such as mutagenic chemicals or radiation followed by selection of mutants. Bacterial mutations can also be achieved by one of skill in the art through recombinant DNA technology. For instance, the method of allelic exchange using the pKSV7 vector described in Camilli et al., Molecular Micro. 8:143-157 (1993) is suitable for use in generating mutants including deletion mutants. Alternatively, the gene replacement protocol described in Biswas et al., J. Bacteriol. 175:3628-3635 (1993), can be used. Other similar methods are known to those of ordinary skill in the art.

The construction of a variety of bacterial mutants is described in U.S. patent application Serial No. 10/883,599, U.S. Patent Publication No. 2004/0197343, and U.S. Patent Publication No. 2004/0228877.

The degree of attenuation in uptake of the attenuated bacteria by non-phagocytic cells need not be an absolute attenuation in order to provide a safe and effective vaccine. In some embodiments, the degree of attenuation is one that provides for a reduction in toxicity sufficient to prevent or reduce the symptoms of toxicity to levels that are not life threatening.

In some embodiments, the *Listeria* cannot form colonies, replicate, and/or divide. In some embodiments of the invention, the *Listeria* is attenuated for proliferation relative to parental or wildtype *Listeria.*

In some embodiments, the attenuated *Listeria* is killed, but metabolically active (KBMA) (US Patent Pub. No. 2004/0197343 and Brockstedt, et al., Nat. Med., 11:853-60 (2005)).

The nucleic acid of a population of a *Listeria* can be modified by a variety of methods. The nucleic acid of the microbe can be modified by physical means, e.g. irradiation with ultraviolet light or ionizing radiation. Ionizing radiation, such as x-rays or γ-rays, may be used to cause single-strand or double-strand breaks in the nucleic acid. Ultraviolet radiation may be used to cause pyrimidine dimers in the nucleic acid. The appropriate dose of radiation is determined by assessing the effects of the radiation on replication and protein expression as detailed above.

The nucleic acid of the *Listeria* can also be modified by chemical means, e.g. by reaction with a nucleic acid targeted compound (also referred to herein as a nucleic acid targeting compound). In some embodiments, the *Listeria* is treated with a nucleic acid targeted compound that can modify the nucleic acid such that proliferation of the *Listeria* is attenuated. In some embodiments, the *Listeria* is treated with a nucleic acid targeted compound that can modify the nucleic acid such that the proliferation of the *Listeria* is attenuated, wherein the Listeria population is still able to express a desired protein antigen to a degree sufficient to elicit an immune response. The nucleic acid targeted compound is not limited to a particular mechanism of modifying the nucleic acid. Such compounds modify the nucleic acid either by reacting directly with the nucleic acid (i.e. all or some portion of the compound covalently binds to the nucleic acid), or by indirectly causing the modification of the nucleic acid (e.g. by causing oxygen damage via generation of singlet oxygen or oxygen radicals, by generating radicals of the compound that cause damage, or by other mechanisms of reduction or oxidation of the nucleic acid). Enediynes are an example of a class of compounds that form radical species that result in the cleavage of DNA double strands [Nicolaou et al., Proc. Natl. Acad. Sci. USA, 90:5881-5888 (1993)]. Compounds that react directly with the nucleic acid may react upon activation of the compound, for example upon radiation of the compound. Compounds that react indirectly to cause modification of the nucleic acid may require similar activation to generate either an activated species of the compound or to generate some other active species. While not being limited to the means for activation of nucleic acid targeted compounds, one embodiment of the invention includes the use of photoactivated compounds that either react directly with the nucleic acid or that generate a reactive species such as a reactive oxygen species (e.g. singlet oxygen) which then reacts with the nucleic acid.

The nucleic acid targeted compounds preferentially modify nucleic acids without significantly modifying other components of a biological sample. Such compounds provide adequate modification of the nucleic acid without significantly altering or damaging cell membranes, proteins, and lipids. Such compounds may modify these other cell components to some degree that is not significant. These cell components such as cell membranes, proteins and lipids are not significantly altered if their biological function is sufficiently maintained. In the case of treating a *Listeria* with a nucleic acid targeted compound, the nucleic acid modification is such that the replication of the *Listeria* is attenuated while the cell membranes, proteins and lipids of the *Listeria* are essentially unaffected such that Listerial gene expression is active (e.g. the enzymes required for this are not significantly affected), and the surface of the *Listeria* maintains essentially the same antigenicity as a *Listeria* that has not been treated with the compound. As a result, such compounds are useful in preparing an inactivated *Listeria* for use as a vaccine since the proliferation of the *Listeria* is sufficiently attenuated while maintaining sufficient antigenicity or immunogenicity to be useful as a vaccine. Because the compounds specifically modify nucleic acids, the modification can be controlled to a desired level so that replication is attenuated while maintaining a sufficient level of protein expression. The modification can be controlled by varying the parameters of the reaction, such as compound concentration, reaction media, controlling compound activation factors such as light dose or pH, or controlling compounds that cause oxygen damage by controlling the oxygen concentration (either physically, e.g. by degassing, or chemically, by use of oxygen scavengers). A nucleic acid targeted compound is any compound that has a tendency to preferentially bind nucleic acid, i.e. has a measurable affinity for nucleic acid. Such compounds have a stronger affinity for nucleic acids than for most other components of a biological sample, especially components such as proteins, enzymes, lipids and membranes. The nucleic acid targeting provides specificity for the modification of nucleic acids without significantly affecting other components of the biological sample, such as the machinery for gene transcription and protein translation.

Compounds can be targeted to nucleic acids in a number of modes. Compounds which bind by any of the following modes or combinations of them are considered nucleic acid targeted compounds. Intercalation, minor groove binding, major groove binding, electrostatic binding (e.g. phosphate backbone binding), and sequence-specific binding (via sequence recognition in the major or minor groove) are all noncovalent modes of binding to nucleic acids. Compounds that include one or more of these modes of binding will have a high affinity for nucleic acids. While the invention is not limited to the following compounds, some examples of compounds having these modes of binding to nucleic acid are as follows: intercalators are exemplified by acridines, acridones, proflavin, acriflavine, actinomycins, anthracyclinones, beta-rhodomycin A, daunamycin, thiaxanthenones, miracil D, anthramycin, mitomycin, echinomycin, quinomycin, triostin, diacridines, ellipticene (including dimers, trimers and analogs), norphilin A, fluorenes and flourenones, fluorenodiamines, quinacrine, benzacridines, phenazines, phenanthradines, phenothiazines, chlorpromazine, phenoxazines, benzothiazoles, xanthenes and thio-xanthenes, anthraquinones, anthrapyrazoles, benzothiopyranoindoles, 3,4-benzpyrene, benzopyrene diol epoxidie, 1-pyrenyloxirane, benzanthracene-5,6-oxide, benzodipyrones, benzothiazoles, quinolones, chloroquine, quinine, phenylquinoline carboxamides, furocoumarins (e.g. psoralens, isopsoralens, and sulfur analogs thereof), ethidium salts, propidium, coralyne, ellipticine cation and derivatives, polycyclic hydrocarbons and their oxirane derivatives, and echinimycin; minor groove binders are exemplified by distamycin, mitomycin, netropsin, other lexitropsins, Hoechst 33258 and other Hoechst dyes, DAPI (4',6'-diamidine-2-phenylindole), berenil, and triarylmethane dyes; major groove binders are exemplified by aflatoxins; electrostatic binders are exemplified by spermine, spermidine, and other polyamines; and sequence-specific binders are exemplified by nucleic acids or analogues which bind by such sequence-specific interactions as triple helix formation, D-loop formation, and direct base pairing to single stranded targets. Other sequence-specific binding compounds include poly pyrrole compounds, poly pyrrole imidazole compounds, cyclopropylpyrroloindole compounds and related minor groove binding compounds [Wemmer, Nature Structural Biology, 5(3):169-171 (1998), Wurtz et al., Chemistry & Biology 7(3):153-161 (2000), Anthoney et al., Am. J. Pharmacogenomics 1(1):67-81(2001)].

In addition to targeting nucleic acids, the compounds are also able to react with the nucleic acid, resulting in covalent binding to the nucleic acid. Nucleic acid alkylators are a class of compounds that can react covalently with nucleic acid and include, but are not limited to, mustards (e.g. mono or bis haloethylamine groups, and mono haloethylsulfide groups), mustard equivalents (e.g. epoxides, alpha-halo ketones) and mustard intermediates (e.g. aziridines, aziridiniums and their sulfur analogs), methanesulphonate esters, and nitroso ureas. The nucleic acid alkylators typically react with a nucleophilic group on the nucleic acid. It is the combination of the nucleic acid alkylating activity and the nucleic acid targeting ability of these compounds that gives them the ability to covalently react specifically with nucleic acids, providing the desired modification of the nucleic acid of *Listerias* for use in the present invention. The specificity of these compounds may be further enhanced by the use of a quencher that will not enter the *Listeria.* Such a quencher will quench reactions with the surface of the *Listeria* while still allowing the nucleic acid targeted compounds to react with the Listeria nucleic acid. A discussion of such quenching can be found in US Patent number 6,270,952. The modification of the Listeria nucleic acid can be controlled by adjusting the compound concentration and reaction conditions. The appropriate concentration and reaction conditions are determined by assessing their effects on replication and protein expression as detailed above. The compounds used in the present invention are effective at concentrations of about 10 pM to 10 mM, also about 100 pM to 1 mM, also about 1 nM to 10 µM, also about 1-500 nM, also about 1-200 nM or about 1-100 nM. A discussion of nucleic acid targeted, nucleic acid reactive compounds for specific reaction with nucleic acids, in particular Listerial nucleic acids, can be found in US patents 6,143,490 and 6,093,725.

The nucleic acid can be modified by using a nucleic acid targeted compound that requires activation with radiation in order to cause the nucleic acid modification. Such compounds are targeted to nucleic acids as discussed above. These compounds include, but are not limited to, acridines, acridones, anthyrl derivatives, alloxazines (e.g. riboflavin), benzotriazole derivatives, planar aromatic diazo derivatives, planar aromatic cyano derivatives, toluidines, flavines, phenothiazines (e.g. methylene blue), furocoumarins, angelicins, psoralens, sulfur analogs of psoralens, quinolones, quinolines, quinoxalines, napthyridines, fluoroquinolones, anthraquinones, and anthracenes. Many of these compounds are used as DNA photocleavage agents [Da Ros et al., Current Pharmaceutical Design 7:1781 (2001)]. While the invention is not limited to the method of activation of the nucleic acid targeted compounds, typically, the compounds can be activated with light of particular wavelengths. The effective wavelength of light depends on the nature of the compound and can range anywhere from approximately 200 to 1200 nm. For some of these compounds, activation causes modification of the nucleic acid without direct binding of the compound to the nucleic acid, for example by generating reactive oxygen species in the vicinity of the nucleic acid. For some of these compounds, activation results in binding of the compound directly to the nucleic acid (i.e. the compound binds covalently). Some of these compounds can react with the nucleic acid to form an interstrand crosslink. Psoralens are an example of a class of compounds that crosslink nucleic acids. These compounds are typically activated with UVA light (320-400 nm). Psoralen compounds for use in the present invention are exemplified in US patents 6,133,460 and 5,593,823. Again, it is the combination of nucleic acid targeting and the ability to modify the nucleic acid upon activation that provide specific reactivity with nucleic acids. The modification of the Listerial nucleic acid can be controlled by adjusting the compound concentration, reaction conditions and light dose. The appropriate concentration and light dose are determined by assessing their effects on replication and protein expression as detailed above. In addition to compound concentration and level of light exposure, the reaction is affected by the conditions under which the sample is dosed with UVA light. For example, the required overall concentration for irradiating a population of *Listeria* in a buffered media is going to vary from a population that is cultured in a growth media (e.g. BHI, Triphase Soy Broth). The photoreaction may be affected by the contents of the growth media, which may interact with the psoralen, thereby requiring a higher overall concentration of the psoralen. In addition, the effective dosing of the *Listeria* may depend on the growth phase of the organism and the presence or absence of compound during the growth phase. In one embodiment, the population of *Listeria* comprises growth media during the psoralen UVA treatment. In one embodiment, the psoralen is added to the population of *Listeria,* the population is cultured to grow the *Listeria* in the presence of psoralen and growth media, and the UVA treatment is performed at some point in the growth phase of the *Listeria.* In one embodiment, the population is grown to an OD of 0.5-1 (1 x 10⁷ to 1x 10⁹ CFU/mL) in the presence of the psoralen prior to irradiation with an appropriate dose of UVA light. Psoralen compounds are effective at concentrations of about 10 pM to 10 mM, also about 100 pM to 1 mM, also about 1 nM to 10 µM, also about 1-500 nM, also about 1-200 nM or about 1-100 nM, with the UVA light dose ranging from about 0.1 - 100 J/cm², also about 0.1-20 J/cm², or about 0.5-10 J/cm², 0.5-6 J/cm² or about 2-6 J/cm². In one embodiment, the *Listeria* is treated in the presence of growth media at psoralen concentrations of about 10 pM to 10 mM, also about 1-5000 nM, also about 1-500 nM, also about 5-500 nM, or about 10-400 nM. In one embodiment, the *Listeria* treated in the presence of growth media is grown to an OD of 0.5-1 in the presence of psoralen at concentrations of about 10 pM to 10 mM, also about 1-5000 nM, also about 1-500 nM, also about 5-500 nM, or about 10-400 nM. Following the growth to an OD of 0.5-1, the *Listeria* population is irradiated with UVA light at a dose ranging from about 0.1 - 100 J/cm², also about 0.1-20 J/cm², or about 0.5-10 J/cm², 0.5-6 J/cm² or about 2-6 J/ cm².

In some embodiments, the nucleic acid targeting compound used to modify the nucleic acid of the *Listeria* is an alkylator such as β-alanine, N-(acridin-9-yl), 2-[bis(2-chloroethyl)amino]ethyl ester. In other embodiments, the nucleic acid targeting compound used to modify the nucleic acid of the *Listeria* is a psoralen compound (e.g., 4'-(4-amino-2-oxa)butyl-4,5',8-trimethylpsoralen, also referred to herein as "S-59") activated by UVA irradiation.

In one embodiment, the invention includes a method of making a vaccine composition comprising treating a Listerial population so that the Listerial nucleic acid is modified so that the proliferation of the Listerial population is attenuated, wherein the Listerial gene expression is substantially unaffected. In another embodiment, the invention includes a method of making a vaccine composition comprising treating a Listerial population so that the Listerial nucleic acid is modified so that the proliferation of the Listerial population is attenuated, wherein the Listerial gene expression is substantially unaffected, and then using that Listerial population to load an antigen-presenting cell with antigen and induce activation/maturation of the antigen-presenting cell. In one embodiment, the Listerial population is treated by irradiation. In one embodiment, the Listerial population is treated by reacting with a nucleic acid targeted compound that indirectly causes the modification of the nucleic acid. In a further embodiment, the nucleic acid targeted compound is activated by irradiation, wherein activation of the compound causes the indirect modification of the nucleic acid. In a further embodiment, activation of the nucleic acid targeted compound results in a reactive oxygen species that modifies the nucleic acid. In one embodiment, the Listerial population is treated by reacting with a nucleic acid targeted compound that reacts directly with the nucleic acid. In one embodiment, the nucleic acid targeted compound is reacted at a concentration of about 10 pM to 10 mM, also about 100 pM to 1 mM, also about 1-500 nM, also about 1-200 nM or about 1-100 nM. In one embodiment, the nucleic acid targeted compound comprises an alkylator. In one embodiment, the alkylator is selected from the group consisting of mustards, mustard intermediates and mustard equivalents. In one embodiment, the nucleic acid targeted compound comprises a nucleic acid targeting group selected from the group consisting of intercalators, minor groove binders, major groove binders, electrostatic binders, and sequence-specific binders. In one embodiment, the nucleic acid targeted compound reacts directly with the nucleic acid upon activation of the compound. In one embodiment, the activation of the compound is by irradiation. In one embodiment, the irradiation is UVA irradiation. In a preferred embodiment, the nucleic acid targeted compound is a psoralen compound activated by UVA irradiation. In one embodiment, the psoralen compound is at a concentration of about 10 pM to 10 mM, also about 100 pM to 1 mM, also about 1-500 nM, also about 1-200 nM or about 1-100 nM, and the UVA irradiation is at a dose of about 0.1 - 100 J/cm², also about 0.1-20 J/cm², or about 0.5-5 J/cm² or about 2-4 J/cm². In one embodiment, the proliferation of the Listerial population is attenuated by at least about 0.3 log, also at least about 1 log, about 2 log, about 3 log, about 4 log, about 6 log, or at least about 8 log. In another embodiment, the proliferation of the Listerial population is attenuated by about 0.3 to > 10 log, about 2 to >10 log, about 4 to >10 log, about 6 to >10 log, about 0.3-8 log, about 0.3-6 log, about 0.3-5 log, about 1-5 log, or about 2-5 log. In one embodiment, the expression of an antigen by the Listerial population is at least about 10%, about 25%, about 50%, about 75%, or at least about 90% of the expression of the antigen by a Listerial population that has not been treated to modify the nucleic acid. In one embodiment, the antigen expressed is an antigen from the *Listeria* itself. In one embodiment, the *Listeria* comprises a heterologous nucleic acid sequence encoding an antigen. In one embodiment, the antigen is a disease associated antigen. In one embodiment, the antigen is associated with a disease selected from the group consisting of infectious diseases, autoimmune diseases, allergies, cancers, and other hyperproliferative diseases. In one embodiment, the antigen is a tumor associated antigen. In one embodiment, the tumor antigen is selected from the group consisting of differentiation antigens, tissue specific antigens, developmental antigens, tumor-associated viral antigens, cancer-testis antigens, embryonic antigens, oncoprotein antigens, over-expressed protein antigens and mutated protein antigens. In one embodiment, the tumor antigen is selected from the group consisting of mesothelin, Sp17, gp100, PR3, PAGE-4, TARP, WT-1, NY-ESO-1 and SPAS-1. In one embodiment, the *Listeria* comprises a genetic mutation. In one embodiment, the genetic mutation results in the attenuation of the ability of the *Listeria* to repair Listerial nucleic acid that has been modified. In one embodiment, the genetic mutation is in the gene selected from the group consisting of *phrB, uvrA, uvrB, uvrC, uvrD* and *recA,* or their functionally equivalent genes, depending on the genus and species of the *Listeria.* In one embodiment, the genetic mutation is in one or more of the genes selected from the group consisting of *phrB, uvrA, uvrB, uvrC, uvrD* and *recA,* or their functionally equivalent genes. In one embodiment, the genetic mutation results in the attenuation in the activity of a DNA repair enzyme selected from the group consisting of PhrB, UvrA, UvrB, UvrC, UvrD and RecA. In a further embodiment, *Listeria* having these mutations are treated with a psoralen activated by UVA irradiation. In an embodiment, the *Listeria* is *Listeria monocytogenes.* In one embodiment, the *Listeria* comprises a mutation that results in the attenuation of the ability of the *Listeria* to invade non-phagocytic cells without significantly affecting the uptake of the *Listeria* by phagocytic cells. In one embodiment, the *Listeria* mutation is in an internalin gene(s). In one embodiment, the *Listeria* mutation is in the gene selected from the group consisting of *inlA*, *inlB*, and any gene encoding an internalin. In one embodiment, the *Listeria monocytogenes* comprises a genetic mutation in both the *inlA* and *inlB* genes. In one embodiment, the *Listeria* comprises a mutation that results in the attenuation of the ability of the *Listeria* to escape the phagolysosome of an infected cell. In one embodiment, the *Listeria* mutation is in the *hly* gene. In one embodiment, the *Listeria* comprises a mutation that results in the attenuation of the polymerization of actin by the *Listeria.* In a preferred embodiment, the *Listeria* mutation is in the *actA* gene. In one embodiment, the *Listeria* comprises mutations in the *actA* gene and one or more internalin genes. In a preferred embodiment, the *Listeria* comprises a mutation in the *actA* gene and the *inlB* gene, preferably the *Listeria* comprises an *actA*/*inlB* deletion mutant. In a preferred embodiment, the *Listeria monocytogenes actA*/*inlB* deletion mutant further comprises a deletion mutation in the *uvrAB* gene.

The *Listeria,* may, in some embodiments, be attenuated by a nucleic acid targeting compound. In some embodiments, the nucleic-acid targeting compound is a nucleic acid alkylator, such as β-alanine, N-(acridin-9-yl), 2-[bis(2-chloroethyl)amino]ethyl ester. In some embodiments, the nucleic acid targeting compound is activated by irradiation, such as UVA irradiation. In some embodiments, the *Listeria* is treated with a psoralen compound. For instance, in some embodiments, the bacterium are modified by treatment with a psoralen, such as 4'-(4-amino-2-oxa)butyl-4,5',8-trimethylpsoralen ("S-59"), and UVA light. In some embodiments, the nucleic acid of the bacterium has been modified by treatment with a psoralen compound and UVA irradiation. Descriptions of methods of modifying bacteria to attenuate them for proliferation using nucleic acid targeting compounds are described in U.S. Patent Pub. No. 2004/0197343 and Brockstedt, et al., Nat. Med., 11:853-60 (2005). In some embodiments, the *Listeria* is attenuated for DNA repair.

For example, for treatment of *Listeria* such as Δ*actA*Δ*uvrAB L. monocytogenes,* in some embodiments, S-59 psoralen can be added to 200 nM in a log-phase culture of (approximately) OD₆₀₀=0.5, followed by inactivation with 6 J/m² of UVA light when the culture reaches an optical density of one. Inactivation conditions are optimized by varying concentrations of S-59, UVA dose, the time of S-59 exposure prior to UVA treatment as well as varying the time of treatment during bacterial growth of the *Listeria actA*/*uvrAB* strain. The parental *Listeria* strain is used as a control. Inactivation of *Listeria* (log-kill) is determined by the inability of the bacteria to form colonies on BHI (Brain heart infusion) agar plates. In addition, one can confirm the continued metabolic activity and expression of proteins such as LLO in the bacteria in the S-59/UVA inactivated *Listeria* using ³⁵S-pulse-chase experiments to determine the synthesis and secretion of newly expressed proteins post S-59 / UVA inactivation. Expression of LLO using ³⁵S-metabolic labeling can be routinely determined. S-59/UVA inactivated *Listeria actA*/*uvrAB* can be incubated for 1 hour in the presence of ³⁵S-Methionine. Expression and/or secretion of proteins such as LLO can be determined of both whole cell lysates, and TCA precipitation of bacterial culture fluids. LLO-specific monoclonal antibodies can be used for immunoprecipitation to verify the continued expression and secretion from recombinant *Listeria* post inactivation.

In some embodiments, the *Listeria* attenuated for proliferation are also attenuated for nucleic acid repair and/or are defective with respect to at least one DNA repair enzyme. For instance, in some embodiments, the bacterium in which nucleic acid has been modified by a nucleic acid targeting compound such as a psoralen (combined with UVA treatment) is a *uvrAB* deletion mutant.

In some embodiments, the proliferation of the *Listeria* is attenuated by at least about 0.3 log, also at least about 1 log, about 2 log, about 3 log, about 4 log, about 6 log, or at least about 8 log. In another embodiment, the proliferation of the *Listeria* is attenuated by about 0.3 to > 10 log, about 2 to >10 log, about 4 to >10 log, about 6 to >10 log, about 0.3-8 log, about 0.3-6 log, about 0.3-5 log, about 1-5 log, or about 2-5 log. In some embodiments, the expression of LLO by the *Listeria* is at least about 10%, about 25%, about 50%, about 75%, or at least about 90% of the expression of LLO in non-modified *Listeria.*

### VI. Pharmaceutical compositions, immunogenic compositions, and/or vaccines

A variety of different compositions such as pharmaceutical compositions, immunogenic compositions, and vaccines comprising the *Listeria* described herein are also provided by the present disclosure. In some embodiments, the compositions are isolated.

As used herein, "carriers" includes any and all solvents, dispersion media, vehicles, coatings, diluents, antifungal agents, isotonic and absorption delaying agents, buffers, carrier solutions, suspensions, colloids, and the like. Pharmaceutically acceptable carriers are well known to those of ordinary skill in the art, and include any material which, when combined with an active ingredient, allows the ingredient to retain biological activity and is non-reactive with the subject's immune system. For instance, pharmaceutically acceptable carriers include, but are not limited to, water, buffered saline solutions (e.g., 0.9% saline), emulsions such as oil/water emulsions, and various types of wetting agents. Possible carriers also include, but are not limited to, oils (e.g., mineral oil), dextrose solutions, glycerol solutions, chalk, starch, salts, glycerol, and gelatin.

While any suitable carrier known to those of ordinary skill in the art may be employed in the pharmaceutical compositions, the type of carrier will vary depending on the mode of administration. Compositions of the present invention may be formulated for any appropriate manner of administration, including for example, topical, oral, nasal, intravenous, intracranial, intraperitoneal, subcutaneous or intramuscular administration. In some embodiments, for parenteral administration, such as subcutaneous injection, the carrier comprises water, saline, alcohol, a fat, a wax or a buffer. In some embodiments, any of the above carriers or a solid carrier, such as mannitol, lactose, starch, magnesium stearate, sodium saccharine, talcum, cellulose, glucose, sucrose, and magnesium carbonate, are employed for oral administration.

Compositions comprising such carriers are formulated by well known conventional methods (see, for example, Remington's Pharmaceutical Sciences, 18th edition, A. Gennaro, ed., Mack Publishing Co., Easton, PA, 1990; and Remington, The Science and Practice of Pharmacy 20th Ed. Mack Publishing, 2000).

In addition to pharmaceutical compositions, immunogenic compositions are provided. For instance, the invention provides an immunogenic composition comprising a recombinant bacterium described herein.

In some embodiments, the recombinant bacterium in the immunogenic composition releases the polypeptide comprising the antigen at a level sufficient to induce an immune response to the antigen upon administration of the composition to a host (e.g., a mammal such as a human). In some embodiments, the immune response stimulated by the immunogenic composition is a cell-mediated immune response. In some embodiments, the immune response stimulated by the immunogenic composition is a humoral immune response. In some embodiments, the immune response stimulated by the immunogenic composition comprises both a humoral and cell-mediated immune response.

It can be determined if a particular form of recombinant bacteria (and/or a particular expression cassette) is useful in an immunogenic composition (or as a vaccine) by testing the ability of the recombinant bacteria to stimulate an immune response *in vitro* or in a model system.

These immune cell responses can be measured by both *in vitro* and *in vivo* methods to determine if the immune response of a particular recombinant bacterium (and/or a particular expression cassette) is effective. One possibility is to measure the presentation of the protein or antigen of interest by an antigen-presenting cell that has been mixed with a population of the recombinant bacteria. The recombinant bacteria may be mixed with a suitable antigen presenting cell or cell line, for example a dendritic cell, and the antigen presentation by the dendritic cell to a T cell that recognizes the protein or antigen can be measured. If the recombinant bacteria are expressing the protein or antigen at a sufficient level, it will be processed into peptide fragments by the dendritic cells and presented in the context of MHC class I or class II to T cells. For the purpose of detecting the presented protein or antigen, a T cell clone or T cell line responsive to the particular protein or antigen may be used. The T cell may also be a T cell hybridoma, where the T cell is immortalized by fusion with a cancer cell line. Such T cell hybridomas, T cell clones, or T cell lines can comprise either CD8+ or CD4+ T cells. The dendritic cell can present to either CD8+ or CD4+ T cells, depending on the pathway by which the antigens are processed. CD8+ T cells recognize antigens in the context of MHC class I while CD4+ recognize antigens in the context of MHC class II. The T cell will be stimulated by the presented antigen through specific recognition by its T cell receptor, resulting in the production of certain proteins, such as IL-2, tumor necrosis factor-α (TNF-α), or interferon-γ (IFN-γ), that can be quantitatively measured (for example, using an ELISA assay, ELISPOT assay, or Intracellular Cytokine Staining (ICS)). These are techniques that are well known in the art.

Alternatively, a hybridoma can be designed to include a reporter gene, such as β-galactosidase, that is activated upon stimulation of the T cell hybridoma by the presented antigens. The increase in the production of β-galactosidase can be readily measured by its activity on a substrate, such as chlorophenol red-B-galactoside, which results in a color change. The color change can be directly measured as an indicator of specific antigen presentation.

Additional *in vitro* and *in vivo* methods for assessing the antigen expression of recombinant bacteria vaccines of the present invention are known to those of ordinary skill in the art. It is also possible to directly measure the expression of a particular heterologous antigen by recombinant bacteria. For example, a radioactively labeled amino acid can be added to a cell population and the amount of radioactivity incorporated into a particular protein can be determined. The proteins synthesized by the cell population can be isolated, for example by gel electrophoresis or capillary electrophoresis, and the amount of radioactivity can be quantitatively measured to assess the expression level of the particular protein. Alternatively, the proteins can be expressed without radioactivity and visualized by various methods, such as an ELISA assay or by gel electrophoresis and Western blot with detection using an enzyme linked antibody or fluorescently labeled antibody.

Elispot assay, Intracellular Cytokine Staining Assay (ICS), measurement of cytokine expression of stimulated spleen cells, and assessment of cytotoxic T cell activity *in vitro* and *in vivo* are all techniques for assessing immunogenicity known to those in the art.

In addition, therapeutic efficacy of the vaccine composition can be assessed more directly by administration of the immunogenic composition or vaccine to an animal model such as a mouse model, followed by an assessment of survival or tumor growth. For instance, survival can be measured following administration of the *Listeria* and challenge.

Mouse models useful for testing the immunogenicity of an immunogenic composition or vaccine expressing a particular antigen can be produced by first modifying a tumor cell so that it expresses the antigen of interest or a model antigen and then implanting the tumor cells expressing the antigen of interest into mice. The mice can be vaccinated with the candidate immunogenic composition or vaccine comprising a recombinant bacterium expressing a polypeptide comprising the antigen of interest or a model antigen prior to implantation of the tumor cells (to test prophylactic efficacy of the candidate composition) or following implantation of the tumor cells in the mice (to test therapeutic efficacy of the candidate composition).

As an example, CT26 mouse murine colon carcinoma cells can be transfected with an appropriate vector comprising an expression cassette encoding the desired antigen or model antigen using techniques standard in the art. Standard techniques such as flow cytometry and Western blots can then be used to identify clones expressing the antigen or model antigen at sufficient levels for use in the immunogenicity and/or efficacy assays.

Alternatively, candidate compositions can be tested which comprise a recombinant bacterium expressing an antigen that corresponds to or is derived from an antigen endogenous to a tumor cell line (e.g., the retroviral gp70 tumor antigen AH1 endogenous to CT26 mouse murine colon carcinoma cells, or the heteroclitic epitope AH1-A5). In such assays, the tumor cells can be implanted in the animal model without further modification to express an additional antigen. Candidate vaccines comprising the antigen can then be tested.

As indicated, vaccine compositions comprising the bacteria described herein are also provided.

In some embodiments, the vaccine compositions comprise antigen-presenting cells (APC) which have been infected with any of the recombinant bacteria described herein. In some embodiments the vaccine (or immunogenic or pharmaceutical composition) does not comprise antigen-presenting cells (i.e., the vaccine or composition is a bacteria-based vaccine or composition, not an APC-based vaccine or composition).

Methods of administration suitable for administration of vaccine compositions (and pharmaceutical and immunogenic compositions) are known in the art, and include oral, intravenous, intradermal, intraperitoneal, intramuscular, intralymphatic, intranasal and subcutaneous routes of administration.

Vaccine formulations are known in the art and in some embodiments may include numerous additives, such as preservatives (e.g., thimerosal, 2-phenyoyx ethanol), stabilizers, adjuvants (e.g. aluminum hydroxide, aluminum phosphate, cytokines), antibiotics (e.g., neomycin, streptomycin), and other substances. In some embodiments, stabilizers, such as lactose or monosodium glutamate (MSG), are added to stabilize the vaccine formulation against a variety of conditions, such as temperature variations or a freeze-drying process. In some embodiments, vaccine formulations may also include a suspending fluid or diluent such as sterile water, saline, or isotonic buffered saline (e.g., phosphate buffered to physiological pH). Vaccine may also contain small amount of residual materials from the manufacturing process.

For instance, in some embodiments, the vaccine compositions are lyophilized (i.e., freeze-dried). The lyophilized preparation can be combined with a sterile solution (e.g., citrate-bicarbonate buffer, buffered water, 0.4% saline, or the like) prior to administration.

In some embodiments, the vaccine compositions may further comprise additional components known in the art to improve the immune response to a vaccine, such as adjuvants or co-stimulatory molecules. In addition to those listed above, possible adjuvants include chemokines and bacterial nucleic acid sequences, like CpG. In some embodiments, the vaccines comprise antibodies that improve the immune response to a vaccine, such as CTLA4. In some embodiments, co-stimulatory molecules comprise one or more factors selected from the group consisting of GM-CSF, IL-2, IL-12, IL-14, IL-15, IL-18, B7.1, B7.2, and B7-DC are optionally included in the vaccine compositions of the present invention. Other co-stimulatory molecules are known to those of ordinary skill in the art.

In additional aspects, the present disclosure provides methods of improving a vaccine or immunogenic composition comprising *Listeria* that express an antigen.

Methods of producing the recombinant *Listeria,* immunogenic composition or vaccine of the present disclosure are also provided. For instance, in one embodiment, a method of producing a vaccine comprising a recombinant bacterium (e.g. a recombinant *Listeria* bacterium) comprises introducing a recombinant nucleic acid molecule into the bacterium, wherein the recombinant nucleic acid molecule encodes an antigen. In some cases, the recombinant *Listeria* comprises a PrfA* mutation. In other cases, the recombinant *Listeria* comprises a null *prfA* allele. In this case, a genetically engineered *prfA** allele is integrated into the Listeria genome; for example in the *tRNA^{arg}* gene (Port, G.C. and Freitag, N.E. (2007) Infect. Immunity 75:5886-5897). In some embodiments, a recombinant polynucleotide operably linked to a PrfA responsive regulatory element is introduced into the recombinant PrfA* bacterium, wherein the recombinant polynucleotide encodes an antigen. In some embodiments, a recombinant nucleic acid molecule comprising (a) a first polynucleotide encoding a signal peptide and (b) a second polynucleotide encoding an antigen, wherein the second polynucleotide is in the same translational reading frame as the first polynucleotide, wherein the recombinant nucleic acid molecule encodes a fusion protein comprising the signal peptide and the antigen and wherein the fusion protein is operably linked to a PrfA responsive regulatory element, is introduced into a PrfA* bacterium to produce the vaccine. The recombinant nucleic acid molecule used to produce the vaccine is, in some embodiments, a recombinant nucleic acid molecule, comprising (a) a first polynucleotide encoding a Listerial ActA, ActA fragment or variant thereof, and (b) a second polynucleotide encoding a polypeptide, wherein the second polynucleotide is in the same translational reading frame as the first polynucleotide, wherein the recombinant nucleic acid molecule encodes a protein chimera in which the non-Listerial polypeptide is fused to the ActA, ActA fragment or variant thereof, or is inserted within the ActA, ActA fragment or variant thereof.

### VII. Methods of Use

A variety of methods of using the *Listeria* or pharmaceutical, immunogenic, or vaccine compositions described herein for inducing immune responses, and/or preventing or treating conditions in a host (e.g., a mammal) are provided. In some embodiments, the condition that is treated or prevented is a disease. In some embodiments, the disease is cancer. In some embodiments, the disease is an infectious disease.

As used herein, "treatment" or "treating" (with respect to a condition or a disease) encompasses an approach for obtaining beneficial or desired results. In preferred embodiments, these results include clinical results. For purposes of this invention, beneficial or desired results with respect to a disease may include, but are not limited to, one or more of the following: improving a condition associated with a disease, curing a disease, lessening severity of a disease, delaying progression of a disease, alleviating one or more symptoms associated with a disease, increasing the quality of life of one suffering from a disease, and/or prolonging survival. Likewise, for purposes of this invention, beneficial or desired results with respect to a condition may include, but are not limited to, one or more of the following: improving a condition, curing a condition, lessening severity of a condition, delaying progression of a condition, alleviating one or more symptoms associated with a condition, increasing the quality of life of one suffering from a condition, and/or prolonging survival. For instance, in those embodiments where the compositions described herein are used for treatment of cancer, the beneficial or desired results may include, but are not limited to, one or more of the following: reducing the proliferation of (or destroying) neoplastic or cancerous cells, reducing metastasis of neoplastic cells found in cancers, shrinking the size of a tumor, decreasing symptoms resulting from the cancer, increasing the quality of life of those suffering from the cancer, decreasing the dose of other medications required to treat the disease, delaying the progression of the cancer, and/or prolonging survival of patients having cancer.

As used herein, the terms "preventing" disease or "protecting a host" from disease (used interchangeably herein) encompass, but are not limited to, one or more of the following: stopping, deferring, hindering, slowing, retarding, and/or postponing the onset or progression of a disease, stabilizing the progression of a disease, and/or delaying development of a disease. The terms "preventing" a condition or "protecting a host" from a condition (used interchangeably herein) encompass, but are not limited to, one or more of the following: stopping, deferring, hindering, slowing, retarding, and/or postponing the onset or progression of a condition, stabilizing the progression of a condition, and/or delaying development of a condition. The period of this prevention can be of varying lengths of time, depending on the history of the disease or condition and/or individual being treated. By way of example, where the vaccine is designed to prevent or protect against an infectious disease caused by a pathogen, the terms "preventing" disease or "protecting a host" from disease encompass, but are not limited to, one or more of the following: stopping, deferring, hindering, slowing, retarding, and/or postponing the infection by a pathogen of a host, progression of an infection by a pathogen of a host, or the onset or progression of a disease associated with infection of a host by a pathogen, and/or stabilizing the progression of a disease associated with infection of a host by a pathogen. Also, by way of example, where the vaccine is an anti-cancer vaccine, the terms "preventing" disease or "protecting the host" from disease encompass, but are not limited to, one or more of the following: stopping, deferring, hindering, slowing, retarding, and/or postponing the development of cancer or metastasis, progression of a cancer, or a reoccurrence of a cancer.

In one aspect, described herein is a method of inducing an immune response in a host (e.g., mammal) to an antigen, comprising administering to the host an effective amount of a bacterium described herein or an effective amount of a composition (e.g., a pharmaceutical composition, immunogenic composition, or vaccine) comprising a bacterium described herein.

In some embodiments, the immune response is an MHC Class I immune response. In other embodiments, the immune response is an MHC Class II immune response. In still other embodiments, the immune response that is induced by administration of the bacteria or compositions is both an MHC Class I and an MHC Class II response. Accordingly, in some embodiments, the immune response comprises a CD4+ T-cell response. In some embodiments, the immune response comprises a CD8+ T-cell response. In some embodiments, the immune response comprises both a CD4+ T-cell response and a CD8+ T-cell response. In some embodiments, the immune response comprises a B-cell response and/or a T-cell response. B-cell responses may be measured by determining the titer of an antibody directed against the antigen, using methods known to those of ordinary skill in the art. In some embodiments, the immune response which is induced by the compositions described herein is a humoral response. In other embodiments, the immune response which is induced is a cellular immune response. In some embodiments, the immune response comprises both cellular and humoral immune responses. In some embodiments, the immune response is antigen-specific. In some embodiments, the immune response is an antigen-specific T-cell response.

In addition to methods of inducing immune responses, described herein are methods of preventing or treating a condition or disease in a host (e.g., a mammalian subject such as human patient). The methods comprise administration to the host of an effective amount of a bacterium described herein, or a composition comprising a bacterium described herein. In some embodiments, the disease is cancer. In some embodiments, the disease is an infectious disease.

In some embodiments, the disease is cancer. In some embodiments, where the condition being treated or prevented is cancer, the disease is melanoma, breast cancer, pancreatic cancer, liver cancer, colon cancer, colorectal cancer, lung cancer, brain cancer, testicular cancer, ovarian cancer, squamous cell cancer, gastrointestinal cancer, cervical cancer, kidney cancer, thyroid cancer or prostate cancer. In some embodiments, the cancer is melanoma. In some embodiments, the cancer is pancreatic cancer. In some embodiments, the cancer is colon cancer. In some embodiments, the cancer is prostate cancer. In some embodiments, the cancer is metastatic.

In other embodiments, the disease is an infectious disease or another disease caused by a pathogen such as a virus, bacterium, fungus, or protozoa. In some embodiments, the disease is an infectious disease.

In some embodiments, the use of the *Listeria* in the prophylaxis or treatment of a cancer comprises the delivery of the *Listeria* to cells of the immune system of an individual to prevent or treat a cancer present or to which the individual has increased risk factors, such as environmental exposure and/or familial disposition. In other embodiments, the use of the bacteria in the prophylaxis or treatment of a cancer comprises delivery of the bacteria to an individual who has had a tumor removed or has had cancer in the past, but is currently in remission.

In some embodiments, administration of composition comprising a bacterium described herein to a host elicits a CD4+ T-cell response in the host. In some other embodiments, administration of a composition comprising a bacterium described herein to a host elicits a CD8+ T-cell response in the host. In some embodiments, administration of a composition comprising a bacterium described herein elicits both a CD4+ T-cell response and a CD8+ T-cell response in the host.

The efficacy of the vaccines or other compositions for the treatment of a condition can be evaluated in an individual, for example in mice. A mouse model is recognized as a model for efficacy in humans and is useful in assessing and defining the vaccines of the present invention. The mouse model is used to demonstrate the potential for the effectiveness of the vaccines in any individual. Vaccines can be evaluated for their ability to provide either a prophylactic or therapeutic effect against a particular disease. For example, in the case of infectious diseases, a population of mice can be vaccinated with a desired amount of the appropriate vaccine of the invention, where the bacterium expresses an infectious disease associated antigen. The mice can be subsequently infected with the infectious agent related to the vaccine antigen and assessed for protection against infection. The progression of the infectious disease can be observed relative to a control population (either non vaccinated or vaccinated with vehicle only or a bacterium that does not contain the appropriate antigen).

In the case of cancer vaccines, tumor cell models are available, where a tumor cell line expressing a desired tumor antigen can be injected into a population of mice either before (therapeutic model) or after (prophylactic model) vaccination with a composition comprising a bacterium of the invention containing the desired tumor antigen. Vaccination with a bacterium containing the tumor antigen can be compared to control populations that are either not vaccinated, vaccinated with vehicle, or with a bacterium that expresses an irrelevant antigen. The effectiveness of the vaccine in such models can be evaluated in terms of tumor volume as a function of time after tumor injection or in terms of survival populations as a function of time after tumor injection. In one embodiment, the tumor volume in mice vaccinated with a composition comprising the bacterium is about 5%, about 10%, about 25%, about 50%, about 75%, about 90% or about 100% less than the tumor volume in mice that are either not vaccinated or are vaccinated with vehicle or a bacterium that expresses an irrelevant antigen. In another embodiment, this differential in tumor volume is observed at least about 10, about 17, or about 24 days following the implant of the tumors into the mice. In one embodiment, the median survival time in the mice vaccinated with the composition comprising a bacterium is at least about 2, about 5, about 7 or at least about 10 days longer than in mice that are either not vaccinated or are vaccinated with vehicle or bacteria that express an irrelevant antigen.

The host (i.e., subject) in the methods described herein, is any vertebrate, preferably a mammal, including domestic animals, sport animals, and primates, including humans. In some embodiments, the host is a mammal. In some embodiments, the host is a human.

The delivery of the *Listeria,* or a composition comprising the strain, may be by any suitable method, such as intradermal, subcutaneous, intraperitoneal, intravenous, intramuscular, intralymphatic, oral or intranasal, as well as by any route that is relevant for any given malignant or infectious disease or other condition. In some embodiments, the method of administration is mucosal.

The compositions comprising the bacteria and an immunostimulatory agent may be administered to a host simultaneously, sequentially or separately. Examples of immunostimulatory agents include, but are not limited to IL-2, IL-12, GMCSF, IL-15, B7.1, B7.2, and B7-DC and IL-14.

As used herein, an "effective amount" of a bacterium or composition (such as a pharmaceutical composition or an immunogenic composition) is an amount sufficient to effect beneficial or desired results. For prophylactic use, beneficial or desired results includes results such as eliminating or reducing the risk, lessening the severity, or delaying the outset of the disease, including biochemical, histologic and/or behavioral symptoms of a disease, its complications and intermediate pathological phenotypes presenting during development of the disease. For therapeutic use, beneficial or desired results includes clinical results such as inhibiting or suppressing a disease, decreasing one or more symptoms resulting from a disease (biochemical, histologic and/or behavioral), including its complications and intermediate pathological phenotypes presenting during development of a disease, increasing the quality of life of those suffering from a disease, decreasing the dose of other medications required to treat the disease, enhancing effect of another medication, delaying the progression of the disease, and/or prolonging survival of patients. An effective amount can be administered in one or more administrations. For purposes of this invention, an effective amount of drug, compound, or pharmaceutical composition is an amount sufficient to accomplish prophylactic or therapeutic treatment either directly or indirectly. As is understood in the clinical context, an effective amount of a drug, compound, or pharmaceutical composition may or may not be achieved in conjunction with another drug, compound, or pharmaceutical composition. Thus, an effective amount may be considered in the context of administering one or more therapeutic agents, and a single agent may be considered to be given in an effective amount if, in conjunction with one or more other agents, a desirable result may be or is achieved.

In some embodiments, for a therapeutic treatment of a cancer, an effective amount includes an amount that will result in the desired immune response, wherein the immune response either slows the growth of the targeted tumors, reduces the size of the tumors, or preferably eliminates the tumors completely. The administration of the vaccine may be repeated at appropriate intervals, and may be administered simultaneously at multiple distinct sites in the vaccinated individual. In some embodiments, for a prophylactic treatment of a cancer, an effective amount includes a dose that will result in a protective immune response such that the likelihood of an individual to develop the cancer is significantly reduced. The vaccination regimen may be comprised of a single dose, or may be repeated at suitable intervals until a protective immune response is established.

Described herein are methods for eliciting an immune response and in particular methods for eliciting a boost immune response, including an enhanced boost response to a target antigen present in a priming vaccine administered to a mammal. The target antigens may be those associated with a disease state, for example, those identified as present on a cancerous cell or a pathogenic agent. Subsequent to an effective dose of the priming vaccine, a second vaccine comprising an attenuated metabolically active PrfA* *Listeria* that encodes and expresses an immunologically active portion of the target antigen is administered. In the described herein an initial immune response is elicited by administering to the mammal an effective dose of a priming vaccine. The priming vaccine, with the exception of PrfA* *Listeria,* does not contain metabolically active *Listeria* that encodes the target antigen. The priming vaccine may contain either the target antigen itself, for example, a protein with or without an adjuvant, a tumor cell lysate, an irradiated tumor cell, an antigen-presenting cell pulsed with peptides of the target antigen (e.g. a dendritic cell), or it may contain an agent that provides the target antigen. Suitable agents that provide a target antigen include recombinant vectors, for example, bacteria, viruses, and naked DNA. Recombinant vectors are prepared using standard techniques known in the art, and contain suitable control elements operably linked to the nucleotide sequence encoding the target antigen. See, for example, Plotkin, et al. (eds.) (2003) Vaccines, 4th ed., W.B. Saunders, Co., Phila., PA.; Sikora, et al. (eds.) (1996) Tumor Immunology Cambridge University Press, Cambridge, UK; Hackett and Harn (eds.) Vaccine Adjuvants, Humana Press, Totowa, NJ; Isaacson (eds.) (1992) Recombinant DNA Vaccines, Marcel Dekker, NY, NY; Morse, et al. (eds.) (2004) Handbook of Cancer Vaccines, Humana Press, Totowa, NJ), Liao, et al. (2005) Cancer Res. 65:9089-9098; Dean (2005) Expert Opin. Drug Deliv. 2:227-236; Arlen, et al. (2003) Expert Rev. Vaccines 2:483-493; Dela Cruz, et al. (2003) Vaccine 21:1317-1326; Johansen, et al. (2000) Eur. J. Pharm. Biopharm. 50:413-417; Excler (1998) Vaccine 16:1439-1443; Disis, et al. (1996) J. Immunol. 156:3151-3158). Peptide vaccines are described (see, e.g., McCabe, et al. (1995) Cancer Res. 55:1741-1747; Minev, et al. (1994) Cancer Res. 54:4155-4161; Snyder, et al. (2004) J. Virology 78:7052-7060.

Virus-derived vectors include viruses, modified viruses, and viral particles. The virus-derived vectors can be administered directly to a mammalian subject, or can be introduced *ex vivo* into an antigen presenting cell (APC), where the APC is then administered to the subject.

Viral vectors may be based on, e.g., Togaviruses, including alphaviruses and flaviviruses; alphaviruses, such as Sindbis virus, Sindbis strain SAAR86, Semliki Forest virus (SFV), Venezuelan equine encephalitis (VEE), Eastern equine encephalitis (EEE), Western equine encephalitis, Ross River virus, Sagiyami virus, O'Nyong-nyong virus, Highlands J virus. Flaviviruses, such as Yellow fever virus, Yellow fever strain 17D, Japanese encephalitis, St. Louis encephalitis, Tick-borne encephalitis, Dengue virus, West Nile virus, Kunjin virus (subtype of West Nile virus); arterivirus such as equine arteritis virus; and rubivirus such as rubella virus, herpesvirus, modified vaccinia Ankara (MVA); avipox viral vector; fowlpox vector; vaccinia virus vector; influenza virus vector; adenoviral vector, human papilloma virus vector; bovine papilloma virus vector, and so on. Viral vectors may be based on an orthopoxvirus such as variola virus (smallpox), vaccinia virus (vaccine for smallpox), Ankara (MVA), or Copenhagen strain, camelpox, monkeypox, or cowpox. Viral vectors may be based on an avipoxvirus virus, such as fowlpox virus or canarypox virus. Viral vectors may be based on Vesicular Stomatitis Virus (VSV) or Yellow Fever Virus (YFV).

Adenoviral vectors and adeno-associated virus vectors (AAV) are available, where adenoviral vectors include adenovirus serotype 5 (adeno5; Ad5), adeno6, adeno11, adeno26 and adeno35. Available are at least 51 human adenovirus serotypes, classified into six subgroups (subgroups A, B, C, D, E, and F). Adenovirus proteins useful, for example, in assessing immune response to an "empty" advenoviral vector, include hexon protein, such as hexon 3 protein, fiber protein, and penton base proteins, and human immune responses to adenoviral proteins have been described (see, e.g., Wu, et al. (2002) J. Virol. 76:12775-12782; Mascola (2006) Nature 441:161-162; Roberts, et al. (2006) Nature 441:239-243).

Antigen presenting cell (APC) vectors, such as a dendritic cell (DC) vector, include cells that are loaded with an antigen, loaded with a tumor lysate, or transfected with a composition comprising a nucleic acid, where the nucleic acid can be, e.g., a plasmid, mRNA, or virus. DC/tumor fusion vaccines may also be used. See, e.g., Di Nicola, et al. (2004) Clin. Cancer Res. 10:5381-5390; Cerundolo, et al. (2004) Nature Immunol. 5:7-10; Parmiani, et al. (2002) J. Natl. Cancer Inst. 94:805-818; Kao, et al. (2005) Immunol. Lett. 101:154-159; Geiger, et al. (2005) J. Transl. Med. 3:29; Osada, et al. (2005) Cancer Immunol. Immunother. Nov.5,1-10 [epub ahead of print]; Malowany, et al. (2005) Mol. Ther. 13:766-775; Morse and Lyerly (2002) World J. Surg. 26:819-825; Gabrilovich (2002) Curr. Opin. Mol. Ther. 4:454-458; Morse, et al. (2003) Clin. Breast Cancer 3 Suppl.4:S164-S172; Morse, et al. (2002) Cancer Chemother. Biol. Response Modif. 20:385-390; Arlen, et al. (2003) Expert Rev. Vaccines 2:483-493; Morse and Lyerly (1998) Expert Opin. Investig. Drugs 7:1617-1627; Hirschowitz, et al. (2004) J. Clin. Oncol. 22:2808-2815; Vasir, et al. (2005) Br. J. Haematol. 129:687-700; Koido, et al. (2005) Gynecol. Oncol. 99:462-471.

Tumor cells, for example, autologous and allogeneic tumor cells, are available as vaccines (Arlen, et al. (2005) Semin. Oncol. 32:549-555). A vaccine may also comprise a modified tumor cell, for example, a tumor cell lysate, or an irradiated tumor cell. The tumor cell can also be modified by incorporating a nucleic acid encoding an molecule such as a cytokine (GM CSF, IL 12, IL 15, and the like), a NKG2D ligand, CD40L, CD80, CD86, and the like (see, e.g., Dranoff (2002) Immunol. Rev. 188:147-154; Jain, et al. (2003) Ann. Surg. Oncol. 10:810-820; Borrello and Pardoll (2002) Cytokine Growth Factor Rev. 13:185-193; Chen, et al. (2005) Cancer Immunol. Immunother. 27:1-11; Kjaergaard, et al. (2005) J. Neurosurg. 103:156-164; Tai, et al. (2004) J. Biomed. Sci. 11:228-238; Schwaab, et al. (2004) J. Urol. 171:1036-1042; Friese, et al. (2003) Cancer Res. 63:8996-9006; Briones, et al. (2002) Cancer Res. 62:3195-3199; Vieweg and Dannull (2003) Urol. Clin. North Am. 30:633-643; Mincheff, et al. (2001) Crit. Rev. Oncol. Hematol. 39:125-132).

Vaccines may include naked DNA vectors and naked RNA vectors. These vaccines containing nucleic acids may be administered by a gene gun, electroporation, bacterial ghosts, microspheres, microparticles, liposomes, polycationic nanoparticles, and the like (see, e.g., Donnelly, et al. (1997) Ann. Rev. Immunol. 15:617-648; Mincheff, et al. (2001) Crit. Rev. Oncol. Hematol. 39:125-132; Song, et al. (2005) J. Virol. 79:9854-9861; Estcourt, et al. (2004) Immunol. Rev. 199:144-155).

Reagents and methodologies for administration of naked nucleic acids, e.g., by way of a gene gun, intradermic, intramuscular, and electroporation methods, are available. The nucleic acid vaccines may comprise a locked nucleic acid (LNA), where the LNA allows for attachment of a functional moiety to the plasmid DNA, and where the functional moiety can be an adjuvant (see, e.g., Fensterle, et al. (1999) J. Immunol. 163:4510-4518; Strugnell, et al. (1997) Immunol. Cell Biol. 75:364-369; Hertoughs, et al. (2003) Nucleic Acids Res. 31:5817-5830; Trimble, et al. (2003) Vaccine 21:4036-4042; Nishitani, et al. (2000) Mol. Urol. 4:47-50; Tuting (1999) Curr. Opin. Mol. Ther. 1:216-225). Nucleic acid vaccines can be used in combination with reagents that promote migration of immature dendritic cells towards the vaccine, and a reagent that promotes migration of mature DCs to the draining lymph node where priming can occur, where these reagents encompass MIP 1alpha and Flt3L (see, e.g., Kutzler and Weiner (2004) J. Clin. Invest. 114:1241-1244; Sumida, et al. (2004) J. Clin. Invest. 114:1334-1342).

Bacterial vectors include, for example, *Salmonella*, *Shigella*, *Yersinia*, *Lactobacillus, Streptococcus, Bacille Calmette Guerin*, *Bacillus anthracis*, and *Escherichia coli.* The bacterium can be engineered to contain a nucleic acid encoding a recombinant antigen, a heterologous antigen, or an antigen derived from a tumor, cancer cell, or infective agent. Moreover, the bacterium can modified to be attenuated. In another aspect, the non listerial bacterial vaccine can be absent of any nucleic acid encoding a recombinant antigen (see, e.g., Xu, et al. (2003) Vaccine 21:644-648; Pasetti, et al. (2003) J. Virol. 77:5209-5219; Loessner and Weiss (2004) Expert Opin. Biol. Ther. 4:157-168; Grangette, et al. (2002) Vaccine 20:3304-3309; Byrd, et al. (2002) Vaccine 20:2197-2205; Edelman, et al. (1999) Vaccine 17:904-914; Domenech, et al. (2005) Microbes and Infection 7:860-866).

An effective amount of a priming vector or boosting vector to be supplied in one or multiple doses of a vaccine can be determined by one of skill in the art. Such an amount will fall in a range that can be determined through routine trials.

The prime vaccine and the boost vaccine can be administered by any one or combination of the following routes. In one aspect, the prime vaccine and boost vaccine are administered by the same route. In another aspect, the prime vaccine and boost vaccine are administered by different routes. The term "different routes" encompasses, but is not limited to, different sites on the body, for example, a site that is oral, non oral, enteral, parenteral, rectal, intranode (lymph node), intravenous, arterial, subcutaneous, intramuscular, intratumor, peritumor, intratumor, infusion, mucosal, nasal, in the cerebrospinal space or cerebrospinal fluid, and so on, as well as by different modes, for example, oral, intravenous, and intramuscular.

An effective amount of a prime or boost vaccine may be given in one dose, but is not restricted to one dose. Thus, the administration can be two, three, four, five, six, seven, eight, nine, ten, eleven, twelve, thirteen, fourteen, fifteen, sixteen, seventeen, eighteen, nineteen, twenty, or more, administrations of the vaccine. Where there is more than one administration of a vaccine the administrations can be spaced by time intervals of one minute, two minutes, three, four, five, six, seven, eight, nine, ten, or more minutes, by intervals of about one hour, two hours, three, four, five, six, seven, eight, nine, ten, 11, 12, 13, 14, 15, 16, 17, 18, 19, 20, 21, 22, 23, 24 hours, and so on. In the context of hours, the term "about" means plus or minus any time interval within 30 minutes. The administrations can also be spaced by time intervals of one day, two days, three days, four days, five days, six days, seven days, eight days, nine days, ten days, 11 days, 12 days, 13 days, 14 days, 15 days, 16 days, 17 days, 18 days, 19 days, 20 days, 21 days, and combinations thereof. The invention is not limited to dosing intervals that are spaced equally in time, but encompass doses at non equal intervals, such as a priming schedule consisting of administration at 1 day, 4 days, 7 days, and 25 days, just to provide a non limiting example.

The following may be taken into consideration in determining the relative timing of the prime vaccine and boost vaccine. It has been found that administration of an antigen, or nucleic acid encoding an antigen, can stimulate expansion of antigen specific immune cells, resulting in a peak, followed by contraction of the number of antigen specific immune cells (see, e.g., Badovinac, et al. (2002) Nature Immunol. 3:619-626). Initiation of the boost vaccination can be administered before the peak is reached, coincident with the peak, or after the peak.

Administration of the boost vaccination can be initiated when a population of antigen specific immune cells has expanded (increased in number) to at least 20% the maximal number of antigen specific immune cells that is eventually attained; to at least 30%; to at least 40%; to at least 50%; to at least 60%; to at least 70%; to at least 80%; to at least 90%; to at least 95%; to at least 99% the maximal number of antigen specific immune cells that is eventually attained. Additional schedules of prime boost vaccines are available, for example, the boost vaccination can be initiated when the population of antigen specific cells has contracted to under 90% the maximal number of antigen specific cells; under 80%; under 70%; under 60%; under 50%; under 40%; under 30%; under 20%; under 10%; under 5%; under 1.0%; under 0.5%; under 0.1%; under 0.05%; or under 0.01% the maximal number of antigen specific immune cells. The antigen specific cells can be identified as specific for a vector specific antigen (specific for empty vector), or specific for a heterologous antigen expressed by a nucleic acid contained in the vector.

In other aspects, administration of the boost vaccination can be initiated at about 5 days after the prime vaccination is initiated; about 10 days after the prime vaccination is initiated; about 15 days; about 20 days; about 25 days; about 30 days; about 35 days; about 40 days; about 45 days; about 50 days; about 55 days; about 60 days; about 65 days; about 70 days; about 75 days; about 80 days, about 6 months, and about 1 year after administration of the prime vaccination is initiated.

The boost vaccination can be administered 5-10 days after the prime vaccination; 10-15 days after the prime vaccination; 15-20 days after the prime vaccination; 20-25 days after the prime vaccination; 25-30 days after the prime vaccination; 30-40 days after the prime vaccination; 40-50 days after the prime vaccination; 50-60 days after the prime vaccination; 60-70 days after the prime vaccination; and so on.

The period of time between initiation of the prime vaccination and initiating the boost vaccination can be determined by one of skill in the art. For example, it can be based on an algorithm that is sensitive to physiologic parameters measured after the prime immunization has occurred.

The dosage and regimen will be determined, at least in part, be determined by the potency of the modality, the vaccine delivery employed, the need of the subject and be dependent on the judgment of the practitioner.

For example, the PrfA* *Listeria* in the vaccines used in the invention can be administered in a dose, or dosages, where each dose comprises between 10⁷ and 10⁸ *Listeria* per 70 kg body weight; 2 x 10⁷ and 2 x 10⁸ *Listeria* per 70 kg body weight; 5 x 10⁷ and 5 x 10⁸ *Listeria* per 70 kg body weight; 10⁸ and 10⁹ *Listeria* per 70 kg body weight; between 2.0 x 10⁸ and 2.0 x 10⁹ *Listeria* per 70 kg; between 5.0 x 10⁸ to 5.0 x 10⁹ *Listeria* per 70 kg; between 10⁹ and 10¹⁰ *Listeria* per 70 kg; between 2 x 10⁹ and 2 x 10¹⁰ *Listeria* per 70 kg; between 5 x 10⁹ and 5 x 10¹⁰ *Listeria* per 70 kg; between 10¹¹ and 10¹² *Listeria* per 70 kg; between 2 x 10¹¹ and 2 x 10¹² *Listeria* per 70 kg; between 5 x 10¹¹ and 5 x 10¹² *Listeria* per 70 kg; between 10¹² and 10¹³ *Listeria* per 70 kg; between 2 x 10¹² and 2 x 10¹³ *Listeria* per 70 kg; between 5 x10¹² and 5 x 10¹³ *Listeria* per 70 kg; and so on, wet weight. Also provided are each of the above doses, based in a per 1.7 square meters surface area basis, or on a 1.5 kg liver weight basis. It is to be noted that a mouse liver, at the time of administering the *Listeria* of the invention, weighs about 1.5 grams. Human liver weighs about 1.5 kilograms.

In some embodiments of the invention the boost dose of PrfA* *Listeria* will enhance the prime dose immune response by at least two-fold, at times between about three- and five-fold or five-fold to ten-fold, or from ten-fold to 100-fold or greater. In some embodiments of the invention the prime dose and boost dose will have a synergistic effect on the immune response. In some embodiments of the invention the enhanced immune response will include a T-cell response, and in some embodiments the T-cell response will be a CD8+ T-cell response. In some embodiments of the invention the prime dose and boost dose will break the mammal's tolerogenic state towards the target antigen. Examples of all of these embodiments are provided below.

Cancers and infections can be treated and/or inhibited by administering reagents that modulate the immune system. The prime-boost methods encompassed within the invention give rise to immune responses that are upregulated, and include breaking tolerance to self-antigens. Thus, it is expected that these prime-boost methods will be useful in inhibiting the growth of cancers and/or ameliorating one or more symptoms associated with a cancer. It is also expected that the prime-boost methods will be useful in the prophylaxis and/or treatment of a disease caused by a pathogenic agent.

In addition to the above, these regimens can be used to determine whether a mammal will be responsive to a treatment. For example, when a prime-boost regimen towards a specific antigen is used, failure to obtain a significant immune response after the boost suggests that the mammal is non-responsive towards the target antigen and an alternative mode of treatment should be pursued. Examples of this could be when the genetic background of the cancer or pathogenic agent is such that the target antigen is absent or modified in a way that it is not cross-reactive with the target antigen.

In some embodiments, the therapeutic treatment of an individual for cancer may be started on an individual who has been diagnosed with a cancer as an initial treatment, or may be used in combination with other treatments. For example, individuals who have had tumors surgically removed or who have been treated with radiation therapy or by chemotherapy may be treated with the vaccine in order to reduce or eliminate any residual tumors in the individual, or to reduce the risk of a recurrence of the cancer. In some embodiments, the prophylactic treatment of an individual for cancer, would be started on an individual who has an increased risk of contracting certain cancers, either due to environmental conditions or genetic predisposition.

The dosage of the pharmaceutical compositions or vaccines that are given to the host will vary depending on the species of the host, the size of the host, and the condition or disease of the host. The dosage of the compositions will also depend on the frequency of administration of the compositions and the route of administration. The exact dosage is chosen by the individual physician in view of the patient to be treated.

In some embodiments, a single dose of the pharmaceutical compositions, immunogenic compositions, or vaccines comprising the *Listeria* described herein comprises from about 10² to about 10¹² of the bacterial organisms. In another embodiment, a single dose comprises from about 10⁵ to about 10¹¹ of the bacterial organisms. In another embodiment, a single dose comprises from about 10⁶ to about 10¹¹ of the bacterial organisms. In still another embodiment, a single dose of the pharmaceutical composition or vaccine comprises from about 10⁷ to about 10¹⁰ of the bacterial organisms. In still another embodiment, a single dose of the pharmaceutical composition or vaccine comprises from about 10⁷ to about 10⁹ of the bacterial organisms.

The *Listeria* of the present invention, in some embodiments, is administered in a dose, or dosages, where each dose comprises at least about 1000 *Listeria* units/kg body weight, at least about 10,000 *Listeria* units/kg body weight, at least about 100,000 *Listeria* units/kg body weight, at least about 1 million *Listeria* units/kg body weight, or at least about 10 million. *Listeria* units/kg body weight. The present invention provides the above doses where the units of *Listeria* are colony forming units (CFU), the equivalent of CFU prior to psoralen-treatment, or where the units are number of *Listeria* cells. In some embodiments, the effective amount of attenuated *Listeria* that is measured comprises at least about 1 x 10³ CFU/kg or at least about 1 x 10³ *Listeria* cells/kg. In some embodiments, the effective amount of attenuated *Listeria* that is measured comprises at least about 1 x 10⁵ CFU/kg or at least about 1 x 10⁵ *Listeria* cells/kg. In certain embodiments, the effective amount of attenuated *Listeria* that is measured comprises at least about 1 x 10⁶ CFU/kg or at least about 1 x 10⁶ *Listeria* cells/kg. In some embodiments, the effective amount of attenuated *Listeria* that is measured comprises at least about 1 x 10⁷ CFU/kg or at least about 1 x 10⁷ *Listeria* cells/kg. In some further embodiments, the effective amount of attenuated *Listeria* that is measured comprises at least about 1 x 10⁸ CFU/kg or at least about 1 x 10⁸ *Listeria* cells/kg.

In some embodiments, a single dose of the pharmaceutical composition, immunogenic composition, or vaccine comprising the *Listeria* described herein comprises from about 1 CFU/kg to about 1 x 10¹⁰ CFU/kg (CFU = colony forming units). In some embodiments, a single dose of the composition comprises from about 10 CFU/kg to about 1 x 10⁹ CFU/kg. In another embodiment, a single dose of the composition or vaccine comprises from about 1 x 10² CFU/kg to about 1 x 10⁸ CFU/kg. In still another embodiment, a single dose of the composition or vaccine comprises from about 1 x 10³ CFU/kg to about 1 x 10⁸ CFU/kg. In still another embodiment, a single dose of the composition or vaccine comprises from about 1 x 10⁴ CFU/kg to about 1 x 10⁷ CFU/kg. In some embodiments, a single dose of the composition comprises at least about 1 CFU/kg. In some embodiments, a single dose of the composition comprises at least about 10 CFU/kg. In another embodiment, a single dose of the composition or vaccine comprises at least about 1 x 10² CFU/kg. In still another embodiment, a single dose of the composition or vaccine comprises at least about 1 x 10³ CFU/kg. In still another embodiment, a single dose of the composition or vaccine comprises from at least about 1 x 10⁴ CFU/kg.

In some embodiments, the proper (i.e., effective) dosage amount for one host, such as human, may be extrapolated from the LD₅₀ data for another host, such as a mouse, using methods known to those in the art.

In some embodiments, the pharmaceutical composition, immunogenic composition, or vaccine for use in the invention may be administered without subsequent administration with antibiotics. In cases where a live *Listeria* is administered to a host, it may be necessary to administer antibiotics to the host to limit Listerial replication following vaccination. In cases where the *Listeria* are attenuated for growth in the host; however, it may not be necessary to administer an antibiotic to control the growth of the *Listeria* in the host. In some aspects of the present invention, the *Listeria* is attenuated for growth in the host. For example, in some aspects of the invention, the *Listeria* is killed, but metabolically active. In these cases, it may not be necessary to administer an antibiotic to control the growth of the *Listeria* in the host.

In some embodiments, the pharmaceutical composition, immunogenic composition, or vaccine comprises antigen-presenting cells, such as dendritic cells, which have been infected with the *Listeria* described herein. In some embodiments, an individual dosage of an antigen-presenting cell based vaccine comprising bacteria such as those described herein comprises between about 1x10³ to about 1x10¹⁰ antigen-presenting cells. In some embodiments, an individual dosage of the vaccine comprises between about 1x10⁵ to about 1x10⁹ antigen-presenting cells. In some embodiments, an individual dosage of the vaccine comprises between about 1x10⁷ to about 1x10⁹ antigen-presenting cells.

In some embodiments, multiple administrations of the dosage unit are preferred, either in a single day or over the course of a week or month or year or years. In some embodiments, the dosage unit is administered every day for multiple days, or once a week for multiple weeks. In some embodiments, the *Listeria* are administered to the mammalian subject at least twice, at least three times, at least four times, at least five times, at least 10 times, or at least 20 times.

The invention also provides a method of inducing MHC class I antigen presentation or MHC class II antigen presentation on an antigen-presenting cell comprising contacting a bacterium described herein with an antigen-presenting cell.

Also described herein is a method of inducing an immune response in a host to an antigen comprising, the following steps: (a) contacting a *Listeria* bacterium described herein with an antigen-presenting cell from the host, under suitable conditions and for a time sufficient to load the antigen-presenting cells; and (b) administering the antigen-presenting cell to the host.

### VIII. Kits

The present disclosure further provides kits and articles of manufacture comprising the *Listeria* described herein, or compositions comprising the *Listeria* described herein.

### EXAMPLES

The following examples are provided to illustrate, but not to limit, the invention.

### EXAMPLE 1

In this study, the impact of a three PrfA* mutant polypeptides, including G145S, G155S and Y63C, on the potency of isogenic live-attenuated and KBMA vaccine strains was assessed.

### MATERIALS AND METHODS

Mice. 6-12 week old female C57BL/6 and Balb/c mice were obtained from Charles River Laboratories (Wilmington, MA). Studies were performed under animal protocols approved by the Anza Institutional Animal Care and Use Committee.

Bacterial Strains. *Listeria monocytogenes* vaccine strains were constructed in the *Listeria monocytogenes* Δ*actA*/Δ*inlB*/Δ*uvrAB* strain (9). PrfA* variants were constructed by cloning the various *prfA* alleles with 800 bp to 1 kb of flanking homology into the temperature-sensitive allelic exchange vector pKSV7 and used to replace the wild-type allele using standard procedures (12). Strains with activated *prfA* phenotypes were screened for increased zones of both phospholipase activity on egg-yolk overlay plates (50) and hemolysis on horse-blood agar (Remel). Phenotypically correct clones were confirmed by sequencing the genomic *prfA* locus. An antigen expression cassette termed "Quadvac" construct, expressing four Vaccinia CD8+ T cell epitopes of varying strength and the ovalbumin (OVA)-derived CD8+ T cell epitope SIINFEKL from a single synthetic gene, was designed in silico where the epitopes were strung together and spaced with a linker sequence. The amino acid sequence was codon-optimized for expression in *Listeria monocytogenes* using Gene Designer software (48), synthesized (DNA2.0, Menlo Park, CA), and cloned downstream of the *actA* promoter and in-frame with the amino terminus of the *actA* gene. The construct was cloned into a derivative of the pPL2 integration vector and stably integrated at the *tRNA^{Arg}* locus of the bacterial chromosome in the various *prfA** strain backgrounds as described previously (23). All molecular constructs were confirmed by DNA sequencing.

Western blot detection of heterologous antigen expression. Western blots from broth culture were performed on equivalent amounts of TCA-precipitated supernatants of bacterial cultures grown in yeast extract media to an OD₆₀₀ of 0.7 (late log). For western blots from *Listeria monocytogenes* infected host cells, J774 cells or DC2.4 cells were inoculated with an multiplicity of infection (MOI) of 50 or 100 for 1 hour, the cells were washed 3x with PBS and DMEM media supplemented with 50 µg/mL gentamycin. For early timepoints, DC2.4s were harvested at 1.5 or 2.5 hr post infection. For late time points, J774 cells were harvested at 7 hours. Cells were lysed with SDS sample buffer, collected and run on 4-12% polyacrylamide gels and transferred to nitrocellulose membranes for western blot analysis. All western blots utilized a polyclonal antibody raised against the mature N-terminus of the ActA protein.

Immunizations. Live-attenuated bacteria were prepared for immunization from overnight cultures grown in yeast extract media. KBMA *Listeria monocytogenes* strains were S59-psoralen and UVA treated as previously described (9). Photochemically inactivated bacteria (KBMA Lm) were washed once with DPBS, resuspended in 8% DMSO, and then stored at -80° C. Bacteria were diluted in Hank's balanced salt solution (HBSS) for injection. Injection stocks of live-attenuated bacteria were plated to confirm colony forming units (CFU). 5×10⁶ CFU live-attenuated bacteria and 1×10⁸ particles of KBMA bacteria were administered either i.v. into tail vein in 200 µL volume or intramuscularly (i.m.) into a single tibialis cranialis muscle in 30 µL volume, boost vaccination given in alternate tibialis cranialis muscle.

Detection of serum cytokines and chemokines. Serum was collected from mice by retro-orbital bleed 2, 4, and 8 hrs post-vaccination with KBMA or 4, 8, and 24 hrs with live-attenuated Lm. Cytokines/chemokines were measured using the mouse inflammation Cytometric Bead Array kit (BD Biosciences, San Jose, CA) according to the manufacturer's instructions. Samples were acquired using a FACSCan to flow cytometer (BD Biosciences). Data were analyzed using Cytometric Bead Array software (BD Biosciences).

Peptides. OVA-57-264 (SIINFEKL), Loo190-201 (NEKYAQAYPNVS), HSV-gB2 (SSIEFARL), B8R₂₀₋₂₇ (TSYKFESV), K3L₆₋₁₅ (YSLPNAGDVI), C4L₁₂₅₋₁₃₂ (LNFRFENV), A42R₈₈₋₉₆ (YAPVSPIVI) (27) peptides were synthesized by Synthetic Biomolecules (San Diego, CA).

Reagents for flow cytometry. CD3 FITC or PE-Cy7 (clone 145-2C11), CD4 FITC (clone GK1.5), CD8 PE-Cy7 or APC-Cy7 (clone 53-6.7), CD19 FITC (clone MB 19-1), TNF PE (clone MP6-XT22), IFN-γ APC (clone XMG 1.2) were purchased from eBioscience (San Diego, CA). CD8α PerCP (clone 53-6.7) was purchased from BD Biosciences (San Jose, CA).

In vivo cytotoxicity assay. Splenocytes from naïve recipients were pulsed with a 1 µM concentration of either control (HSV-gB2) or target (B8R or A42R) peptide. Cells were then labeled with 0.2 µM (CFSE^{lo}), 1µM (CFSE^{med}) or 5 µM (CFSE^{hi}) concentrations of Carboxyfluorescein Diacetate Succinimidyl Ester (CFSE, Molecular Probes, Eugene, OR). 3×10⁶ labeled spleen cells of each population were mixed and injected i.v. Spleens were harvested 16 hours later and the proportion of target to control population determined and percentage killing calculated.

Intracellular Staining of Antigen-Specific T Cells. Splenocytes were stimulated for 5 hours with the relevant peptide in the presence of brefeldin A for intracellular cytokine staining as previously described (Brockstedt, D.G et al. (2004) Proc. Natl. Acad. Sci. USA 101:13832-13837). Stimulated cells were surface stained for CD4 and CD8, then fixed and permeabilized using the cytofix/cytoperm kit (BD Biosciences, San Jose, CA). Cells were then stained for IFN-γ, TNF-α and/or IL-2. Samples were acquired using a FACSCanto flow cytometer (BD Biosciences). Data were gated to include exclusively CD4+ or CD8+ events, then the percentage of these cells expressing IFN- y determined. Data was analyzed using FlowJo software (Treestar, Ashland, OR).

*Listeria monocytogenes* protection studies. To assess protective immunity, Balb/c mice were vaccinated with the indicated strains, and challenged 14 days post vaccination with 2x LD₅₀ of wild-type *Listeria monocytogenes* (1×10⁵ colony forming units; CFU), and CFU in spleen was measured three days later in organ homogenates as described previously (Bahjat, K.S. et al. (2005) J. Immunol. 179:7376-7384). Median lethality (LD₅₀) values were determined as described previously (10).

Vaccinia virus protection studies. C57BL/6 mice were given prime and boost vaccinations separated by 27 days with *Listeria monocytogenes* Quadvac strains, and 28 days later mice were challenged intraperitoneally with 1×10⁷ plaque forming units (PFU) of vaccinia virus. Protection was evaluated by measuring viral titer in the ovaries five days after virus challenge as described (1).

Statistical Analysis. Differences in protection against vaccinia virus or *Listeria monocytogenes* challenges were determined by the Student's t-test. Unless otherwise indicated, all experiments were conducted at least twice. Unless otherwise indicated, all experiments were conducted at least twice.

### RESULTS

**Construction and characterization of isogenic *PrfA*Listeria monocytogenes* vaccine strains.** We hypothesized that induction of the PrfA regulon prior to immunization would increase the immunologic potency of recombinant live-attenuated and KBMA *Listeria monocytogenes* vaccines. To test this hypothesis, we constructed a panel of isogenic strains on a *Listeria monocytogenes* background that contained complete coding region deletions of *actA, inlB, uvrA,* and *uvrB* (*Listeria monocytogenes* Δ*actA*/Δ*inlB*/Δ*uvrAB*) that only varied in *prfA.* We selected three *prfA* mutants that were generated by chemical mutagenesis or were spontaneous mutants, and encoded a constitutively active PrfA* protein (37, 41). Strains with PrfA* G155S, G145S, or Y63C have been shown previously to either increase the expression of an *actA* promoter dependent β-glucouronidase reporter protein relative to isogenic strains with the native *prfA,* and in some cases increase the virulence of wild-type (WT) *Listeria monocytogenes* (26, 28, 37, 41).

To enable us to distinguish immunologic potency differences between isogenic *Listeria monocytogenes* vaccine strains, we constructed an Ag expression cassette that encoded five well-defined H-2^{b}-restricted MHC class I epitopes that have been shown previously to elicit a range of CD8+ T cell responses in mice. A construct encoding four tandemly spaced vaccinia virus (A42R, C4L, K3L, B8R) epitopes and the chicken ovalbumin (SL8) epitope was synthesized and then cloned under control of the PrfA-regulated *actA* promoter as a fusion protein with the 100 N-terminal amino acids of ActA. The construct known as "Quadvac" was cloned into a derivative of pPL2 and then integrated at the *tRNA^{Arg}* locus in the four isogenic strains (*Listeria monocytogenes* Δ*actA*/Δ*inlB*/Δ*uvrAB*) harboring the WT, G145S, G155S, or Y63C *prfA* alleles, as described previously (Figure 1A) (23). The four isogenic vaccine strains all grew equivalently in yeast extract or brain heart infusion broth culture (data not shown). Vaccine strains grown in yeast extract broth culture were used either directly as a live-attenuated *Listeria monocytogenes* vaccine (10), or photochemically inactivated with the synthetic psoralen S-59 and long-wave UV light, to yield a KBMA *Listeria monocytogenes* vaccine, as described previously (9).

We compared the level of Ag expression and secretion in broth culture from the four live attenuated *Listeria monocytogenes* vaccines, and as expected, higher expression levels were observed in PrfA* strains compared to the strain with a WT *prfA* allele (Figure 1B). While over-expression of PrfA-dependent genes in PrfA* strains grown in broth culture combined with enhanced invasion of epithelial cells has been well described (28, 34, 41, 47), little is known whether the PrfA* phenotype is recapitulated in infected cultured mammalian cells. We evaluated Ag expression from the *Listeria monocytogenes* vaccine strains in phagocytic mouse macrophage or dendritic cell (DC) lines, J774 and DC2.4, respectively, rather than non-phagocytic cell lines such as HepG2 (liver) or PtK2 (epithelial). The *Listeria monocytogenes* Δ*actAl*Δ*inlB* strain cannot mediate InlB-dependent infection of liver cells expressing the hepatocyte growth factor receptor, and epithelial tissues do not represent a major target that is relevant to the intramuscular and intravenous immunization routes use in this investigation. Surprisingly, Ag expression levels in J774 macrophages infected with the isogenic *Listeria monocytogenes* vaccine strains were equivalent regardless of *prfA* allele (Figure 1C). Notably, Ag expression levels were also equivalent at early time points in DC2.4 DCs (Figure ID). Infection (uptake) and intracellular growth of all *Listeria monocytogenes* vaccine strains in J774 (Figure 1E) and DC2.4 (not shown) cell lines was equivalent and not dependent on *prfA* allele.

**PrfA* minimally increases the virulence of *Listeria monocytogenes* Δ*actA*/Δ*inlB*/Δ*uvrAB* strains**. It has been reported previously that PrfA* mutant *Listeria* have up to a 10-fold increased virulence in Balb/c mice. For example, *prfA* G155S decreased the LD₅₀ value of its isogenic wild-type strain from 2×10⁴ cfu to 3×10³ cfu (41). However, the impact of PrfA* mutants on the virulence of attenuated strains is unknown. As the immunization dose used for *Listeria monocytogenes* vaccine studies in mice is typically one-tenth the LD₅₀ value, we measured the impact the three PrfA* mutants used in this investigation had on *Listeria monocytogenes* Δ*actA*/Δ*inlB*/Δ*uvrAB* virulence, a strain that is rapidly cleared from the liver following IV administration, and is attenuated by more than 3 logs in mice compared to WT *Listeria monocytogenes* (6, 9, 10). PrfA* marginally increased the virulence of *Listeria monocytogenes* Δ*actA*/Δ*inlB*/Δ*uvrAB* virulence, with the LD₅₀ value decreased only 2.1-fold (3.5 × 10⁷ cfu vs. 7.3×10⁷ cfu) in isogenic strains harboring the *prfA* G145S or *prfA* Y63C alleles, and 1.4 fold (5.2 × 10⁷ cfu vs. 7.3 × 10⁷ cfu) in the isogenic strain harboring the *prfA* G155S allele (Table 5).

Lm-based vaccines, including attenuated *Listeria monocytogenes* Δ*actAl*Δ*inlB* based strains are potent activators of innate immunity, as reflected by the Th1 polarizing pro-inflammatory serum cytokine profile induced in response to IV administration (4). As activation of innate immunity is related to the quality of the *Listeria monocytogenes* vaccine-induced immune response, we measured the serum cytokines levels at several time points during the first 24 hours following IV administration of the isogenic vaccine strains. C57BL/6 mice were injected intravenously with 5×10⁶ cfu, a dose that approximated the 0.1 LD₅₀ value for the four isogenic vaccine strains. All three PrfA* vaccine strains induced statistically significant higher levels of proinflammatory cytokines/chemokines within eight hours of administration compared to the vaccine strain with native *prfA* (Figure 2A). No significant differences between the three PrfA* strains were observed; however, increased mouse-to-mouse variability was observed in mice given the PrfA* Y63C vaccine strain.

**Table 5. Virulence Of Live-Attenuated Listeria monocytogenes Strains**

| Strain ID | Genotype | prfA | LD₅₀ (cfu) | Fold change to wt |
|---|---|---|---|---|
| BH1299 | Lm ΔactAΔinlBΔuvrAB | wt | 7.3×10⁷ | - |
| BH1371 | Lm Δ*actA* Δ*inlB* Δ*uvrAB* prfAG155S | G155S | 5.2×10⁷ | 1.4 |
| BH1375 | Lm Δ*actA* Δ*inlB* Δ*uvrAB* prfAG145S | G145S | 3.5×10⁷ | 2.1 |
| BH1379 | Lm Δ*actA* Δ*inlB* Δ*uvrAB* prfAY63C | Y63C | 3.5×10⁷ | 2.1 |

**PrfA* increases the immunogenicity of live-attenuated *Listeria monocytogenes* vaccines**. We evaluated the immunogenicity of the isogenic vaccine strains to assess the impact of PrfA* on vaccine potency. To facilitate comparison, isogenic vaccine strains expressed a common heterologous Ag (termed "Quadvac") comprised of multiple defined H-2^{b}-restricted MHC class I vaccinia virus epitopes (A42R, C4L, K3L, B8R) that have been shown to elicit high, intermediate and low frequency T cell responses following vaccinia virus infection, and in addition, the strong OVA epitope, SL8. This strategy allowed us both to rank the magnitude of vaccine-induced CD8+ T cell responses over a dose range of immunization, and to evaluate the quality of the response by challenge with vaccinia virus.

Groups of female C57BL/6 mice were immunized IV with 5×10⁶ cfu of the four isogenic *Listeria monocytogenes* Quadvac strains, and the CD8+ and (Lm-specific) CD4+ T cell frequency was determined by intracellular cytokine staining at the peak of the response (10), seven days following a single immunization. The PrfA* G155S *Listeria monocytogenes* vaccine induced antigen-specific T cell responses of greater magnitude compared to the G145S and Y63C PrfA* vaccine strains and to the strain with WT *prfA* (Figure 2B and 2C). The increased magnitude of the antigen-specific IFN-γ+ T cells in PrfA* G155S *Listeria monocytogenes* vaccine immunized mice was greater not only with the immunodominant SL8 and B8R epitopes, but significantly also with intermediate and low epitopes, A42R, C4L and K3L. The LLO-specific CD4+ T cell response was increased two-fold in mice immunized with the PrfA*G155S vaccine strain compared to the other vaccine strains.

**PrfA* G155S increases the immunogenicity of KBMA *Listeria monocytogenes* vaccines.** We hypothesized that constitutive activation of PrfA and induction of the PrfA regulon might improve the immunogenicity of KBMA *Listeria monocytogenes* through a variety of mechanisms, including increased escape from the vacuole, as well as an increased expression level of the heterologous antigen in the cytosol of antigen presenting cells. We demonstrated previously that CD8+ T cell potency and protective immunity requires that the immunizing *Listeria monocytogenes* strain accesses the cytosol (5). As KBMA vaccine strains are unable to expand in the cytosol, increased efficiency of escape from the phagosome through increased expression of LLO and phospholipases C might enhance vaccine potency.

To assess the innate as well as adaptive immunity to KBMA *Listeria monocytogenes* strains, C57BL/6 mice were immunized IV with 1×10⁸ particles, a well-tolerated dose. As described previously, we used photochemical inactivation conditions that resulted in 10-log killing of *Listeria monocytogenes* vaccine preparations (9). Thus, individual mice had a 10⁻² chance of receiving a single attenuated *Listeria monocytogenes ΔactA*/*ΔinlB*/Δ *uvrAB* bacterium, a non-immunizing dose. Serum cytokine/chemokine levels were measured during the first 8 hours of infection, which we had observed in previous experiments to include the peak of the response, which then returned to background levels within 24 hours (6). KBMA PrfA* vaccine strains induced higher levels of MCP-1, IL-12p70 and IFN-γ than the levels induced by the KBMA vaccine with WT *prfA* (Figure 3A). No significant differences between the three PrfA* strains were observed, but the levels of cytokines induced by the KBMA PrfA* G155S vaccine tended to be higher than the levels induced by the KBMA PrfA* G145S or Y63C vaccines.

We compared the immunogenicity of the isogenic KBMA vaccine strains in C57BL/6 mice, given two vaccinations separated by two weeks. The highest magnitude of the secondary antigen-specific CD8+ T cell response specific for the five Quadvac epitopes was observed in KBMA PrfA* G155S immunized mice (Figures 3B and C). While the magnitude of the CD8+ response was generally higher in mice immunized with the other two KBMA PrfA* vaccine strains as compared to the KBMA WT *prfA,* this was not the case with all CD8 T cell epitopes evaluated (Figures 3B and C). Interestingly, in contrast to the live-attenuated vaccine strains, LLO-specific CD4+ T cell responses were not higher in magnitude among mice immunized with KBMA *Listeria monocytogenes* PrfA* strains. Thus, the *prfA* G155S allele conferred the highest immunogenicity to both live-attenuated and KBMA vaccine strains.

**KBMA PrfA*G155S vaccines have increased immune potency**. It is well established that the magnitude of an induced T cell response is not necessarily representative of the potency of the response. To assess the potency of the vaccine-induced CD8+ T cell response, we assessed the in vivo cytolytic activity specific for two vaccinia epitopes, A42R and B8R. C57BL/6 mice were immunized twice with the four isogenic KBMA *Listeria monocytogenes* strains. We evaluated immunogenicity following two alternative immunization routes: intravenous (IV) and intramuscular (IM). We evaluated IM immunization to assess the potency of KBMA PrfA* vaccine strains when administered by a conventional vaccination route. Robust responses measured by in vivo cytotoxicity specific for the strong B8R epitope were observed in all groups immunized with KBMA PrfA* or WT *prfA* vaccines. The extent of target killing was slightly higher in mice that were immunized IV (Figure 4A and 4B). Potent killing activity was also elicited against the A42R vaccinia virus epitope in mice immunized IV or IM with KBMA PrfA* G155S. However, the killing activity against A42R induced by KBMA vaccines based on wild-type PfrA* and given IM was reduced by 2-fold compared to PrfA* G155S. KBMA vaccines based on G145S or Y63C were of intermediate potency when given by the IM route (Figure 4B). In a dose-response experiment, the superior potency of KBMA PrfA* G155S for inducing B8R responses could be seen compared to mice immunized with KBMA harboring WT *prfA* (Figure 4C).

A relevant measure of vaccine-induced T cell potency is protective immunity against challenge with a live pathogen. We evaluated the T cell potency in mice immunized with KBMA PrfA* G155S or WT *prfA* by challenge with WT *Listeria monocytogenes* or vaccinia virus. Strains of *Listeria monocytogenes* that fail to escape from the phagolysosome fail to induce protective immunity, although antigen-specific T cell responses that can be expanded upon secondary challenge are elicited (5, 20). We previously described that KBMA Lm-based vaccination results in the transient protection against a lethal wild-type *Listeria monocytogenes* challenge. The induced T cell response wanes over time, reminiscent of a T cell response induced in the absence of CD4+ T cell help (3, 45). Immunization of mice with KBMA *Listeria monocytogenes* Δ*actA*/Δ*inlB*/Δ*uvrAB* resulted in a 2-log protection at 14 days. To evaluate the potency of the KBMA PrfA*G155S vaccine-induced adaptive response, mice were immunized once with 1×10⁸ particles and challenged with a 2x LD₅₀ with wild-type *Listeria monocytogenes* 14 days later. Colony-forming units (cfu) were determined in spleen and liver (data not shown) three days later. As shown in Figure 4D, protection from wild-type *Listeria monocytogenes* was improved by 3-logs in mice immunized with KBMA PrfA*G155S compared to KBMA with WT *prfA.*

We then determined whether the enhanced immunologic potency of KBMA PrfA* G155S also extended to vaccinia virus challenge. C57BL/6 mice were immunized twice by an IM route two weeks apart with KBMA PrfA*G155S or WT *prfA* vaccines or with a KBMA control harboring WT *prfA* and not encoding the Quadvac Ags. To evaluate protective memory immunity, mice were challenged with 1×10⁷ pfu of vaccinia virus 30 days following the last immunization and viral titers were determined from the ovaries of the mice 5 days later. Consistent with the improved magnitude of the induced T cell response to the various vaccinia virus epitopes, we observed a statistically significant improved protection by 2-logs against viral challenge in mice that received the PrfA*G155S mutant *Listeria* on the background of the KBMA *Listeria monocytogenes* Δ*actA*/Δ*inlB* strain.

These results demonstrate that PrfA* G155S confers increased immunologic potency to live-attenuated and KBMA *Listeria monocytogenes* vaccines, and, notably providing the ability of KBMA vaccines to elicit protective immunity in rigorous infectious disease challenge models following a conventional immunization route.

### DISCUSSION

Vaccine platforms based on live-attenuated *Listeria monocytogenes* are being developed and evaluated clinically due to an inherent property of stimulating potent innate immunity and acquired cellular immunity (clinicaltrials.gov identifier NCT00327652 and NCT00585845). In the experiments of Example 1, we show that activating the PrfA regulon prior to vaccinating mice significantly enhanced the level of live-attenuated and KBMA *Listeria monocytogenes* vaccine-induced innate and cellular immunity that was correlated with improved protection against challenge with the cognate wild-type bacterial pathogen or vaccinia virus. These results form the basis of a rationale to include the *prfA* G155S allele in future Lm-based vaccines advanced to the clinics.

The immune potency for both live-attenuated and KBMA vaccines was significantly enhanced by activation of the *prfA* regulon prior to immunization. PrfA* enhancement of immune potency was more apparent with KBMA vaccines. We have shown previously that although killed, KBMA vaccines still escape the phagolysosome, a necessary step towards inducing IFN-β and other activating signals in antigen presenting cells required to elicit protective cellular immunity against challenge with wild-type *L. monocytogenes* (5, 9, 30). However, KBMA *Listeria monocytogenes* vaccines can elicit protective immunity after a single immunization only when administered in combination with surrogate help provided by α-CD40 Ab, or when using a homologous prime and boost immunization regimen (5). These results demonstrate a reduced immune potency for KBMA vaccines compared to live-attenuated *Listeria monocytogenes* Δ*actAl*Δ*inlB* vaccine strains, which like wild-type *L. monocytogenes*, can elicit protective immunity after a single immunization. Live *Listeria monocytogenes* strains expanded 100-fold over 7 hours in the cytoplasm of infected macrophages in vitro (Figure ID), resulting in full activation of PrfA and induced expression of *prfA*-dependent genes, including encoded antigens which were driven from the *actA* promoter. Thus, it is not surprising that differences in immune potency among the various live-attenuated vaccines was not as pronounced as that observed with KBMA PrfA* vaccine strains. KBMA vaccines are unable to propagate in cells of the immunized host, and under conditions of a non-replicating vector, induction of the PrfA regulon and expression of an encoded Ag prior to vaccination significantly enhanced immune potency.

In a recently published study, the immunogenicity of wild-type *Listeria monocytogenes* strain 10403 was compared with a different wild-type *Listeria monocytogenes* strain 43251, which contains an unknown activating *prfA* mutation (42). While the *Listeria monocytogenes* strain elicited enhanced LLO- and p60-specific immunity and increased protection against wild-type Listerial challenge, because this study utilized different wild-type *Listeria monocytogenes* strains, it is difficult to draw conclusions regarding underlying mechanisms and possible application to recombinant attenuated vaccine platforms that are appropriate for testing in humans. Furthermore, the impact of constitutive PrfA activation on the immunogenicity of expressed heterologous antigens in recombinant *Listeria monocytogenes* vaccine strains was not evaluated in this study.

The enhanced immune potency of KBMA PrfA* based vaccines could be due to several independent mechanisms. Contributing factors may include increased efficiency of escape from the phagolysosome and increased Ag expression and secretion in the cytosol, ultimately resulting in a higher density of epitopes displayed on MHC class I molecules and more efficient priming of CD8+ T cells. While over-expression of PrfA-dependent virulence genes can increase cytotoxicity, resulting in decreased virulence of wild-type strains and decreased vaccine potency (10, 17, 46), this mechanism does not appear to have affected the relative immunogenicity of the vaccine strains used in this study, as shown by equivalent intracellular growth in J774 cells (Figure 1E). Other possibilities may include enhanced migration of dendritic cells (DCs) to the lymph nodes of animals immunized with PrfA* vaccines, due to an improved proinflammatory cytokine milieu at the site of infection or increased InlA mediated disruption of e-cadherin DC-DC adhesions (22). Although binding of InlA to mouse E-cadherin is diminished as compared to binding to its human homolog (24, 49), increased levels of InlA from PrfA* strains may still enhance this process. On the other hand, it seems unlikely that the enhanced immune potency of KBMA PrfA* vaccines was due to either increased host range or enhanced infection of target cells. Notably, while important for oral infection, for the intramuscular or intravenous routes used in this study, InlA does not play the same significant role in mediating infection of non-phagocytic cells. Furthermore, InlB-mediated infection of hepatocytes via the hepatocyte growth factor receptor is not relevant, since this virulence determinant was deleted from the vaccine strains used in this study. Supporting this notion are the combined observations that infection of cultured macrophage or dendiritic cells was indistinguishable between all of the vaccine strains tested (Figure 1C & ID), and that the virulence of *Listeria monocytogenes* Δ*actAl*Δ*inlB* PrfA* strains was increased only 2- to 4-fold over the *Listeria monocytogenes* Δ*actAl*Δ*inlB* parent strain, which is attenuated by more than 3 logs as compared to wild-type *L. monocytogenes* (10) (Table 5).

While the results presented here demonstrate the importance of activation of the PrfA regulon to increase the potency of *Listeria monocytogenes* vaccines, Ag over-expression observed in broth culture did not necessarily correlate with enhanced immune potency. Strikingly, while *prfA* G155S, G145S, and Y63C mutations all conferred high (and equivalent) over-expression of PrfA-dependent Ag expression of vaccine strains grown in broth culture, only PrfA* G155S vaccines strains had significantly increased immunologic potency. As host-pathogen reactions are by definition a complex multi-factorial process, it is not surprising that enhanced PrfA-dependent expression did not necessarily correlate with optimal immunogenicity. Temporal regulation of PrfA-dependent genes provides expression of particular bacterial proteins in appropriate cellular compartments to facilitate pathogenesis. For example, ActA expression is induced 200-fold in the cytoplasm, to promote host cell actin polymerization and cell-to-cell spread (35, 40). The Ags in this study were expressed as an N-terminal fusion with the first 100 amino acids of ActA, and driven from a native *actA* promoter. In the case of KBMA vaccines, *prfA* G155S provided the appropriate level of PrfA-dependent induction to augment potency, but afforded a sufficient balance of metabolic economy for the photochemically inactivated bacterium. In a recent study utilizing *prfA* L104F to characterize the PrfA-dependent *Listeria monocytogenes* secretome (34), several proteins identified whose expression was not known previously to be related to activated PrfA. This data provides evidence for the multiple bacterial proteins involved in the pathogenesis of wild-type *L. monocytogenes,* and illustrates that PrfA* mutants may have a complex impact on the potency of *Listeria monocytogenes* vaccines.

The overwhelming majority of pre-clinical studies with *Listeria monocytogenes* vaccines have utilized either IV or intraperitoneal administration. There have been few reports examining IM and subcutaneous immunization routes (10, 15). However, oral immunization has been explored in mice, non-human primates, and a single study in humans (2, 8, 29, 33). The three clinical trials with *Listeria monocytogenes* conducted to date or ongoing have utilized IV administration. While KBMA *Listeria monocytogenes* vaccines may have an improved risk-to-benefit profile compared to live-attenuated vaccines, a traditional immunization route of administration may be necessary for broad adoption and/or approval, particularly in prophylactic settings. Here, we show that with a prime-boost immunization regimen KBMA PrfA* G155S vaccines elicited functional cellular immunity following IM immunization that was comparable to live-attenuated *Listeria monocytogenes* vaccines.

By evaluating the immune potency of a panel of isogenic live-attenuated and KBMA *Listeria monocytogenes* vaccine containing various *prfA* alleles, we have shown that constitutive induction of the PrfA regulon prior to immunization significantly enhances the ability of live-attenuated KBMA vaccines to elicit functional cellular immunity, using a conventional immunization route.

### REFERENCES FOR EXAMPLE 1

1. Alexander-Miller, M. A., G. R. Leggatt, and J. A. Berzofsky. (1996). Selective expansion of high- or low-avidity cytotoxic T lymphocytes and efficacy for adoptive immunotherapy. Proc. Natl. Acad. Sci. 93:4102-4107.
2. Angelakopoulos, H., K. Loock, D. Sisul, E. Jensen, J. F. Miller, and E. L. Hohmann. (2002). Safety and Shedding of an Attenuated Strain of Listeria monocytogenes with a Deletion of actA/plcB in Adult Volunteers: a Dose Escalation Study of Oral Inoculation. Infect Immun 70:3592-3601.
3. Bahjat, K. S. unpublished data.
4. Bahjat, K. S., W. Liu, E. E. Lemmens, S. P. Schoenberger, D. A. Portnoy, T. W. Dubensky, and D. Brockstedt. (2006). Activation of a Cytosolic Surveillance Pathway Controls CD8+ T Cell Potency During Bacterial Infection. Infect Immun. 74:6387-97.
5. Bahjat, K. S., W. Liu, E. E. Lemmens, S. P. Schoenberger, D. A. Portnoy, T. W. Dubensky, Jr., and D. G. Brockstedt. (2006). Cytosolic Entry Controls CD8+-T-Cell Potency during Bacterial Infection. Infect Immun 74:6387-6397.
6. Bahjat, K. S., R. A. Prell, H. E. Allen, W. Liu, E. E. Lemmens, M. L. Leong, D. A. Portnoy, T. W. Dubensky, Jr., D. G. Brockstedt, and M. A. Giedlin. (2007). Activation of immature hepatic NK cells as immunotherapy for liver metastatic disease. J Immunol 179:7376-7384.
7. Bouwer, H. G., H. Shen, X. Fan, J. G. Miller, R. A. Barry, and D. J. Hinrichs. (1999). Existing Antilisterial Immunity Does Not Inhibit the Development of a Listeria mortocytogenes-Specific Primary Cytotoxi T-Lymphocyte Response. Inflection and Immunity 67:253-258.
8. Boyer, J. D., P. C. Maciag, R. Parkinson, L. Wu, M. G. Lewis, D. B. Weiner, and Y. Paterson. (2006). Rhesus macaques with high levels of vaccine induced IFN-gamma producing cells better control viral set-point following challenge with SIV239. Vaccine 24:4498-4502.
9. Brockstedt, D. G., K. S. Bahjat, M. A. Giedlin, W. Liu, M. Leong, W. Luckett, Y. Gao, P. Schnupf, D. Kapadia, G. Castro, J. Y. Lim, A. Sampson-Johannes, A. A. Herskovits, A. Stassinopoulos, H. G. Bouwer, J. E. Hearst, D. A. Portnoy, D. N. Cook, and T. W. Dubensky, Jr. (2005). Killed but metabolically active microbes: a new vaccine paradigm for eliciting effector T-cell responses and protective immunity. Nat Med 11:853-860.
10. Brockstedt, D. G., M. A. Giedlin, M. L. Leong, K. S. Bahjat, Y. Gao, W. Luckett, W. Liu, D. N. Cook, D. A. Portnoy, and T. W. Dubensky, Jr. (2004). Listeria-based cancer vaccines that segregate immunogenicity from toxicity. Proc Natl Acad Sci U S A 101:13832-13837.
11. Bruhn, K. W., N. Craft, and J. F. Miller. (2007). Listeria as a vaccine vector. Microbes Inflect 9:1226-1235.
12. Camilli, A., L. G. Tilney, and D. A. Portnoy. (1993). Dual roles of plcA in Listeria monocytogenes pathogenesis. Mol Microbiol 8:143-157.
13. Cohen, J. (2007). AIDS research. Did Merck's failed HIV vaccine cause harm? Science 318:1048-1049.
14. Cohen, J. (2007). AIDS research. Promising AIDS vaccine's failure leaves field reeling. Science 318:28-29.
15. Datta, S. K., S. Okamoto, T. Hayashi, S. S. Shin, I. Mihajlov, A. Fermin, D. G. Guiney, J. Fierer, and E. Raz. (2006). Vaccination with irradiated Listeria induces protective T cell immunity. Immunity 25:143-152.
16. Dramsi, S., I. Biswas, E. Maguin, L. Braun, P. Mastroeni, and P. Cossart. (1995). Entry of Listeria monocytogenes into hepatocytes requires expression of inlB, a surface protein of the internalin multigene family. Mol Microbiol 16:251-261.
17. Glomski, I. J., A. L. Decatur, and D. A. Portnoy. (2003). Listeria monocytogenes mutants that fail to compartmentalize listerolysin O activity are cytotoxic, avirulent, and unable to evade host extracellular defenses. Inflect Immun 71:6754-6765.
18. Gray, M. J., N. E. Freitag, and K. J. Boor. (2006). How the bacterial pathogen Listeria monocytogenes mediates the switch from environmental Dr. Jekyll to pathogenic Mr. Hyde. Infect Immun 74:2505-2512.
19. Gunn, G. R., A. Zubair, C. Peters, Z.-K. Pan, T.-C. Wu, and Y. Paterson. (2001). Two Listeria monocytogenes vaccine vectors that express different molecular forms of human papilloma virus-16 (HPV-16) E7 induce qualitatively different T cell immunity that correlates with their ability to induce regression of established tumors Immortalized by HPV-161. J. Immunol. 167:6471-6479.
20. Hamilton, S. E., V. P. Badovinac, A. Khanolkar, and J. T. Harty. (2006). Listeriolysin O-Deficient Listeria monocytogenes as a Vaccine Delivery Vehicle: Antigen-Specific CD8 T Cell Priming and Protective Immunity. J Immurtol 177:4012-4020.
21. Harty, J. T., A. R. Tvinnereim, and D. W. White. (2000). CD8+ T cell effector mechanisms in resistance to infection. Ann. Rev. Immunol. 18:275-308.
22. Jiang, A., O. Bloom, S. Ono, W. Cui, J. Unternaehrer, S. Jiang, J. A. Whitney, J. Connolly, J. Banchereau, and I. Mellman. (2007). Disruption of E-cadherin-mediated adhesion induces a functionally distinct pathway of dendritic cell maturation. Immunity 27:610-624.
23. Lauer, P., M. Y. Chow, M. J. Loessner, D. A. Portnoy, and R. Calendar. (2002). Construction, characterization, and use of two Listeria monocytogenes site-specific phage integration vectors. J Bacteriol 184:4177-4178.
24. Lecuit, M., S. Dramsi, C. Gottardi, M. Fedor-Chaiken, B. Gumbiner, and P. Cossart. (1999). A single amino acid in E-cadherin responsible for host specificity towards the human pathogen Listeria monocytogenes. Embo J 18:3956-3963.
25. Lorber, B. (1997). Listeriosis. Clin Infect Dis 24:1-9; quiz 10-11.
26. Miner, M. D., G. C. Port, and N. E. Freitag. Submitted 2008. Functional impact of mutational activation on the Listeria monocytogenes central virulence regulator PrfA. J. Biol. Chem*.*
27. Moutaftsi, M., B. Peters, V. Pasquetto, D. C. Tscharke, J. Sidney, H. H. Bui, H. Grey, and A. Sette. (2006). A consensus epitope prediction approach identifies the breadth of murine T(CD8+)-cell responses to vaccinia virus. Nat Biotechnol 24:817-819.
28. Mueller, K. J., and N. E. Freitag. (2005). Pleiotropic Enhancement of Bacterial Pathogenesis Resulting from the Constitutive Activation of the Listeria monocytogenes Regulatory Factor PrfA. Inflect Immun 73:1917-1926.
29. Neeson, P., J. Boyer, S. Kumar, M. G. Lewis, L. Mattias, R. Veazey, D. Weiner, and Y. Paterson. (2006). A DNA prime-oral Listeria boost vaccine in rhesus macaques induces a SIV-specific CD8 T cell mucosal response characterized by high levels of alpha4beta7 integrin and an effector memory phenotype. Virology 354:299-315.
30. O'Riordan, M., C. H. Yi, R. Gonzales, K. D. Lee, and D. A. Portnoy. (2002). Innate recognition of bacteria by a macrophage cytosolic surveillance pathway. Proc Natl Acad Sci U S A 99:13861-13866.
31. Pamer, E. G. (2004). Immune responses to Listeria monocytogenes. Nat Rev Immunol 4:812-823.
32. Paterson, Y., and P. C. Maciag. (2005). Listeria-based vaccines for cancer treatment. Curr Opin Mol Ther 7:454-460.
33. Peters, C., X. Peng, D. Douven, Z. K. Pan, and Y. Paterson. (2003). The induction of HIV Gag-specific CD8+ T cells in the spleen and gut-associated lymphoid tissue by parenteral or mucosal immunization with recombinant Listeria monocytogenes HIV Gag. J Immunol 170:5176-5187.
34. Port, G. C., and N. E. Freitag. (2007). Identification of novel Listeria monocytogenes secreted virulence factors following mutational activation of the central virulence regulator, PrfA. Infect Immun 75:5886-5897.
35. Portnoy, D. A., V. Auerbuch, and I. J. Glomski. (2002). The cell biology of Listeria monocytogenes infection: the intersection of bacterial pathogenesis and cell-mediated immunity. J Cell Biol 158:409-414.
36. Rappuoli, R. (2007). Bridging the knowledge gaps in vaccine design. Nat Biotechnol 25:1361-1366.
37. Ripio, M. T., G. Dominguez-Bernal, M. Lara, M. Suarez, and J. A. Vazquez-Boland. (1997). A Gly145Ser substitution in the transcriptional activator PrfA causes constitutive overexpression of virulence factors in Listeria monocytogenes. J Bacteriol 179:1533-1540.
38. Scortti, M., H. J. Monzo, L. Lacharme-Lora, D. A. Lewis, and J. A. Vazquez-Boland. (2007). The PrfA virulence regulon. Microbes Infect*.*
39. Shen, H., M. K. Slifka, M. Matloubian, E. R. Jensen, R. Ahmed, and J. F. Miller. (1995). Recombinant Listeria monocytogenes as a live vaccine vehicle for the induction of protective anti-viral cell-mediated immunity. Proc. Natl. Acad. Sci. 92:3987-3991.
40. Shetron-Rama, L. M., H. Marquis, H. G. Bouwer, and N. E. Freitag. (2002). Intracellular induction of Listeria monocytogenes actA expression. Infect Immun 70:1087-1096.
41. Shetron-Rama, L. M., K. Mueller, J. M. Bravo, H. G. Bouwer, S. S. Way, and N. E. Freitag. (2003). Isolation of Listeria monocytogenes mutants with high-level in vitro expression of host cytosol-induced gene products. Mol Microbiol 48:1537-1551.
42. Smithey, M. J., S. Brandt, N. E. Freitag, D. E. Higgins, and H. G. Bouwer. (2008). Stimulation of enhanced CD8 T cell responses following immunization with a hyper-antigen secreting intracytosolic bacterial pathogen. J Immunol 180:3406-3416.
43. Starks, H., K. W. Bruhn, H. Shen, R. A. Barry, T. W. Dubensky, D. Brockstedt, D. J. Hinrichs, D. E. Higgins, J. F. Miller, M. Giedlin, and H. G. Bouwer. (2004). Listeria monocytogenes as a vaccine vector: virulence attenuation or existing antivector immunity does not diminish therapeutic efficacy. J Immunol 173:420-427.
44. Stevens, R., A. Lavoy, S. Nordone, M. Burkhard, and G. A. Dean. (2005). Pre-existing immunity to pathogenic Listeria monocytogenes does not prevent induction of immune responses to feline immunodeficiency virus by a novel recombinant Listeria monocytogenes vaccine. Vaccine 23:1479-1490.
45. Tvinnereim, A. R., S. E. Hamilton, and J. T. Harty. (2002). CD8(+)-T-cell response to secreted and nonsecreted antigens delivered by recombinant Listeria monocytogenes during secondary infection. Infect Immun 70:153-162.
46. Vazquez-Boland, J. A., M. Kuhn, P. Berche, T. Chakraborty, G. Dominguez-Bernal, W. Goebel, B. Gonzalez-Zorn, J. Wehland, and J. Kreft. (2001). Listeria pathogenesis and molecular virulence determinants. Chin Microbiol Rev 14:584-640.
47. Vega, Y., M. Rauch, M. J. Banfield, S. Ermolaeva, M. Scortti, W. Goebel, and J. A. Vazquez-Boland. (2004). New Listeria monocytogenes prfA* mutants, transcriptional properties of PrfA* proteins and structure-function of the virulence regulator PrfA. Mol Microbiol 52:1553-1565.
48. Villalobos, A., J. E. Ness, C. Gustafsson, J. Minshull, and S. Govindarajan. (2006). Gene Designer: a synthetic biology tool for constructing artificial DNA segments. BMC Bioinformatics 7:285.
49. Wollert, T., B. Pasche, M. Rochon, S. Deppenmeier, J. van den Heuvel, A. D. Gruber, D. W. Heinz, A. Lengeling, and W. D. Schubert. (2007). Extending the Host Range of Listeria monocytogenes by Rational Protein Design. Cell 129:891-902.
50. Wong, K. K., and N. E. Freitag. 2004. A novel mutation within the central Listeria monocytogenes regulator PrfA that results in constitutive expression of virulence gene products. J Bacteriol 186:6265-6276.

### Example 2

Immunogenicity of *Listeria monocytogenes* strains, Δ*actA*/Δ*inlB*/Δ*uvrAB-*HPV E7 (BH1409) and Δ*actA*/Δ*inlB*/Δ*uvrAB*/PrfA* G155S-HPV E7 (either BH1633 or BH1733), were compared as live-attenuated or as KBMA vaccines.

### MATERIALS AND METHODS

Wildtype and PrfA* G155S *Listeria monocytogenes* expressing HPV E7 as described for QuadVac vaccine in Example 1.

C57BL/6 mice were vaccinated intravenously with either a single administration of 1×10⁷ CFU of live-attenuated *Listeria monocytogenes* or two vaccinations of 3×10⁷ particles of KBMA Lm. Boost vaccination of KBMA was administered 14 days after primary vaccination. Spleens were harvested 7 days after last vaccination. HPV E7₄₉₋₅₇- specific CD8+ and LLO₁₉₀₋₂₀₁-specific CD4+ T cells were measured by IFN-γ ELISpot or by intracellular cytokine staining.

### RESULTS

To determine the impact of PrfA* G155S on the immunologic potency of Lm-based vaccines, C57BL/6 mice were vaccinated with HPV E7-expressing *Listeria monocytogenes* strains that contained either WT PrfA or the PrfA* G155S mutation. Spleens were harvested 7 days after the last vaccination, and E7₄₉₋₅₇- and LLO₁₉₀₋₂₀₁-specific immune responses measured by g-IFN ELISpot assay or intracellular cytokine staining as described in Example 1. Immune responses were assessed after a single vaccination with live-attenuated *Listeria monocytogenes* (1×10⁷ CFU i.v.) or after two vaccinations of photochemically-inactivated, nucleotide excision repair-deleted *Listeria monocytogenes* (KBMA; 3 × 10⁷ particles i.v.). The inclusion of PrfA* G155S dramatically enhanced the E7-specific CD8+ T cell responses induced by both live-attenuated *Listerial monocytogenes* and KBMA *Listerial monocytogenes* vaccine strains (Figure 5). Additionally, CD4+ LLO₁₉₀₋₂₀₁-specific responses were also significantly increased after vaccination with the live-attenuated PrfA* G155S strain as compared to the WT PrfA strain (Figure 6).

## Claims

1. A recombinant *Listeria* bacterium for use in a method of treatment, wherein
(A) said bacterium comprises:
(a) a polynucleotide encoding a PrfA* mutant polypeptide wherein the PrfA* mutant polypeptide comprises a G155S mutation; and
(b) a recombinant polynucleotide comprising
(i) an *actA* promoter; and
(ii) a polynucleotide encoding a heterologous polypeptide, wherein the polynucleotide encoding the heterologous polypeptide is operably linked to the *actA* promoter, and wherein the heterologous polypeptide is non-bacterial or is an antigen of a heterologous infectious pathogen; and
(B) said method is selected from:
(i) a method of inducing an immune response in a host to the non-listerial antigen encoded by the heterologous polypeptide;
(ii) a method of enhancing the immunogenicity of the non-listerial antigen encoded by the heterologous polypeptide in a host;
(iii) a method of preventing or treating a non-listerial infectious or cancerous condition in a host; or
(iv) a method for enhancing an immune response in a mammal to the non-listerial antigen encoded by the heterologous polypeptide in a host, wherein the mammal previously had been administered an effective amount of a prime dose of a vaccine that provided the non-listerial antigen, wherein (a) the vaccine does not contain live, metabolically active *Listeria* that encode the non-listerial antigen; and (b) when the vaccine contains naked DNA encoding the non-listerial antigen.

2. The recombinant *Listeria* bacterium for use according to claim 1, wherein the recombinant polynucleotide encodes a fusion protein comprising a signal peptide and the heterologous polypeptide.

3. The recombinant *Listeria* bacterium for use according to claim 2, wherein the signal peptide is an ActA signal peptide from *Listeria monocytogenes.*

4. The recombinant *Listeria* bacterium for use according to claim 2 or 3, wherein the fusion protein comprises the first 100 amino acids of ActA.

5. The recombinant *Listeria* bacterium for use according to any one of claims 1-4, wherein the heterologous polypeptide comprises an antigen selected from the group consisting of a tumor-associated antigen, a polypeptide derived from a tumor-associated antigen, an infectious disease antigen, and a polypeptide derived from an infectious disease antigen.

6. The recombinant *Listeria* bacterium for use according to claim 5, wherein the heterologous polypeptide is an antigen selected from the group consisting of K-Ras, H-Ras, N-Ras, 12-K-Ras, mesothelin, PSCA, NY-ESO-1, WT-1, survivin, gp100, PAP, proteinase 3, SPAS-1, B-raf, tyrosinase, mdm-2, MAGE, RAGE, MART-1, bcr/abl, Her-2/neu, alphafetoprotein, mammoglobin, hTERT(telomerase), PSA and CEA, or comprises a polypeptide derived from an antigen selected from the group consisting of K-Ras, H-Ras, N-Ras, 12-K-Ras, mesothelin, PSCA, NY-ESO-1, WT-1, survivin, gp100, PAP, proteinase 3, SPAS-1, B-raf, tyrosinase, mdm-2, MAGE, RAGE, MART-1, bcr/abl, Her-2/neu, alphafetoprotein, mammoglobin, hTERT(telomerase), PSA and CEA.

7. The recombinant *Listeria* bacterium for use according to claim 5, wherein the infectious disease antigen is from a virus or a heterologous infectious pathogen selected from the group consisting of a hepatitis virus such as a hepatitis A virus, a hepatitis B virus, or a hepatitis C virus; an influenza virus; a human immunodeficiency virus; papillomavirus; a herpes simplex virus 1; a herpes simplex virus 2; a cytomegalovirus; a *Mycobacterium tuberculosis*; a *Plasmodium falciparum*; or a *Chlamydia trachomaitis.*

8. The recombinant *Listeria* bacterium for use according to any one of claims 1-7, wherein the *Listeria* bacterium belongs to the species *Listeria monocytogenes.*

9. The recombinant *Listeria* bacterium for use according to any one of claims 1-8, which is attenuated for one or more of cell-to-cell spread, entry into non-phagocytic cells, proliferation or DNA repair.

10. The recombinant *Listeria* bacterium for use according to claim 9, wherein the *Listeria* is attenuated by one or more of:
a. an *actA* mutation;
b. an *in*/*B* mutation;
c. a *uvrA* mutation;
d. a *uvrB* mutation;
e. a *uvrC* mutation;
f. a nucleic acid targeted compound; or
g. a *uvrAB* mutation and a nucleic acid targeting compound.

11. The recombinant *Listeria* bacterium for use according to claim 10, wherein the nucleic acid targeting compound is a psoralen.

12. The recombinant *Listeria* bacterium for use according to any one of claims 1-8 which is killed but metabolically active (KBMA).

13. The recombinant *Listeria* bacterium for use according to any one of claims 1-8, wherein the bacterium is attenuated for proliferation by modification of its nucleic acid of the bacterium by reaction with a nucleic acid targeting compound that reacts directly with the nucleic acid; by the presence of nucleic acid cross-links that attenuate the modified bacterium for proliferation; or by the presence of psoralen-nucleic acid adducts that attenuate the bacterium for proliferation.

14. The recombinant *Listeria* bacterium for use according to claim 13, wherein the bacterium further comprises a genetic mutation that attenuates the ability of the bacterium to repair its modified nucleic acid.

15. The recombinant *Listeria* bacterium for use according to claim 14, wherein the bacterium comprises inactivating mutations in *actA*, *inlB*, *uvrA* and *uvrB*; and wherein the bacterium has been attenuated for proliferation by psoralen-nucleic acid crosslinks.

## Patentansprüche

1. Rekombinantes *Listeria*-Bakterium zur Verwendung in einem Behandlungsverfahren, worin
(A) das Bakterium Folgendes umfasst:
(a) ein Polynucleotid, das für ein mutiertes PrfA*-Polypeptid kodiert, worin das mutierte PrfA*-Polypeptid eine G155S-Mutation umfasst; und
(b) ein rekombinantes Polynucleotid, das Folgendes umfasst:
(i) einen actA-Promotor; und
(ii) ein Polynucleotid, das für ein heterologes Polypeptid kodiert, worin das Polynucleotid, das für das heterologe Polynucleotid kodiert, operabel mit dem actA-Promotor verbunden ist und worin das heterologe Polypeptid nicht bakteriell ist oder ein Antigen eines heterologen infektiösen Pathogens ist; und
(B) das Verfahren aus folgenden ausgewählt ist:
(i) einem Verfahren zur Auslösung einer Immunantwort auf das Nicht-*Listeria*-Antigen, für das das heterologe Polypeptid kodiert, bei einem Wirt;
(ii) einem Verfahren zur Verstärkung der Immunogenität des Nicht-*Listeria*-Antigen, für das das heterologe Polypeptid kodiert, in einem Wirt;
(iii) einem Verfahren zur Prävention oder Behandlung eines Nicht-*Listeria*-Infektions- oder Krebsleidens bei einem Wirt; oder
(iv) einem Verfahren zur Verstärkung einer Immunantwort auf das Nicht-*Listeria*-Antigen, für das das heterologe Polypeptid in einem Wirt kodiert, bei einem Säugetier, worin dem Säugetier zuvor eine wirksame Menge einer Einstiegsdosis einer Vakzine verabreicht wurde, die das Nicht-*Listeria*-Antigen bereitgestellt hat, worin (a) die Vakzine kein lebendes, metabolisch aktives *Listeria* enthält, das für das Nicht-*Listeria*-Antigen kodiert; und (b) die Vakzine nackte DNA enthält, die für das Nicht-*Listeria*-Antigen kodiert.

2. Rekombinantes *Listeria*-Bakterium zur Verwendung nach Anspruch 1, worin das rekombinante Polynucleotid für ein Fusionsprotein kodiert, das ein Signalpeptid und das heterologe Polypeptid umfasst.

3. Rekombinantes *Listeria*-Bakterium zur Verwendung nach Anspruch 2, worin das Signalpeptid ein ActA-Signalpeptid aus *Listeria monocytogenes* ist.

4. Rekombinantes *Listeria*-Bakterium zur Verwendung nach Anspruch 2 oder 3, worin das Fusionsprotein die ersten 100 Aminosäuren von ActA umfasst.

5. Rekombinantes *Listeria*-Bakterium zur Verwendung nach einem der Ansprüche 1 bis 4, worin das heterologe Polypeptid ein aus der aus einem tumorassoziierten Antigen, einem von einem tumorassoziierten Antigen stammenden Polypeptid, einem Infektionskrankheitsantigen und einem von einem Infektionskrankheitsantigen stammenden Polypeptid bestehenden Gruppe ausgewähltes Antigen umfasst.

6. Rekombinantes *Listeria*-Bakterium zur Verwendung nach Anspruch 5, worin das heterologe Polypeptid ein aus der aus K-Ras, H-Ras, N-Ras, 12-K-Ras, Mesothelin, PSCA, NY-ESO-1, WT-1, Servivin, gp100, PAP, Proteinase 3, SPAS-1, B-raf, Tyrosinase, mdm-2, MAGE, RAGE, MART-1, bcr/abl, Her-2/neu, Alphafetoprotein, Mammoglobin, hTERT(telomerase), PSA und CEA bestehenden Gruppe ausgewähltes Antigen ist oder ein von aus der aus K-Ras, H-Ras, N-Ras, 12-K-Ras, Mesothelin, PSCA, NY-ESO-1, WT-1, Servivin, gp100, PAP, Proteinase 3, SPAS-1, B-raf, Tyrosinase, mdm-2, MAGE, RAGE, MART-1, bcr/abl, Her-2/neu, Alphafetoprotein, Mammoglobin, hTERT(telomerase), PSA und CEA bestehenden Gruppe ausgewählten Antigen abstammendes Polypeptid umfasst.

7. Rekombinantes *Listeria*-Bakterium zur Verwendung nach Anspruch 5, worin das Infektionskrankheitsantigen aus einem aus der aus einem Hepatitisvirus, wie z.B. einem Hepatitis-A-Virus, einem Hepatitis-B-Virus oder einem Hepatitis-C-Virus; einem Influenzavirus; einem menschlichen Immunschwächevirus; dem Papillomavirus; einem Herpes-simplex-Virus 1; einem Herpes-simplex-Virus 2; einem Zytomegalievirus; einem *Mycobacterium tuberculosis*; einem *Plasmodium falciparum;* oder einem *Chlamydia trachomaitis* bestehenden Gruppe ausgewählten Virus oder heterologen Infektionspathogen stammt.

8. Rekombinantes *Listeria*-Bakterium zur Verwendung nach einem der Ansprüche 1 bis 7, worin das *Listeria*-Bakterium der Spezies *Listeria monocytogenes* angehört.

9. Rekombinantes *Listeria*-Bakterium zur Verwendung nach einem der Ansprüche 1 bis 8, das für eines oder mehrere von Zell/Zell-Spreitung, Eintritt in nicht phagozytische Zellen, Proliferation oder DNA-Reparatur attenuiert ist.

10. Rekombinantes *Listeria*-Bakterium zur Verwendung nach Anspruch 9, worin das *Listeria* durch eines oder mehrere der folgenden attenuiert ist:
a. eine actA-Mutation;
b. eine inlB-Mutation;
c. eine uvrA-Mutation;
d. eine uvrB-Mutation;
e. eine uvrC-Mutation;
f. eine auf eine Nucleinsäure gerichtete Verbindung; oder
g. eine uvrAB-Mutation und eine auf eine Nucleinsäure gerichtete Verbindung.

11. Rekombinantes *Listeria*-Bakterium zur Verwendung nach Anspruch, worin die auf eine Nucleinsäure gerichtete Verbindung ein Psoralen ist.

12. Rekombinantes *Listeria*-Bakterium zur Verwendung nach einem der Ansprüche 1 bis 8, das abgetötet, aber metabolisch aktiv (KBMA) ist.

13. Rekombinantes *Listeria*-Bakterium zur Verwendung nach einem der Ansprüche 1 bis 8, worin das Bakterium durch Modifikation der Nucleinsäure des Bakteriums durch Umsetzung mit einer auf eine Nucleinsäure gerichteten Verbindung, die direkt mit der Nucleinsäure reagiert; durch die Gegenwart von Nucleinsäurevernetzungen, die das modifizierte Bakterium bezüglich Proliferation attenuieren; oder durch die Gegenwart von Psoralen-Nucleinsäure-Addukten, die das Bakterium bezüglich Proliferation attenuieren, attenuiert ist.

14. Rekombinantes *Listeria*-Bakterium zur Verwendung nach Anspruch 13, worin das Bakterium weiters eine genetische Mutation umfasst, die die Fähigkeit des Bakteriums, seine modifizierte Nucleinsäure zu reparieren, attenuiert.

15. Rekombinantes *Listeria*-Bakterium zur Verwendung nach Anspruch 14, worin das Bakterium deaktivierende Mutationen in actA, inlB, uvrA und uvrB umfasst; und worin das Bakterium durch Psoralen-Nucleinsäure-Vernetzungen bezüglich Proliferation attenuiert wurde.

## Revendications

1. Bactérie *Listeria* recombinante destinée à être utilisée dans un procédé de traitement, où
(A) ladite bactérie comprend :
(a) un polynucléotide codant pour un polypeptide mutant PrfA*, où le polypeptide mutant PrfA* comprend une mutation G155S ; et
(b) un polynucléotide recombinant comprenant
(i) un promoteur *actA* ; et
(ii) un polynucléotide codant pour un polypeptide hétérologue, où le polynucléotide codant pour le polypeptide hétérologue est en liaison fonctionnelle avec le promoteur *actA,* et où le polypeptide hétérologue est non bactérien ou est un antigène d'un agent pathogène infectieux hétérologue ; et
(B) ledit procédé est sélectionné parmi :
(i) un procédé d'induction d'une réponse immunitaire chez un hôte contre l'antigène non issu de listeria codé par le polypeptide hétérologue ;
(ii) un procédé de stimulation de l'immunogénicité de l'antigène non issu de listeria codé par le polypeptide hétérologue chez un hôte ;
(iii) un procédé de prévention ou de traitement d'un état pathologique cancéreux ou infectieux non associé à listeria chez un hôte ; ou
(iv) un procédé de stimulation d'une réponse immunitaire chez un mammifère contre l'antigène non issu de listeria codé par le polypeptide hétérologue chez un hôte, où le mammifère a précédemment reçu une administration d'une quantité efficace d'une dose de sensibilisation d'un vaccin qui contenait l'antigène non issu de listeria, où (a) le vaccin ne contient pas de *Listeria* vivante métaboliquement active qui code pour l'antigène non issu de listeria ; et (b) lorsque le vaccin contient de l'ADN nu codant pour l'antigène non issu de listeria.

2. Bactérie *Listeria* recombinante destinée à être utilisée selon la revendication 1, dans laquelle le polynucléotide recombinant code pour une protéine de fusion comprenant un peptide signal et le polypeptide hétérologue.

3. Bactérie *Listeria* recombinante destinée à être utilisée selon la revendication 2, dans laquelle le peptide signal est un peptide signal ActA de *Listeria monocytogenes.*

4. Bactérie *Listeria* recombinante destinée à être utilisée selon la revendication 2 ou 3, dans laquelle la protéine de fusion comprend les 100 premiers acides aminés de ActA.

5. Bactérie *Listeria* recombinante destinée à être utilisée selon l'une quelconque des revendications 1 à 4, dans laquelle le polypeptide hétérologue comprend un antigène sélectionné dans le groupe consistant en un antigène associé aux tumeurs, un polypeptide dérivé d'un antigène associé aux tumeurs, un antigène d'une maladie infectieuse, et un polypeptide dérivé d'un antigène d'une maladie infectieuse.

6. Bactérie *Listeria* recombinante destinée à être utilisée selon la revendication 5, dans laquelle le polypeptide hétérologue est un antigène sélectionné dans le groupe consistant en K-Ras, H-Ras, N-Ras, 12-K-Ras, la mésothéline, le PSCA, NY-ESO-1, WT-1, la survivine, la gp100, PAP, la protéinase 3, SPAS-1, B-raf, tyrosinase, mdm-2, MAGE, RAGE, MART-1, bcr/abl, Her-2/neu, l'alpha-foetoprotéine, la mammoglobine, hTERT (télomérase), le PSA et l'ACE, ou comprend un polypeptide dérivé d'un antigène sélectionné dans le groupe consistant en K-Ras, H-Ras, N-Ras, 12-K-Ras, la mésothéline, le PSCA, NY-ESO-1, WT-1, la survivine, la gp100, PAP, la protéinase 3, SPAS-1, B-raf, tyrosinase, mdm-2, MAGE, RAGE, MART-1, bcr/abl, Her-2/neu, l'alpha-foetoprotéine, la mammoglobine, hTERT (télomérase), le PSA et l'ACE.

7. Bactérie *Listeria* recombinante destinée à être utilisée selon la revendication 5, dans laquelle l'antigène d'une maladie infectieuse est issu d'un virus ou d'un agent pathogène infectieux hétérologue sélectionné dans le groupe consistant en un virus de l'hépatite comme un virus de l'hépatite A, un virus de l'hépatite B, ou un virus de l'hépatite C ; un virus de la grippe ; un virus de l'immunodéficience humaine ; un papillomavirus ; un virus herpès simplex de type 1 ; un virus herpès simplex de type 2 ; un cytomégalovirus ; une *Mycobacterium tuberculosis ;* une *Plasmodium falciparum ;* ou une *Chlamydia trachomatis.*

8. Bactérie *Listeria* recombinante destinée à être utilisée selon l'une quelconque des revendications 1 à 7, où la bactérie *Listeria* appartient à l'espèce *Listeria monocytogenes*.

9. Bactérie *Listeria* recombinante destinée à être utilisée selon l'une quelconque des revendications 1 à 8, qui est atténuée pour un ou plusieurs éléments parmi la propagation intercellulaire, la pénétration dans des cellules non phagocytaires, la prolifération ou la réparation de l'ADN.

10. Bactérie *Listeria* recombinante destinée à être utilisée selon la revendication 9, où la *Listeria* est atténuée par un(e) ou plusieurs parmi :
a. une mutation de *actA* ;
b. une mutation de *inlB* ;
c. une mutation de *uvrA* ;
d. une mutation de *uvrB* ;
e. une mutation de *uvrC* ;
f. un composé ciblé vers un acide nucléique ; ou
g. une mutation de *uvrAB* et un composé ciblant un acide nucléique.

11. Bactérie *Listeria* recombinante destinée à être utilisée selon la revendication 10, où le composé ciblant un acide nucléique est un psoralène.

12. Bactérie *Listeria* recombinante destinée à être utilisée selon l'une quelconque des revendications 1 à 8, qui est tuée mais métaboliquement active (KBMA).

13. Bactérie *Listeria* recombinante destinée à être utilisée selon l'une quelconque des revendications 1 à 8, où la bactérie est atténuée pour la prolifération par une modification de son acide nucléique bactérien par une réaction avec un composé ciblant l'acide nucléique qui réagit directement avec l'acide nucléique ; par la présence de réticulations d'acide nucléique qui atténuent la bactérie modifiée pour la prolifération ; ou par la présence d'adduits psoralène-acide nucléique qui atténuent la bactérie pour la prolifération.

14. Bactérie *Listeria* recombinante destinée à être utilisée selon la revendication 13, où la bactérie comprend en outre une mutation génétique qui atténue l'aptitude de la bactérie à réparer son acide nucléique modifié.

15. Bactérie *Listeria* recombinante destinée à être utilisée selon la revendication 14, où la bactérie comprend des mutations d'inactivation dans *actA*, *inlB*, *uvrA* et *uvrB* ; et où la bactérie a été atténuée pour la prolifération par des réticulations psoralène-acide nucléique.
